(19) Europäisches Patentamt
European Patent Office
Office européen des brevets

(11) EP 3 197 887 B1

(12) **EUROPÄISCHE PATENTSCHRIFT**

(45) Veröffentlichungstag und Bekanntmachung des Hinweises auf die Patenterteilung:
**23.09.2020 Patentblatt 2020/39**

(21) Anmeldenummer: **15759638.8**

(22) Anmeldetag: **27.08.2015**

(51) Int Cl.:
***C07D 405/14*** *(2006.01)* ***C07D 311/80*** *(2006.01)*
***C07D 405/04*** *(2006.01)* ***C07D 407/04*** *(2006.01)*
***C07D 407/10*** *(2006.01)* ***C07D 407/12*** *(2006.01)*
***C07D 407/14*** *(2006.01)* ***C07D 409/10*** *(2006.01)*
***C07D 409/14*** *(2006.01)* ***C07D 413/04*** *(2006.01)*
***C07D 417/04*** *(2006.01)* ***C07D 417/10*** *(2006.01)*
***C07D 471/04*** *(2006.01)* ***C07D 491/052*** *(2006.01)*
***C07D 493/04*** *(2006.01)* ***C07D 513/04*** *(2006.01)*
***H01L 51/50*** *(2006.01)*

(86) Internationale Anmeldenummer:
**PCT/EP2015/001750**

(87) Internationale Veröffentlichungsnummer:
**WO 2016/045769 (31.03.2016 Gazette 2016/13)**

(54) **HETEROCYCLISCHE VERBINDUNGEN MIT BENZO[C]COUMARIN-STRUKTUREN**

HETEROCYCLIC COMPOUNDS WITH BENZO[C]COUMARIN-STRUCTURES

COMPOSÉS HÉTÉROCYCLIQUES À STRUCTURES BENZO [C]COUMARINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.09.2014 EP 14003317**

(43) Veröffentlichungstag der Anmeldung:
**02.08.2017 Patentblatt 2017/31**

(73) Patentinhaber: **Merck Patent GmbH**
**64293 Darmstadt (DE)**

(72) Erfinder:
- **PARHAM, Amir Hossain**
**60486 Frankfurt am Main (DE)**
- **MUJICA-FERNAUD, Teresa**
**64283 Darmstadt (DE)**
- **EBERLE, Thomas**
**76829 Landau (DE)**
- **JATSCH, Anja**
**60489 Frankfurt am Main (DE)**
- **KROEBER, Jonas Valentin**
**60311 Frankfurt am Main (DE)**
- **GROSSMANN, Tobias**
**64297 Darmstadt (DE)**

(56) Entgegenhaltungen:
**EP-A1- 2 468 725**
**WO-A1-03/099901**
**WO-A1-2015/082056**
**WO-A1-2015/106789**
**WO-A2-2014/015935**
**US-A1- 2007 051 922**
**US-A1- 2009 039 765**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 197 887 B1

## Beschreibung

Die vorliegende Erfindung betrifft elektronische Vorrichtungen enthaltend heterocyclischeVerbindungen mit Benzo[c]coumarin-Strukturen, gemäß Anspruch 1.

Elektronische Vorrichtungen, welche organische, metallorganische und/oder polymere Halbleiter enthalten, gewinnen zunehmend an Bedeutung, wobei diese aus Kostengründen und aufgrund ihrer Leistungsfähigkeit in vielen kommerziellen Produkten eingesetzt werden. Als Beispiele seien hier Ladungstransportmaterialien auf organischer Basis (z.B. Lochtransporter auf Triarylamin-Basis) in Kopiergeräten, organischen oder polymeren Leuchtdioden (OLEDs oder PLEDs) und in Anzeige- und Displayvorrichtungen oder organische Photorezeptoren in Kopierern genannt. Organische Solarzellen (O-SC), organische Feldeffekt-Transistoren (O-FET), organische Dünnfilm-Transistoren (O-TFT), organische Schaltelemente (O-IC), organische optische Verstärker und organische Laserdioden (O-Laser) sind in einem fortgeschrittenen Entwicklungsstand und können in der Zukunft große Bedeutung erlangen.

Viele dieser elektronischen Vorrichtungen weisen unabhängig von dem jeweiligen Verwendungszweck folgenden allgemeinen Schichtaufbau auf, der für die jeweilige Anwendung angepasst werden kann:

(1) Substrat,
(2) Elektrode, häufig metallisch oder anorganisch, aber auch aus organischen bzw. polymeren, leitfähigen Materialien,
(3) Ladungsinjektionsschicht/en bzw. Zwischenschicht/en, beispielsweise zum Ausgleich von Unebenheiten der Elektrode ("planarisation layer"), häufig aus einem leitfähigen, dotierten Polymer,
(4) Organische Halbleiter,
(5) evtl. weitere Ladungstransport-, Ladungsinjektions- bzw. Ladungsblockierschichten,
(6) Gegenelektrode, Materialien wie unter (2) genannt,
(7) Verkapselung.

Die obige Anordnung stellt den allgemeinen Aufbau einer organischen, elektronischen Vorrichtung dar, wobei verschiedene Schichten zusammengefasst werden können, so dass im einfachsten Fall eine Anordnung aus zwei Elektroden resultiert, zwischen denen sich eine organische Schicht befindet. Die organische Schicht erfüllt in diesem Fall alle Funktionen, einschließlich der Emission von Licht im Fall von OLEDs. Ein derartiges System ist beispielsweise in der WO 90/13148 A1 auf der Basis von Poly-(p-phenylenen) beschrieben.

Elektronische Vorrichtungen, die polymere Verbindungen mit Benzo[c]-coumarin-Strukturen enthalten, sind unter anderem aus den Veröffentlichungen JP 2009-073808 A, WO 2005/33174 A, WO 2004/39859 A und WO 2003/99901 A bekannt.

In WO2014/015935 und WO2015/082056 werden Benzo[c]coumarinderivate als Startmaterialien zur Herstellung von weiteren Verbindungen beschrieben, die in elektronischen Vorrichtungen verwendet werden können.

In US 2007051922 sind Polymere beschrieben, die Benzo[c]coumarine als Struktureinheiten enthalten.

Bekannte elektronische Vorrichtungen weisen ein brauchbares Eigenschaftsprofil auf. Allerdings besteht die dauerhafte Notwendigkeit, die Eigenschaften dieser Vorrichtungen zu verbessern.

Zu diesen Eigenschaften gehört insbesondere die Energieeffizienz, mit der eine elektronische Vorrichtung die vorgegebene Aufgabe löst. Bei organischen Leuchtdioden, die sowohl auf niedermolekularen Verbindungen als auch auf polymeren Materialien basieren können, sollte insbesondere die Lichtausbeute hoch sein, so dass zum Erreichen eines bestimmten Lichtflusses möglichst wenig elektrische Leistung aufgebracht werden muss. Weiterhin sollte auch zum Erzielen einer vorgegebenen Leuchtdichte eine möglichst geringe Spannung notwendig sein. Ein weiteres Problem stellt insbesondere die Lebensdauer der elektronischen Vorrichtungen dar.

Aufgabe der vorliegenden Erfindung ist daher die Bereitstellung von Verbindungen, welche zu Elektronischen Vorrichtungen mit verbesserten Eigenschaften führen. Insbesondere ist die Aufgabe Lochtransportmaterialien, Lochinjektionsmaterialien, Lochblockiermaterialien, Elektroneninjektionsmaterialien, Elektronenblockiermaterialien und/oder Elektronentransportmaterialien bereitzustellen, welche in der Vorrichtung verbesserte Eigenschaften in Bezug auf Effizienz, Betriebsspannung und/oder Lebensdauer zeigen. Weiterhin sollten sich die Verbindungen möglichst einfach verarbeiten lassen, insbesondere eine gute Löslichkeit und Filmbildung zeigen.

Eine weitere Aufgabe kann darin gesehen werden, elektronische Vorrichtungen mit einer ausgezeichneten Leistungsfähigkeit möglichst kostengünstig und in konstanter Qualität bereitzustellen

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschenderweise wurde gefunden, dass diese sowie weitere nicht explizit genannte Aufgaben, die jedoch aus den hierin einleitend diskutierten Zusammenhängen ohne Weiteres ableitbar oder erschließbar sind, durch Vorrichtungen mit allen Merkmalen des Patentanspruchs 1 gelöst werden.

Gegenstand der Erfindung ist somit eine elektronische Vorrichtung, nach Anspruch 1, enthaltend mindestens eine Verbindung umfassend mindestens eine Struktur der Formel (I)

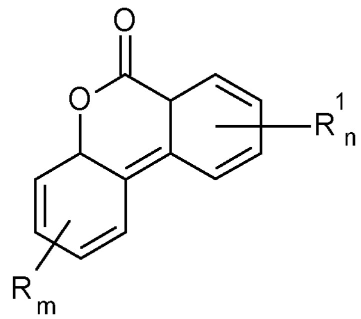

Formel (I).

Für verwendete Symbole gilt:

R ist bei jedem Auftreten gleich oder verschieden H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^2)_3$, $B(OR^2)_2$, $OSO_2R^2$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch C=C , $Si(R^2)_2$, $Ge(R^2)_2$, $Sn(R^2)_2$, C=O, C=S, C=Se, $P(=O)(R^2)$, SO, $SO_2$, O, S oder $CONR^2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ring-atomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder eine Kombination dieser Systeme; dabei können zwei oder mehrere benachbarte Substituenten R auch miteinander, mit dem Ring an dem R gebunden ist, mit einem Ring zu dem R benachbart ist oder einem Rest $R^1$ ein mono- oder polycyclisches, aliphatisches oder aromatisches Ringsystem bilden;

$R^1$ ist bei jedem Auftreten gleich oder verschieden H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^2)_3$, $B(OR^2)_2$, $OSO_2R^2$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C=C , $Si(R^2)_2$, $Ge(R^2)_2$, $Sn(R^2)_2$, C=O, C=S, C=Se, $P(=O)(R^2)$, SO, $SO_2$, O, S oder $CONR^2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder eine Kombination dieser Systeme; dabei können zwei oder mehrere benachbarte Substituenten $R^1$ auch miteinander, mit dem Ring an dem $R^1$ gebunden ist, mit einem Ring zu dem $R^1$ benachbart ist oder einem Rest R ein mono- oder polycyclisches, aliphatisches oder aromatisches Ringsystem bilden;

$R^2$ ist bei jedem Auftreten gleich oder verschieden H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^3)_3$, $B(OR^3)_2$, $OSO_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch C=C , $Si(R^3)_2$, $Ge(R^3)_2$, $Sn(R^3)_2$, C=O, C=S, C=Se, $P(=O)(R^3)$, SO, $SO_2$, O, S oder $CONR^3$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxygruppe mit 5 bis 40 aromatischen Ringatomen, die durch einen oder mehrere Reste $R^3$ substituiert sein kann, oder eine Kombination dieser Systeme; dabei können zwei oder mehrere benachbarte Substituenten $R^2$ auch miteinander ein mono- oder polycyclisches, aliphatisches oder aromatisches Ringsystem bilden;

$Ar^1$ ist bei jedem Auftreten gleich oder verschieden ein aromatisches oder heteroaromatisches Ringsystem mit 5 bis 30 aromatischen Ringatomen, das mit einem oder mehreren Resten $R^2$ substituiert sein kann; dabei können

auch zwei Reste $Ar^1$, welche an dasselbe Phosphoratom binden, durch eine Einfachbindung oder eine Brücke, ausgewählt aus $B(R^3)$, $C(R^3)_2$, $Si(R^3)_2$, C=O, $C=NR^3$, $C=C(R^3)_2$, O, S, S=O, $SO_2$, $N(R^3)$, $P(R^3)$ und $P(=O)R^3$, miteinander verknüpft sein;

$R^3$ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten $R^3$ auch miteinander ein mono- oder polycyclisches, aliphatisches oder aromatisches Ringsystem bilden;

m, n ist jeweils unabhängig voneinander 0, 1, 2, 3 oder 4;

mit der Maßgabe, dass
die Summe aus m und n größer oder gleich 1 ist;
mindestens eine der Gruppen R und/oder $R^1$ in Formel (I) mindestens eine Gruppe L darstellt; und

L eine aromatische Gruppe mit 10 bis 40 C-Atomen, vorzugsweise eine aromatische Gruppe mit 10 bis 20 C-Atomen, oder eine heteroaromatische Gruppe mit 6 bis 40 C-Atomen vorzugsweise eine heteroaromatische Gruppe mit 6 bis 20 C-Atomen, ist, wobei die aromatische und/oder heteroaromatische Gruppe mindestens zwei benachbarte aromatische und/oder heteroaromatische Ringe umfasst, die jeweils kondensiert oder nicht-kondensiert und/oder durch einen oder mehrere Reste $R^2$ substituiert sein können.

Dabei bedeutet "benachbarte Kohlenstoffatome", dass die Kohlenstoffatome direkt aneinander gebunden sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Insbesondere weist die obligatorische Gruppe L eine aromatische und/oder heteroaromatische Gruppe auf, die mindestens zwei benachbarte aromatische und/oder heteroaromatische Ringe umfasst. Demgemäß können die Ringe über eine Bindung miteinander verbunden sind, so dass die Gruppe L beispielsweise eine Biphenylgruppe umfassen kann. Weiterhin können die Ringe kondensiert sein, so dass beispielsweise zwei C-Atome zu den mindenstens zwei aromatischen oder heteroaromatischen Ringen zugehören, wie dies beispielsweise in einer Naphthylgruppe gegeben ist.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60 C-Atome im Ringsystem. Ein heteroaromatisches Ringsystem im Sinne dieser Erfindung enthält 1 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl oder Terphenyl, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{40}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl,

Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 60 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

In einer bevorzugten Ausgestaltung kann vorgesehen sein, dass die Summe der Indices m und n kleiner oder gleich 6, vorzugsweise kleiner oder gleich 5, insbesondere bevorzugt kleiner oder gleich 4, besonders bevorzugt kleiner oder gleich 3 ist. Speziell bevorzugt ist die Summe der Indices m und n 1 oder 2.

Ferner kann vorgesehen sein, dass die Struktur gemäß Formel (I) bevorzugt höchstens 4, vorzugsweise höchstens 3 und besonders bevorzugt eine oder zwei Gruppen L aufweist.

Vorzugsweise kann die Gruppe L in Formel (I) mindestens drei aromatische oder heteroaromatische Ringe umfassen, die nicht-kondensiert oder kondensiert sein können.

Darüber hinaus sind Verbindungen bevorzugt, die dadurch gekennzeichnet sind, dass die Gruppe L in Formel (I) mindestens eine Biphenyl-, Fluorenyl- und/oder Spirobifluorenyl-Gruppe umfasst.

Weiterhin zeigen Verbindungen gemäß Formel (I) überraschende Vorteile, bei denen der Index m 1 oder 2 ist und mindestens einer der Reste R eine Gruppe L darstellt, wobei der Index n vorzugsweise gleich 0 ist.

Ferner können Verbindungen gemäß Formel (I) mit Vorteil eingesetzt werden, bei denen der Index n 1 oder 2 ist und mindestens einer der Reste $R^1$ eine Gruppe L darstellt wobei der Index m vorzugsweise gleich 0 ist.

Darüber hinaus kann vorgesehen sein, dass in Formel (I) der Index m 1 oder 2 ist und der Index n 1 oder 2 ist, wobei mindestens einer der Reste R eine Gruppe L und mindestens einer der Reste $R^1$ eine Gruppe L darstellt.

Für den Fall, dass n und m größer oder gleich 1 ist, können die Reste R und $R^1$ gleich sein. Ferner kann vorgesehen sein, dass sich die Reste R und $R^1$ unterscheiden, wobei dies bevorzugt ist. Hierbei genügt für einen Unterschied, dass ein Strukturelement an einer anderen Stelle an die Benzo[c]coumarin-Struktur bindet. Beispielsweise unterscheidet sich der Rest R von einem Rest $R^1$, falls R eine Gruppe der nachfolgend dargelegten Struktur L-2 ist, während $R^1$ eine Struktur gemäß Formel L-3 darstellt.

Bevorzugt sind Verbindungen umfassend Strukturen der Formel (I), in denen mindestens ein Rest L für eine Gruppe steht, die ausgewählt ist aus den Formeln (L-1) bis (L-14)

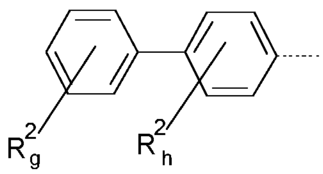
Formel (L-1)

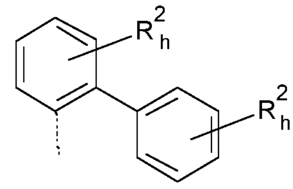
Formel (L-2)

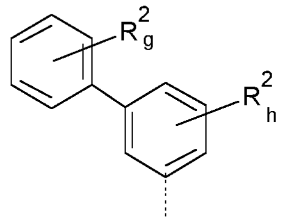
Formel (L-3)

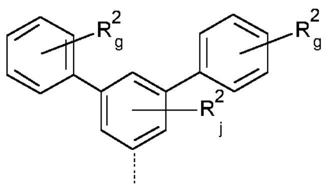
Formel (L-4)

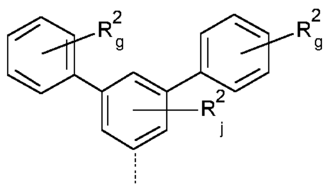
Formel (L-5)

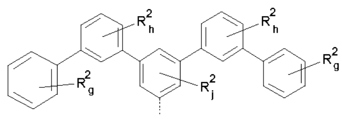
Formel (L-6)

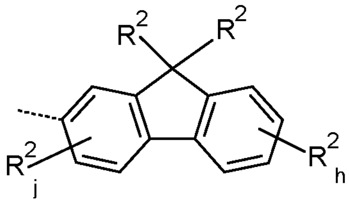
Formel (L-7)

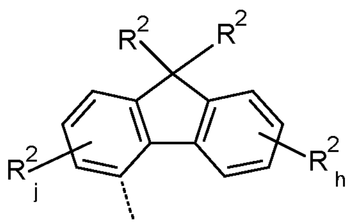
Formel (L-8)

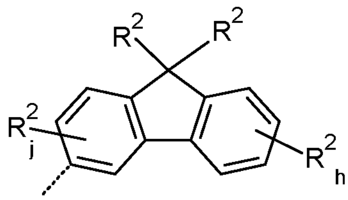
Formel (L-9)

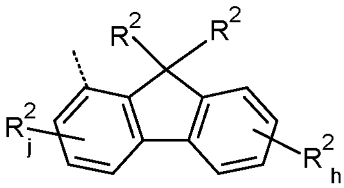
Formel (L-10)

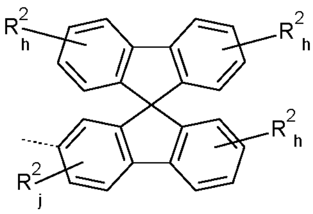
Formel (L-11)

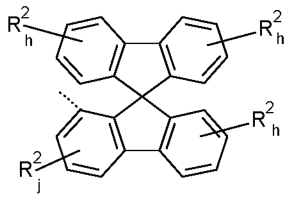
Formel (L-12)

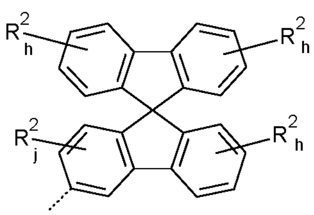
Formel (L-13)

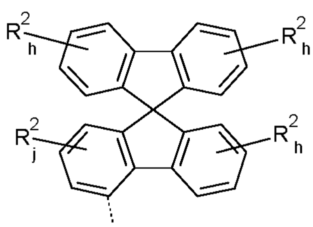
Formel (L-14)

aufweist, wobei die gestrichelte Bindung die Anbindungsposition markiert, g 0, 1, 2, 3, 4 oder 5 ist, h 0, 1, 2, 3 oder 4 ist, j 0, 1, 2 oder 3 ist, Y für O, S oder N($R^1$) steht und $R^1$ sowie $R^2$ die zuvor für Formel (I) genannten Bedeutungen aufweisen.

Vorzugsweise kann vorgesehen sein, dass die Gruppe L in Formel (I) mindestens eine Heteroarylgruppe mit einem Stickstoffatom umfasst.

Bevorzugt sind weiterhin Verbindung gemäß Formel (I), wobei die Gruppe L in Formel (I) mindestens eine Carbazol-, Diazin-, Triazin-, Benzothiophen- und/oder Benzofuran-Gruppe umfasst.

Bevorzugt sind Verbindungen umfassend Strukturen der Formel (I), wobei in Struktur gemäß Formel (I) mindestens ein Rest L für eine Gruppe steht, die ausgewählt ist aus den Formeln (L-15) bis (L-39)

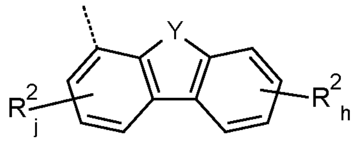

Formel (L-15)

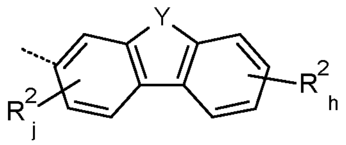

Formel (L-16)

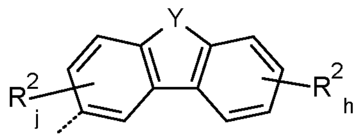

Formel ($R^1$-17)

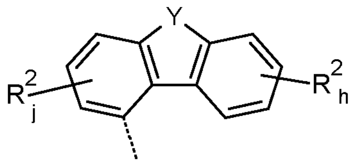

Formel (L-18)

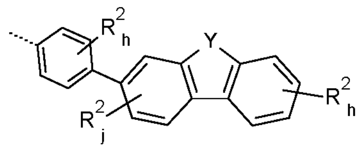

Formel (L-19)

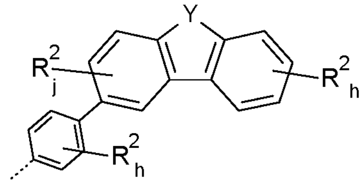

Formel ($R^1$-20)

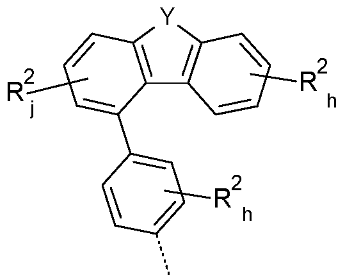

Formel (L-21)

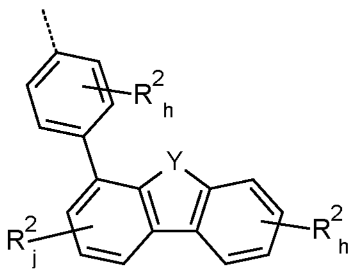

Formel (L-22)

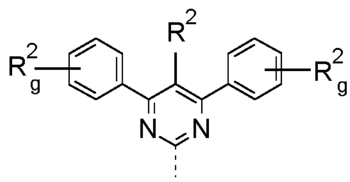

Formel (L-23)

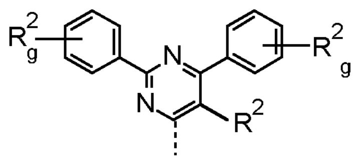

Formel (L-24)

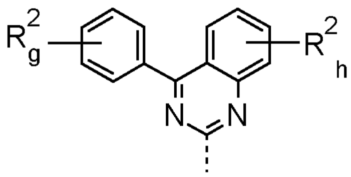

Formel (L-25)

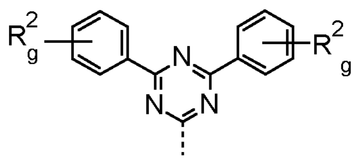

Formel (L-26)

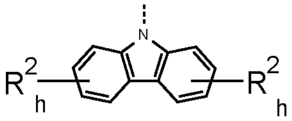

Formel (L-27)

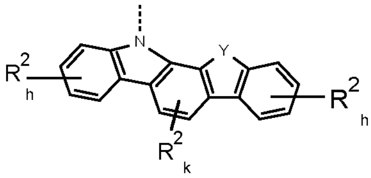

Formel (L-28)

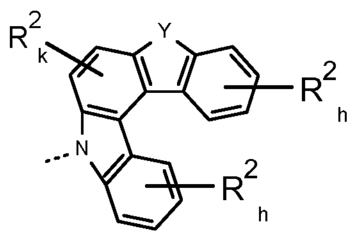

Formel (L-29)

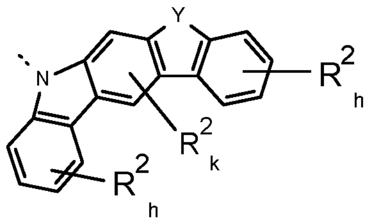

Formel (L-30)

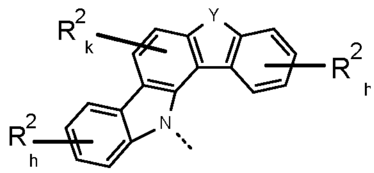

Formel (L-31)

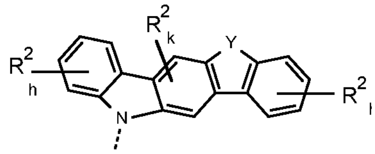

Formel (L-32)

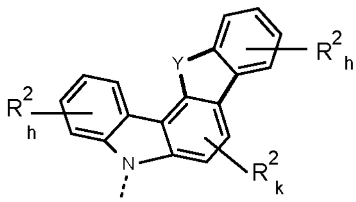

Formel (L-33)

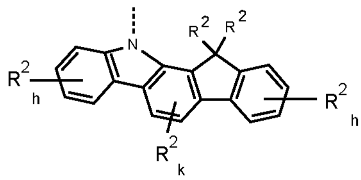

Formel (L-34)

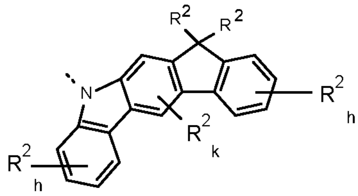

Formel (L-35)

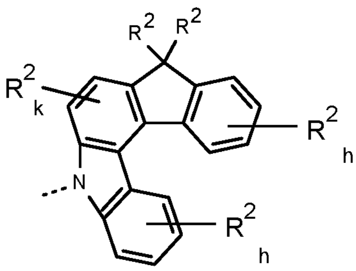

Formel (L-36)

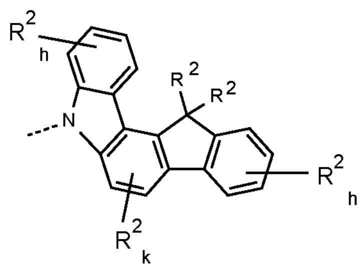

Formel (L-37)

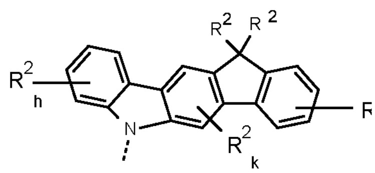

Formel (L-38)

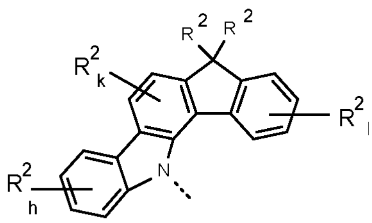

Formel (L-39)

, wobei die gestrichelte Bindung die Anbindungsposition markiert, g 0, 1, 2, 3, 4 oder 5 ist, h 0, 1, 2, 3 oder 4 ist, j 0, 1, 2 oder 3 ist, , k 0, 1 oder 2 ist, Y für O, S oder N($R^1$) steht und $R^1$ sowie $R^2$ die zuvor für Formel (I) genannten Bedeutungen aufweisen. Vorzugsweise ist die Summe der Indices g, h, j und k in einer Struktur gemäß den Formeln (L-1) bis (L-39) kleiner oder gleich 5, vorzugsweise 0, 1, 2 oder 3 und besonders bevorzugt 0 oder 1.

Bevorzugt sind Verbindungen umfassend Strukturen der Formel (I), wobei in Struktur gemäß Formel (I) mindestens ein Rest L für eine Gruppe steht, die ausgewählt ist aus den Formeln (L-40) bis (L-42)

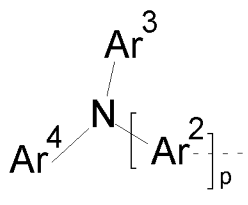

Formel (L-40)

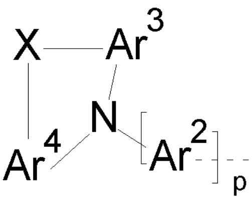

Formel (L-41)

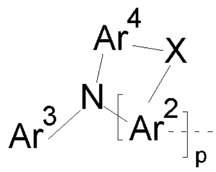

Formel (L-42)

, wobei die gestrichelte Bindung die Anbindungsposition markiert und $Ar^2$, $Ar^3$, $Ar^4$ jeweils unabhängig eine Arylgruppe mit 6 bis 40 C-Atomen oder eine Heteroarylgruppe mit 3 bis 40 C-Atomen ist, welche jeweils durch einen oder mehrere Reste $R^1$ substituiert sein kann;
p 0 oder 1 ist und
X für eine Bindung, $CR^1_2$, C=O, $N(R^1)$, $B(R^1)$, $SiR^1_2$, O oder S, vorzugsweise $CR^1_2$, C=O, $N(Ar^1)$, O oder S steht, wobei die Reste $R^1$ und $Ar^1$ die zuvor für Formel (I) genannten Bedeutungen aufweisen.

Die Struktur gemäß Formel (I) oder einer der bevorzugten Ausführungsformen dieser Struktur umfasst mindestens eine Gruppe L, wie diese zuvor näher dargelegt wurde. Die Art der funktionellen Gruppe L beieinflusst die Eigenschaften der Verbindung, wobei diese Eigenschaften über einen weiten Bereich eingestellt werden können.

Für Verbindungen, die vorzugsweise als Lochtransportmaterial (HTM), oder Elektronenblockerschicht (EBL) eingesetzt werden, kann L bevorzugt für einen Rest mit mindestens einer Diarylamino-Gruppe stehen. Bei einer Verwendung eines Carbazol-Derivats als Strukturelement der Gruppe L, werden unter anderem Verbindungen erhalten, die bevorzugt als Triplettmatrixmaterial (TMM), besonders bevorzugt als elektronenleitendes Triplettmatrixmaterial (e-TMM) oder als Lochblockierschicht (HBL) eingesetzt werden können. Steht L für ein aromatisches Sytem, insbesondere für eine Arylgruppe, die besonders bevorzugt keine Heteroatome umfasst, kann das Material mit überraschendem Vorteil als Elektronentransportmaterial (ETM) Verwendung finden. In diesem Zusammenhang ist festzuhalten, dass die erhaltenen Verbindungen im Allgemeinen ein wesentlich besseres Eigenschaftsprofil durch die Gegenwart des Benzo[c]coumarin-Strukturelement aufweisen als vergleichbare Verbindungen gemäß dem Stand der Technik. Besonders bevorzugt sind insbesondere Verbindungen, umfassend Strukturen der Formel (I), bzw. die zuvor oder nachfolgend aufgeführten bevorzugten Ausführungsformen, die als Matrixmaterial oder als Elektronentransportmaterial eingesetzt werden können.

Gemäß einer besonderen Ausführungsform der Vorrichtung der vorliegenden Erfindung kann die Gruppe L in der Struktur gemäß Formel (I) bevorzugt eine Carbazol-, Indenocarbazol-, Indolocarbazol-, Arylamin- oder eine Diarylamingruppe darstellen. Verbindungen der Formel (I) mit mindestens einer Carbazol-, Indenocarbazol-, Indolocarbazol-, Arylamin- oder eine Diarylamingruppe können bevorzugt als Matrixmaterial eingesetzt werden.

Ferner kann die Gruppe L in der Struktur gemäß Formel (I) bevorzugt eine Pyridin-, Pyrimidin-, Pyrazin-, Pyridazin-, Triazin-, Dibenzofuran-, Dibenzothiophen-, Fluoren-, Spirobifluoren-, Anthracen- oder Benzimidazolgruppe darstellen. Verbindungen der Formel (I) mit mindestens einer Pyridin-, Pyrimidin-, Pyrazin-, Pyridazin-, Triazin-, Dibenzofuran-, Dibenzothiophen-, Fluoren-, Spirobifluoren-, Anthracen- oder Benzimidazolgruppe können mit Vorteil als Elektronentransportmaterial (ETM) verwendet werden.

Neben mindestens einer Gruppe L kann eine Struktur gemäß Formel (I) weitere Substituenten umfassen, die zwar der Definition von R oder $R^1$ entsprechen, nicht aber der zuvor, beispielsweise für Formel (I) dargelegten Definition von L. Besondere bevorzugt umfasst eine Struktur gemäß Formel (I) höchstens drei, bevorzugt höchstens zwei Reste R und/oder $R^1$, die nicht der Definition von L entsprechen. Speziell bevorzugt umfasst eine Struktur gemäß Formel (I) höchstens einen Rest R und/oder $R^1$, der nicht der Definition von L entspricht. Besonders bevorzugt gemäß der Erfindung entsprechen alle Reste R und/oder $R^1$ der Definition von L.

Weiterhin sind Verbindungen gemäß Formel (I) bevorzugt, in denen die Struktur gemäß Formel (I) höchstens eine reaktive Gruppe umfasst. Vorzugsweise ist die reaktive Gruppe ausgewählt aus Triflat, Brom, Iod, Chlor, Boronsäure oder Boronsäureester, besonders bevorzugt aus Br, Cl und $B(OR^2)_2$.

Besonders bevorzugte Verbindungen umfassen Strukuren gemäß den folgenden Formeln (II), (III), (IV), (V), (VI), (VII), (VIII) und/oder (IX)

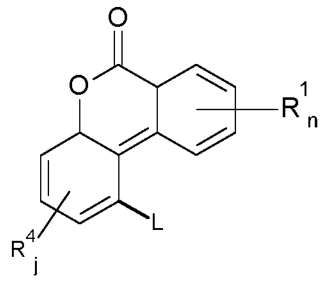

Formel (II)

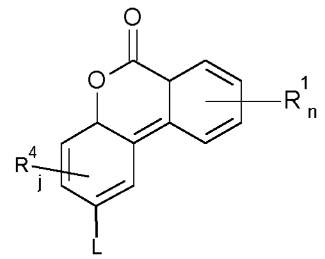

Formel (III)

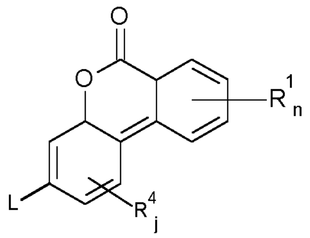

Formel (IV)

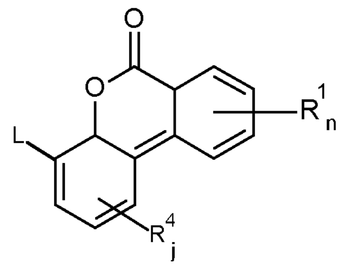

Formel (V)

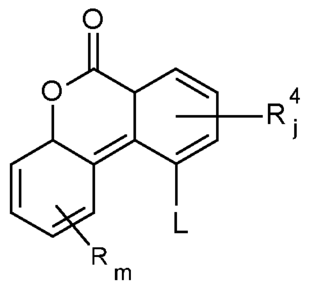

Formel (VI)

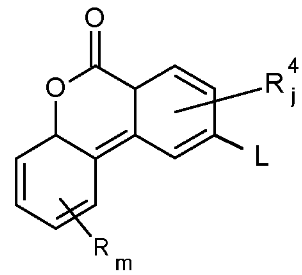

Formel (VII)

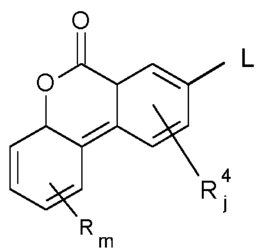

Formel (VIII)

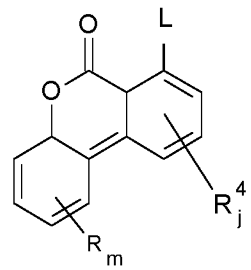

Formel (IX)

, wobei die dargelegten Symbole R, $R^1$, L und die Indices m und n die zuvor für Formel (I) dargelegten Bedeutungen haben, der Index j 0, 1, 2 oder 3, vorzugsweise 0, 1 oder 2 und besonders bevorzugt 0 oder 1 bedeutet und $R^4$ bei jedem Auftreten gleich oder verschieden H, D, F, Cl, Br, I, CHO, $C(=O)Ar^1$, $P(=O)(Ar^1)2$, $S(=O)Ar^1$, $S(=O)_2Ar^1$, CN, $NO_2$, $Si(R^2)_3$, $B(OR^2)_2$, $OSO_2R^2$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C=C , $Si(R^2)_2$, $Ge(R^2)_2$, $Sn(R^2)_2$, C=O, C=S, C=Se, $P(=O)(R^2)$, SO, $SO_2$, O, S oder $CONR^2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, wobei die Reste $R^2$ und $Ar^1$ die zuvor

für Formel (I) genannte Bedeutung aufweisen, oder eine Kombination dieser Systeme ist; dabei können zwei oder mehrere benachbarte Substituenten $R^4$ auch miteinander, mit dem Ring an dem $R^4$ gebunden ist, mit einem Ring zu dem $R^4$ benachbart ist oder einem Rest R oder $R^1$ ein mono- oder polycyclisches, aliphatisches oder aromatisches Ringsystem bilden.

Besonders bevorzugte Verbindungen umfassen Strukuren gemäß den folgenden Formeln (X), (XI), (XII), (XIII), (XIV), (XV), (XVI) und/oder (XVII)

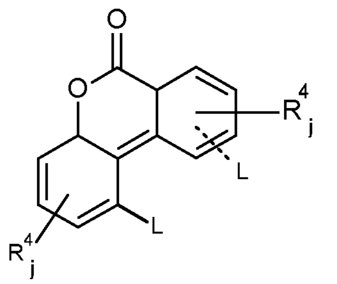

Formel (X)

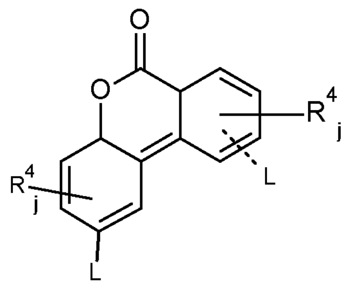

Formel (XI)

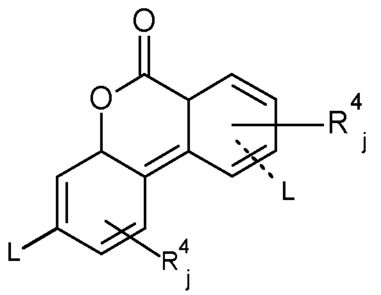

Formel (XII)

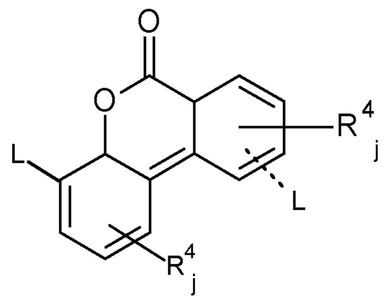

Formel (XIII)

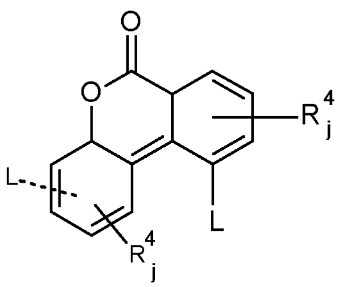

Formel (XIV)

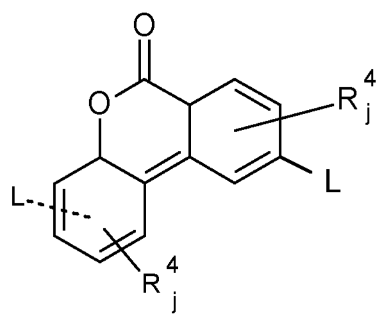

Formel (XV)

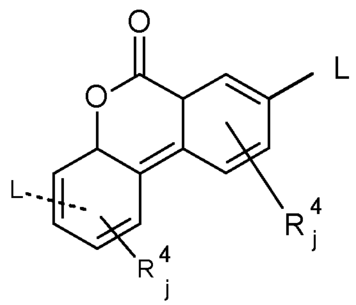

Formel (XVI)

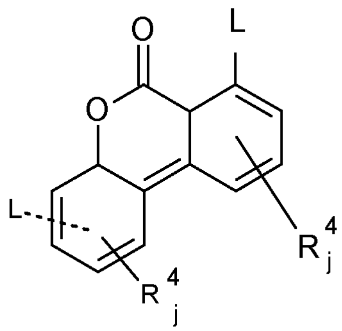

Formel (XVII)

, wobei die dargelegten Symbole R, $R^1$, L und die Indices m und n die zuvor für Formel (I) dargelegten Bedeutungen haben, der Index bei jedem Auftreten gleich oder verschieden j 0, 1, 2 oder 3 bedeutet und

$R^4$ bei jedem Auftreten gleich oder verschieden H, D, F, Cl, Br, I, CHO, $C(=O)Ar^1$, $P(=O)(Ar^1)2$, $S(=O)Ar^1$, $S(=O)_2Ar^1$, CN, $NO_2$, $Si(R_2)_3$, $B(OR^2)_2$, $OSO_2R^2$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C=C , $Si(R^2)_2$, $Ge(R^2)_2$, $Sn(R^2)_2$, C=O, C=S, C=Se, $P(=O)(R^2)$, SO, $SO_2$, O, S oder $CONR^2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, wobei die Reste $R^2$ und $Ar^1$ die für Formel (I) genannten Bedeutungen aufweisen, oder eine Kombination dieser Systeme ist; dabei können zwei oder mehrere benachbarte Substituenten $R^4$ auch miteinander, mit dem Ring an dem $R^4$ gebunden ist oder mit einem Ring zu dem $R^4$ benachbart ist ein mono- oder polycyclisches, aliphatisches oder aromatisches Ringsystem bilden.

Vorzugsweise kann vorgesehen sein, dass der Rest $R^4$ in den Formeln (II), (III), (IV), (V), (VI), (VII), (VIII) und/oder (IX) sowie den Formeln (X), (XI), (XII), (XIII), (XIV), (XV), (XVI) und/oder (XVII) keine Gruppe darstellt, die der Definition des Rests L in Formel (I) entspricht.

Vorzugsweise kann die Verbindung mit Strukturen gemäß Formel (I) Reste R und/oder $R^1$ umfassen, bei denen diese Reste R und/oder $R^1$ bei jedem Auftreten gleich oder verschieden bevorzugt ausgewählt sind aus der Gruppe bestehend aus H, D, F, Br, I, CN, $Si(R^2)_3$, $B(OR^2)_2$, $C(=O)Ar^1$, einer geradkettigen Alkylgruppe mit 1 bis 10 C-Atomen oder einer geradkettigen Alkoxygruppe mit 1 bis 10 C-Atomen oder einer Alkenylgruppe mit 2 bis 10 C-Atomen oder einer verzweigten oder cyclischen Alkoxygruppe mit 3 bis 10 C-Atomen oder einer verzweigten oder cyclischen Alkylgruppe mit 3 bis 10 C-Atomen, die jeweils mit einem oder mehreren Resten $R^2$ substituiert sein kann, wobei ein oder mehrere H-Atome durch D oder F ersetzt sein können, oder einem aromatischen oder heteroaromatischen Ringsystem mit 5 bis 30 aromatischen Ringatomen, das jeweils durch einen oder mehrere Reste $R^2$ substituiert sein kann; dabei können zwei benachbarte Reste R bzw. $R^1$ oder R bzw. $R^1$ mit $R^2$ auch miteinander ein mono- oder polycyclisches, aliphatisches oder aromatisches Ringsystem bilden. Besonders bevorzugt sind diese Reste R oder $R^1$ bei jedem Auftreten gleich oder verschieden ausgewählt aus der Gruppe bestehend aus H, D, F, einer geradkettigen Alkoxygruppe mit 1 bis 6 C-Atomen oder einer verzweigten oder cyclischen Alkoxygruppe mit 3 bis 10 C-Atomen, einer geradkettigen Alkylgruppe mit 1 bis 6 C-Atomen oder einer verzweigten oder cyclischen Alkylgruppe mit 3 bis 10 C-Atomen, wobei ein oder mehrere H-Atome durch D oder F ersetzt sein können, oder einem aromatischen oder heteroaromatischen Ringsystem mit 5 bis 24 aromatischen Ringatomen, das jeweils durch einen oder mehrere Reste $R^2$ substituiert sein kann; dabei können zwei benachbarte Reste R bzw. $R^1$ oder R bzw. $R^1$ mit $R^2$ auch miteinander ein mono- oder polycyclisches, aliphatisches oder aromatisches Ringsystem bilden. Besonders bevorzugt kann mindestens einer der Reste R oder $R^1$ in Formel (I) eine Arylgruppe oder eine Heteroarylgruppe mit 6 bis 18 Kohlenstoffatomen darstellen, die mit bis zu drei Resten $R^2$ substituiert sein kann.

Vorzugsweise kann die Verbindung mit Strukturen gemäß Formel (I) Reste $R^2$ umfassen, bei denen diese Reste $R^2$ bei jedem Auftreten gleich oder verschieden bevorzugt ausgewählt sind aus der Gruppe bestehend aus H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^3)_3$, $B(OR^3)_2$, $OSO_2R^3$, eine geradkettige Alkyl-, Alkoxy- oder Thioalkoxygruppe mit 1 bis 10 C-Atomen oder eine verzweigte oder cyclische Alkyl-, Alkoxy- oder Thioalkoxygruppe mit 3 bis 10 C-Atomen, die jeweils mit einem oder mehreren Resten $R^3$ substituiert sein kann, wobei eine oder mehrere nicht benachbarte $CH_2$-Gruppen durch C=C , $Si(R^3)_2$, $Ge(R^3)_2$, $Sn(R^3)_2$, C=O, C=S, C=Se, $P(=O)(R^3)$, SO, $SO_2$, O, S oder $CONR^3$ ersetzt sein können und wobei ein oder mehrere H-Atome durch D, F, Cl, Br, I, CN oder

$NO_2$ ersetzt sein können,
oder ein aromatisches oder heteroaromatisches Ringsystem mit 5 bis 24 aromatischen Ringatomen, das jeweils durch einen oder mehrere Reste $R^3$ substituiert sein kann, oder eine Aryloxy- oder Heteroaryloxygruppe mit 5 bis 24 aromatischen Ringatomen, die durch einen oder mehrere Reste $R^3$ substituiert sein kann, oder eine Kombination dieser Systeme; dabei können zwei oder mehrere benachbarte Substituenten $R^2$ auch miteinander ein mono- oder polycyclisches, aliphatisches oder aromatisches Ringsystem bilden. Besonders bevorzugt kann mindestens einer der Reste $R^2$ in Formel (I) eine Arylgruppe oder eine Heteroarylgruppe mit 6 bis 18 Kohlenstoffatomen darstellen, die mit bis zu drei Resten $R^3$ substituiert sein kann.

Besonders bevorzugte Verbindungen umfassen Strukuren gemäß den folgenden Formeln 1 bis 244:

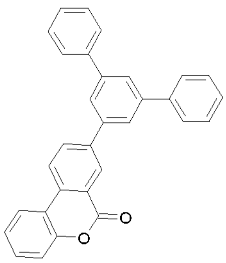

Formel 1

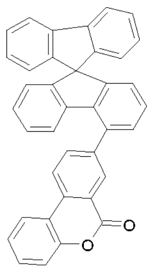

Formel 2

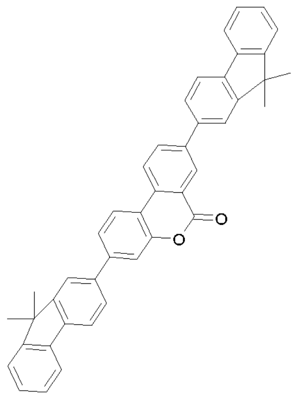

Formel 3

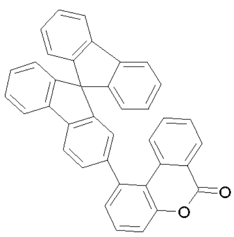

Formel 4

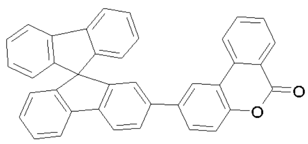

Formel 5

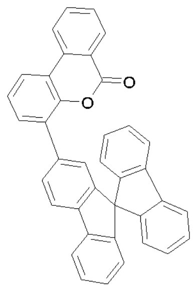

Formel 6

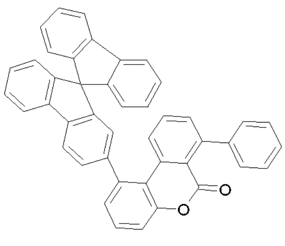

Formel 7

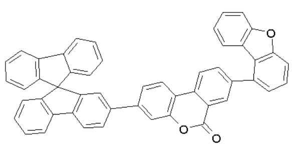

Formel 8

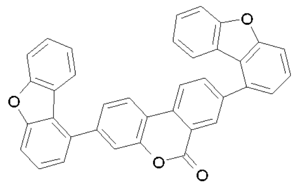

Formel 9

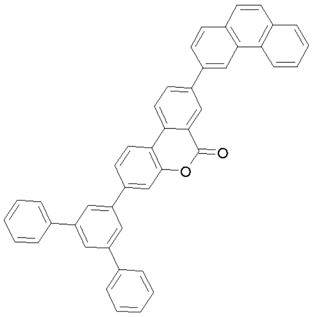

Formel 10

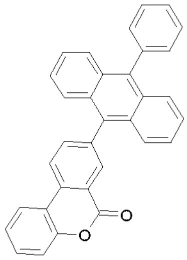

Formel 11

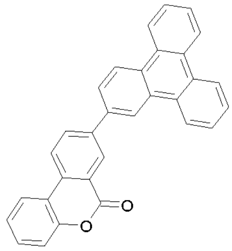

Formel 12

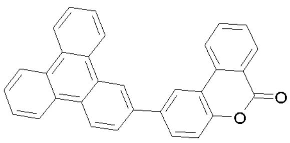

Formel 13

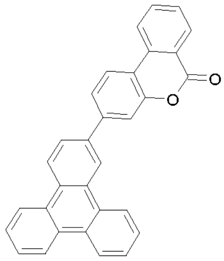

Formel 14

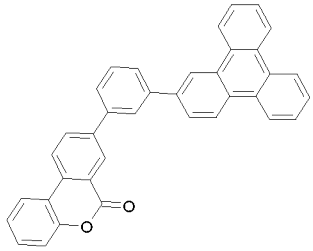

Formel 15

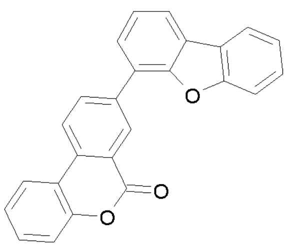

Formel 16

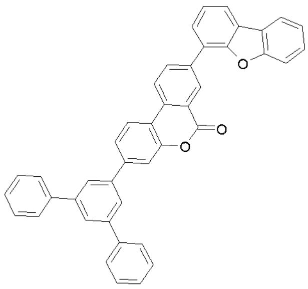

Formel 17

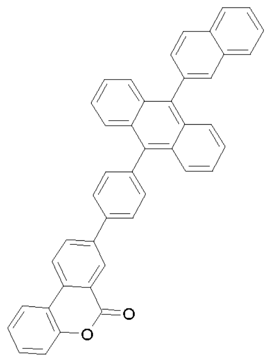

Formel 18

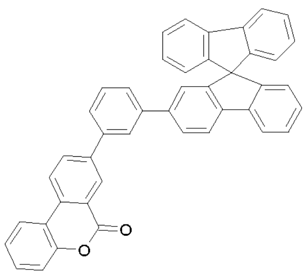

Formel 19

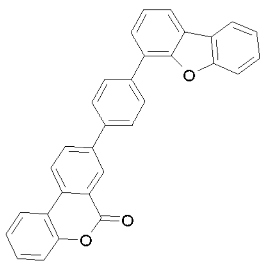

Formel 20

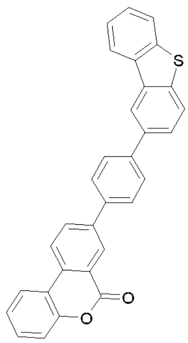

Formel 21

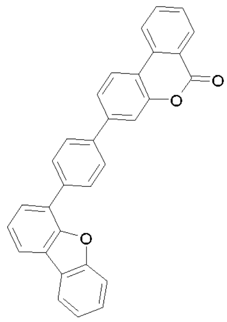
Formel 22

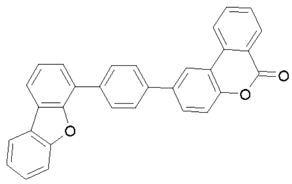
Formel 23

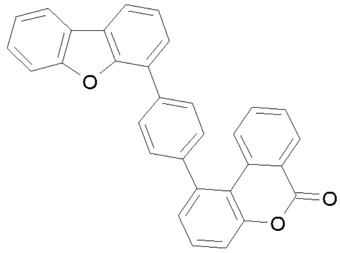
Formel 24

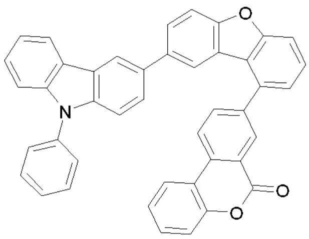
Formel 25

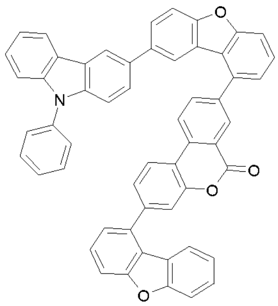
Formel 26

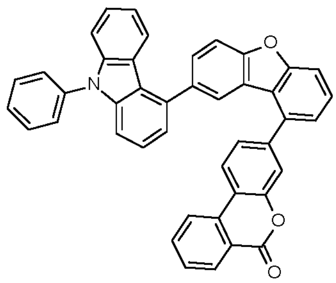
Formel 27

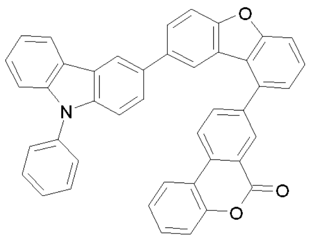
Formel 28

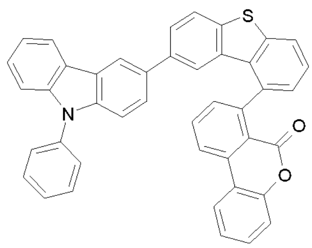
Formel 29

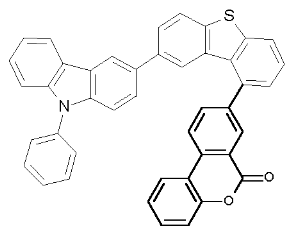
Formel 30

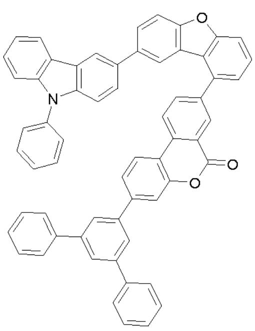
Formel 31

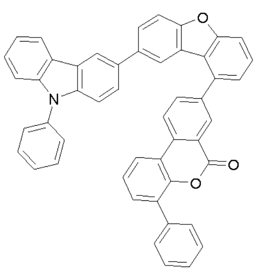
Formel 32

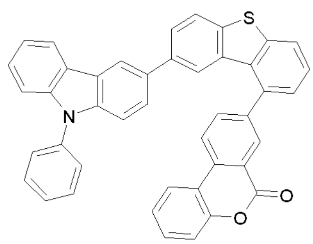
Formel 33

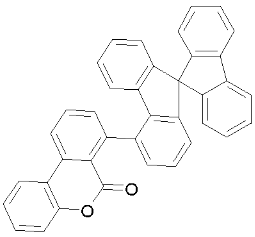
Formel 34

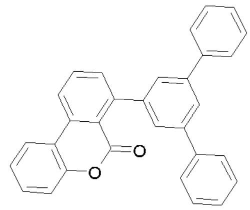
Formel 35

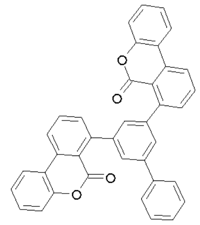
Formel 36

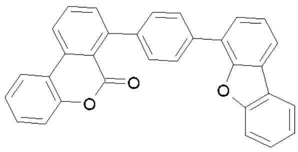
Formel 37

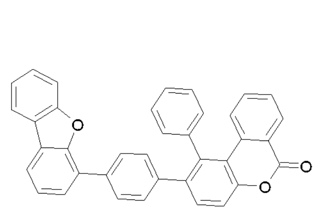
Formel 38

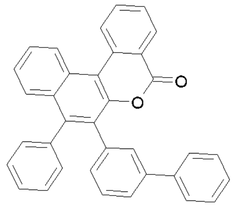
Formel 39

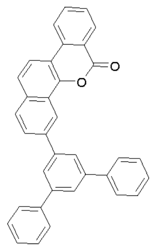
Formel 40

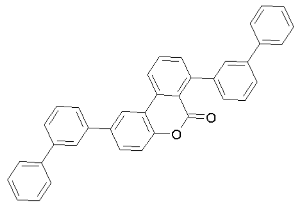
Formel 41

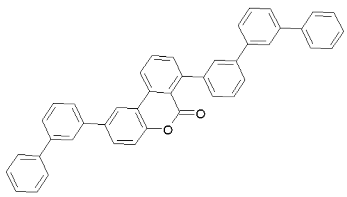
Formel 42

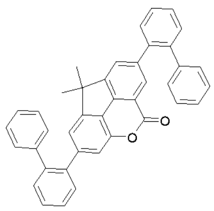
Formel 43

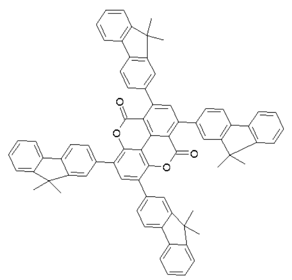
Formel 44

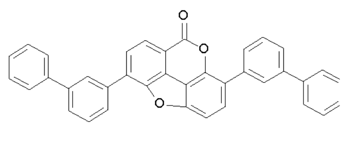
Formel 45

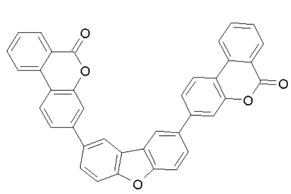
Formel 46

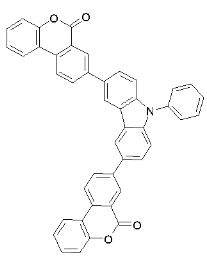
Formel 47

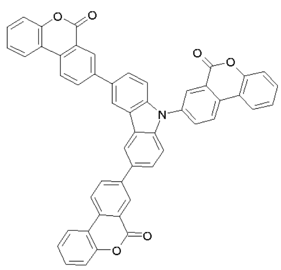
Formel 48

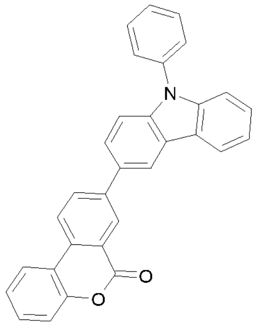

Formel 49

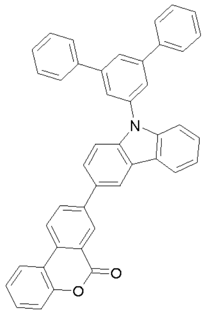

Formel 50

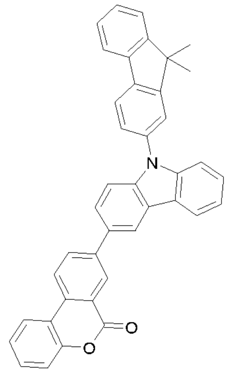

Formel 51

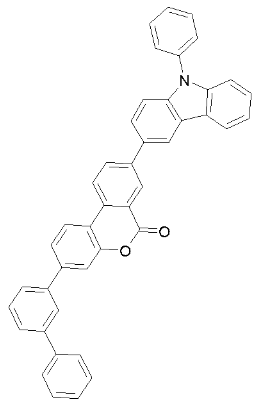

Formel 52

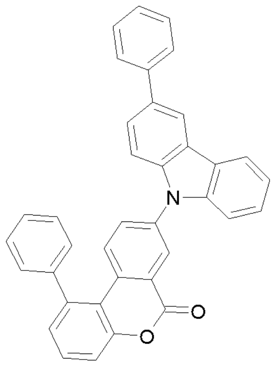

Formel 53

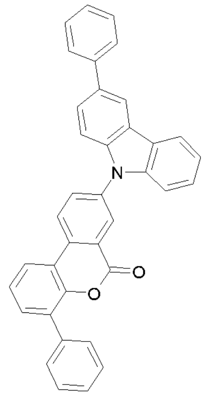

Formel 54

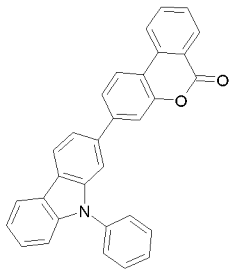

Formel 55

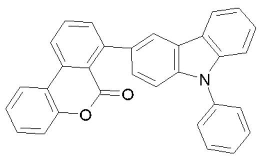

Formel 56

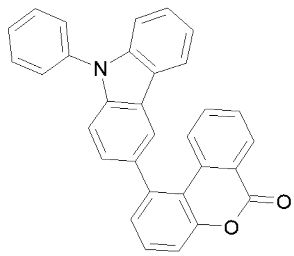

Formel 57

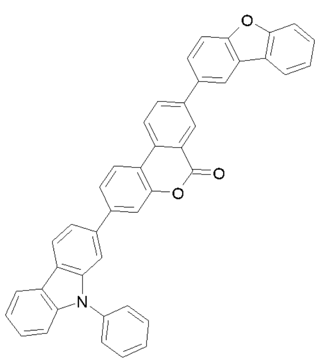

Formel 58

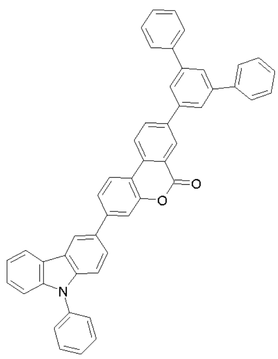

Formel 59

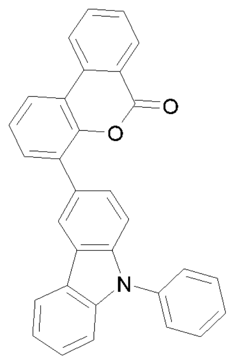

Formel 60

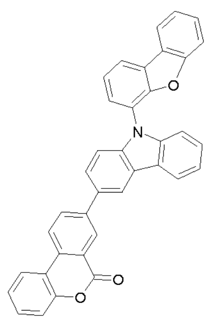
Formel 61

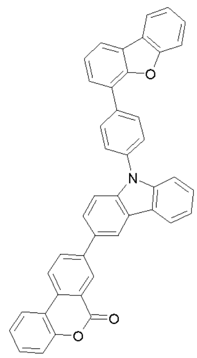
Formel 62

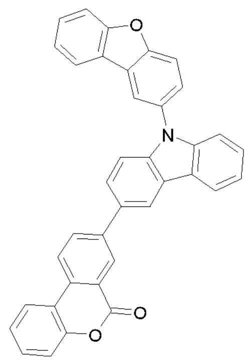
Formel 63

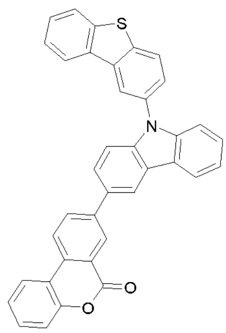
Formel 64

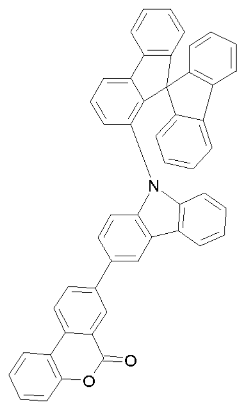
Formel 65

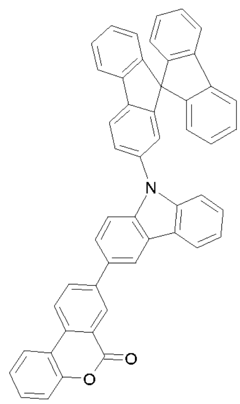
Formel 66

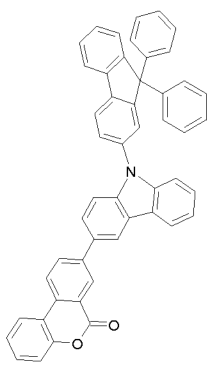
Formel 67

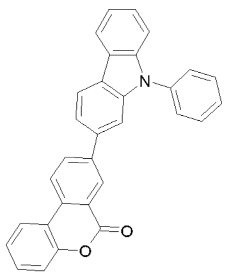
Formel 68

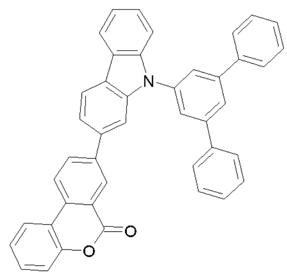
Formel 69

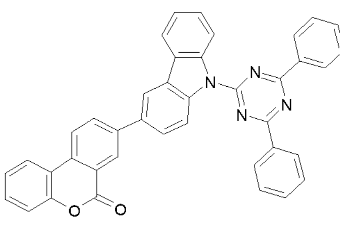
Formel 70

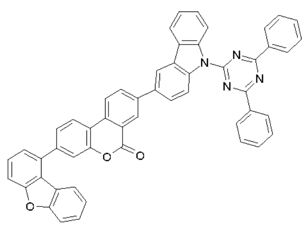
Formel 71

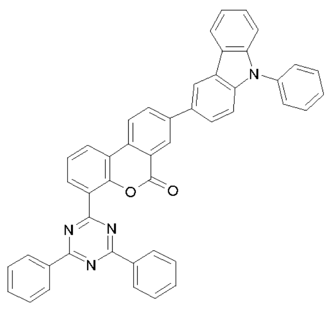
Formel 72

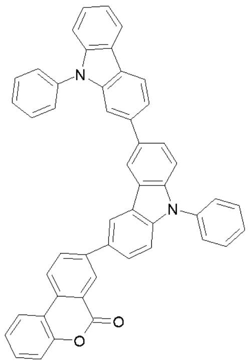

Formel 73

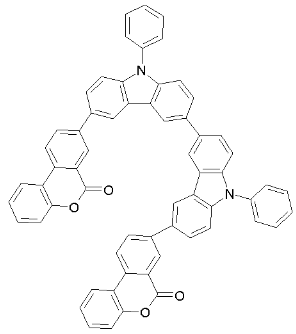

Formel 74

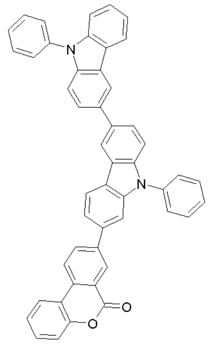

Formel 75

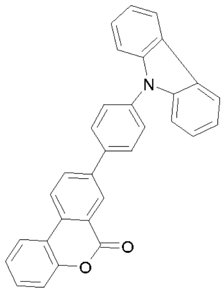

Formel 76

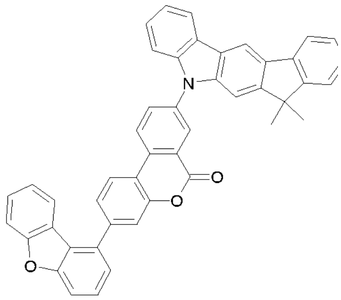

Formel 77

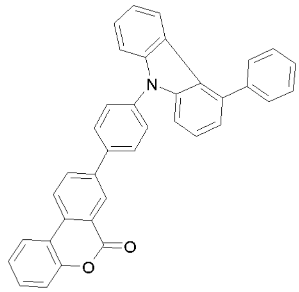

Formel 78

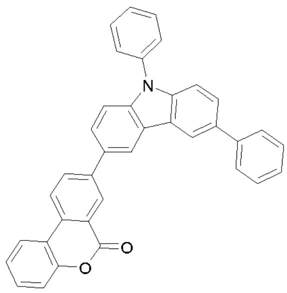

Formel 79

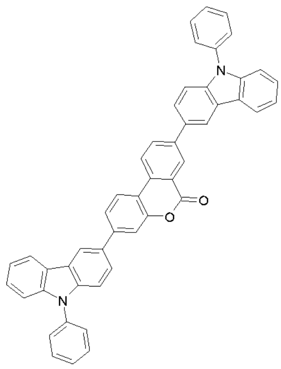

Formel 80

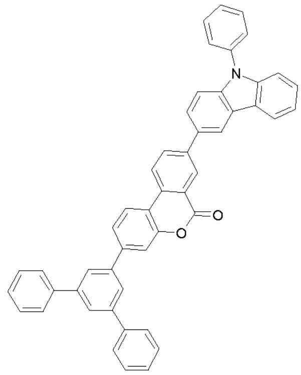

Formel 81

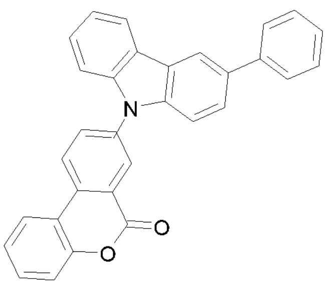

Formel 82

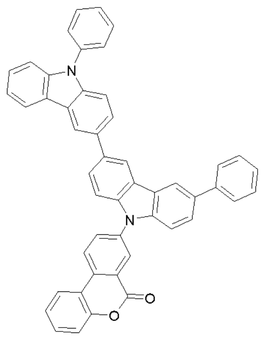

Formel 83

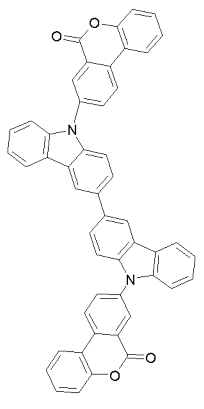

Formel 84

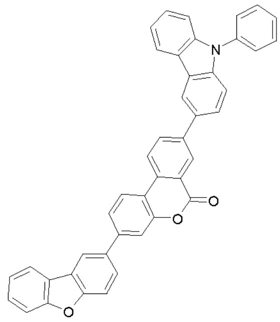

Formel 85

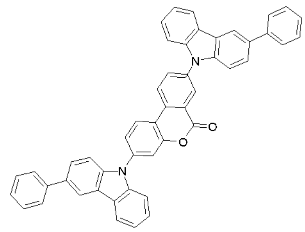

Formel 86

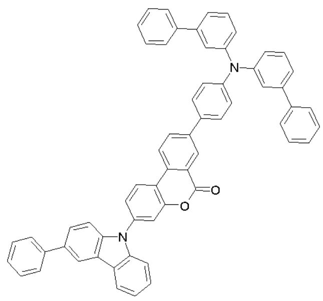

Formel 87

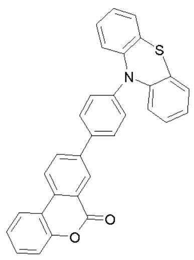

Formel 88

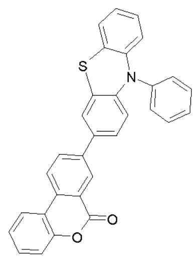

Formel 89

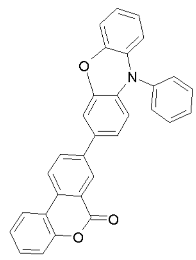

Formel 90

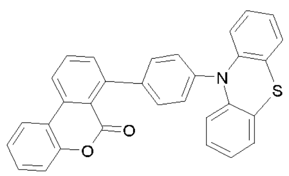

Formel 91

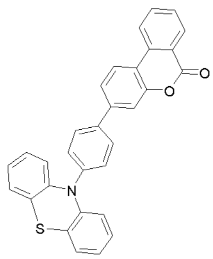

Formel 92

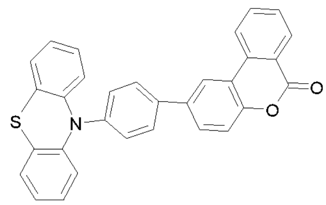

Formel 93

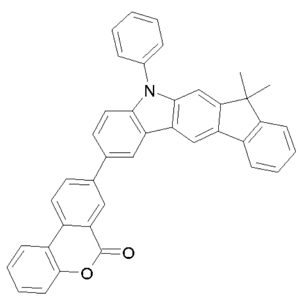

Formel 94

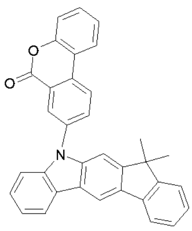

Formel 95

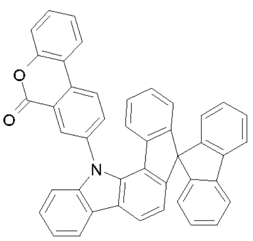

Formel 96

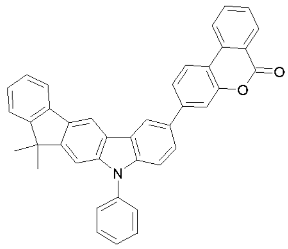

Formel 97

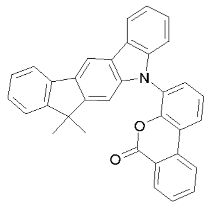

Formel 98

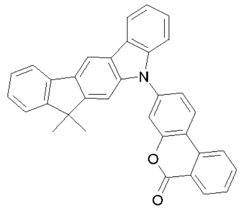

Formel 99

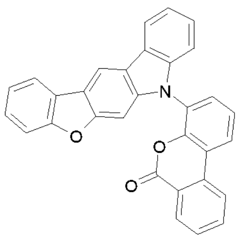

Formel 100

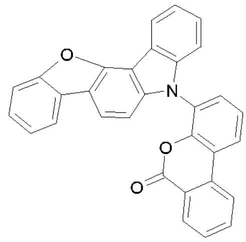

Formel 101

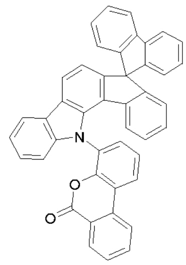

Formel 102

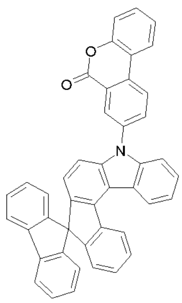

Formel 103

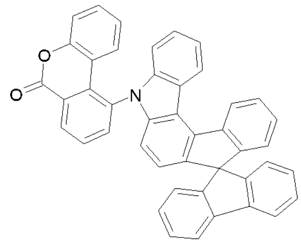

Formel 104

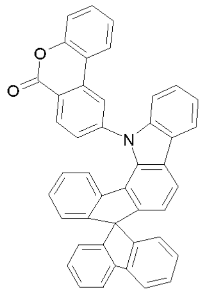

Formel 105

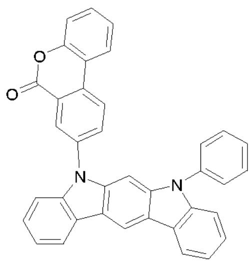

Formel 106

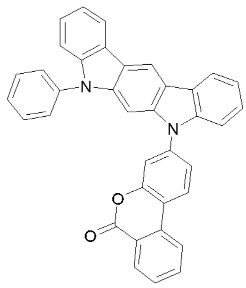

Formel 107

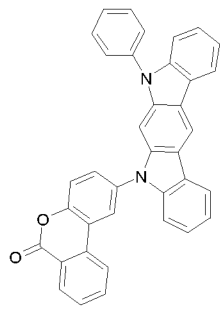

Formel 108

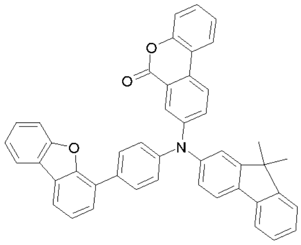

Formel 109

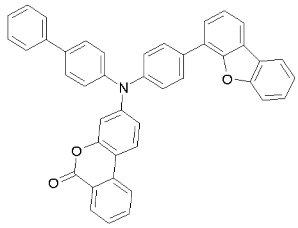

Formel 110

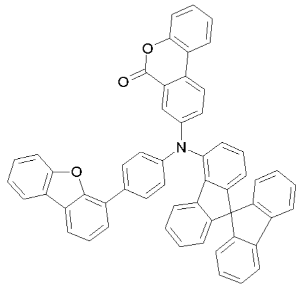

Formel 111

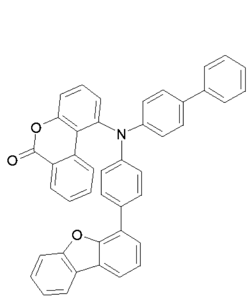

Formel 112

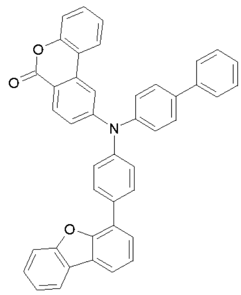

Formel 113

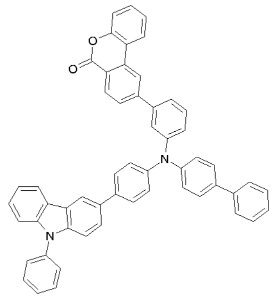

Formel 114

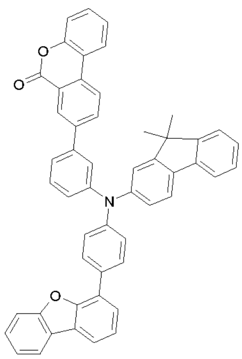

Formel 115

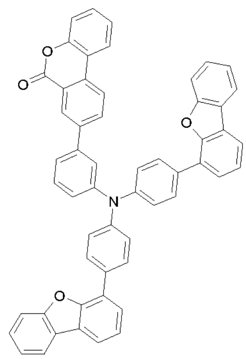

Formel 116

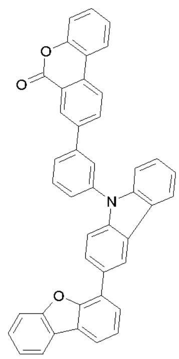

Formel 117

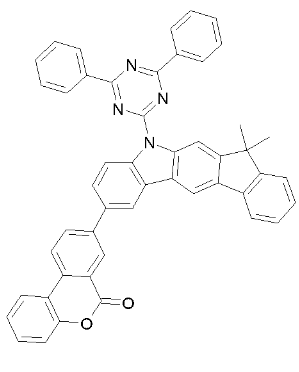

Formel 118

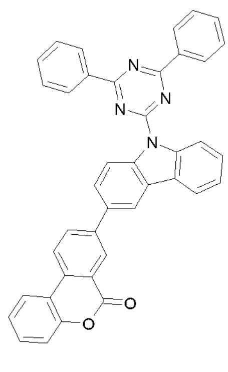

Formel 119

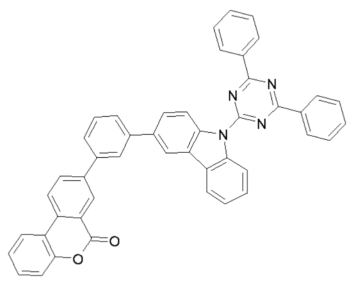

Formel 120

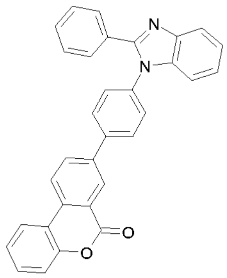

Formel 121

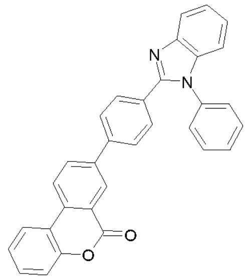

Formel 122

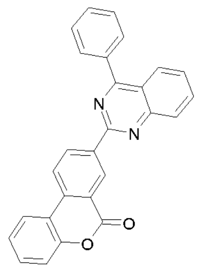

Formel 123

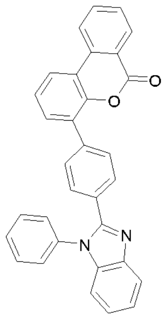

Formel 124

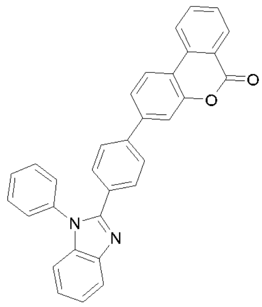

Formel 125

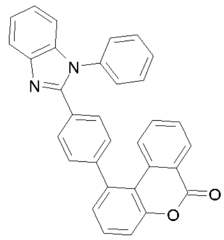

Formel 126

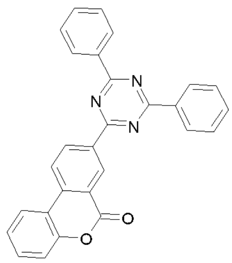

Formel 127

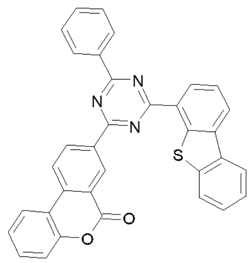

Formel 128

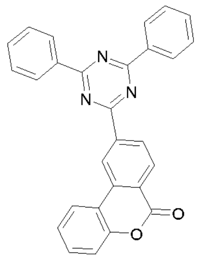

Formel 129

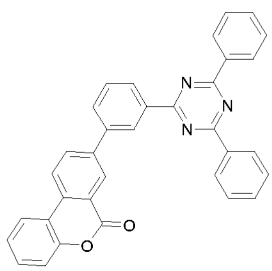

Formel 130

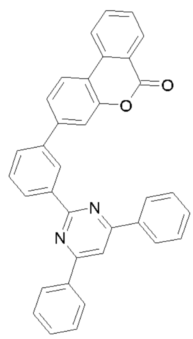

Formel 131

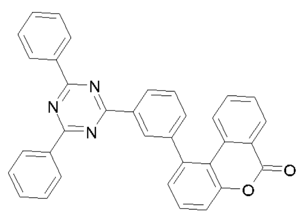

Formel 132

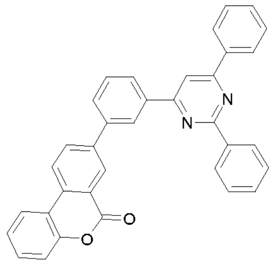
Formel 133

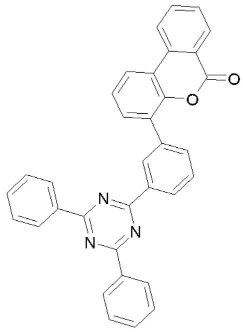
Formel 134

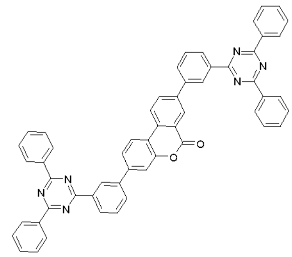
Formel 135

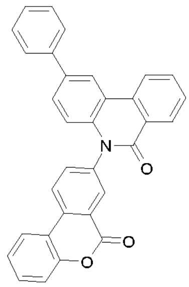
Formel 136

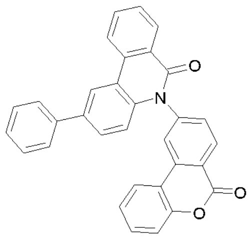
Formel 137

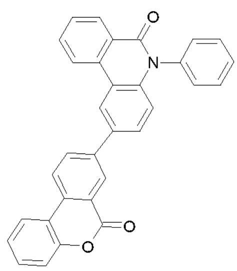
Formel 138

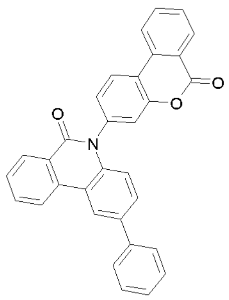
Formel 139

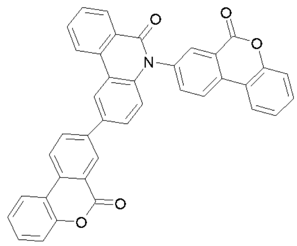
Formel 140

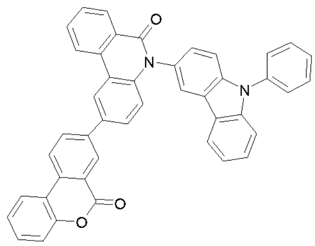
Formel 141

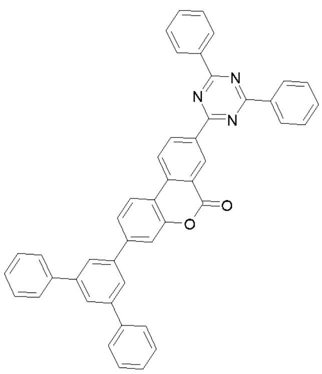
Formel 142

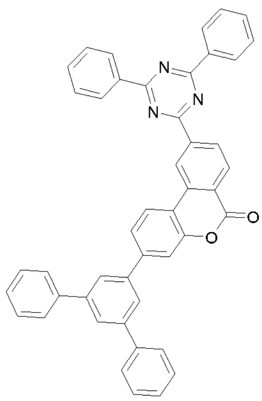
Formel 143

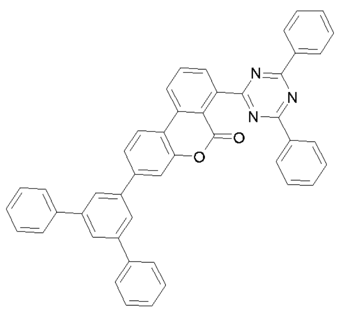
Formel 144

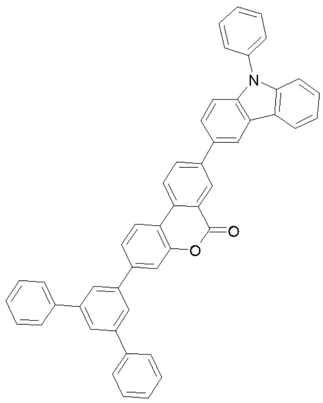

Formel 145

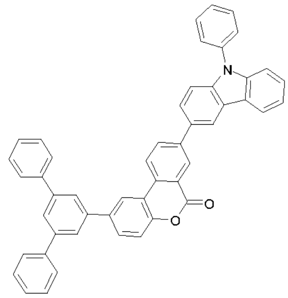

Formel 146

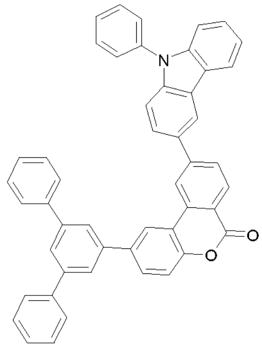

Formel 147

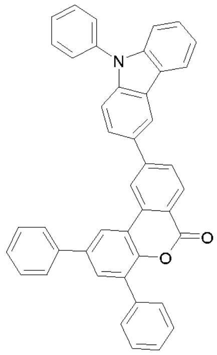

Formel 148

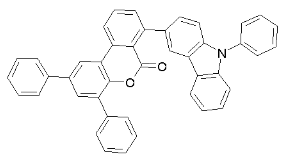

Formel 149

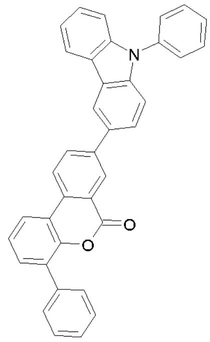

Formel 150

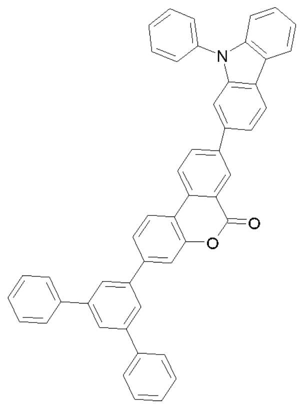

Formel 151

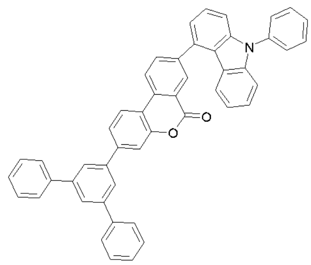

Formel 152

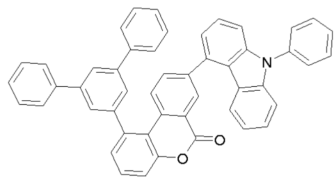

Formel 153

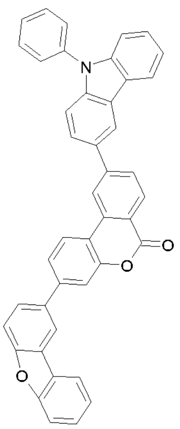

Formel 154

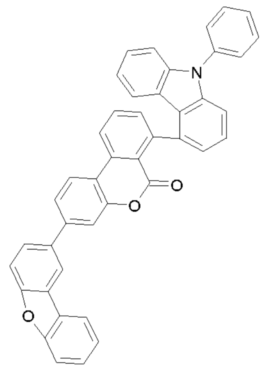

Formel 155

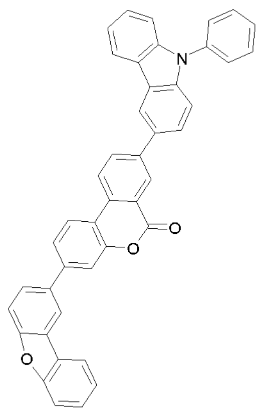

Formel 156

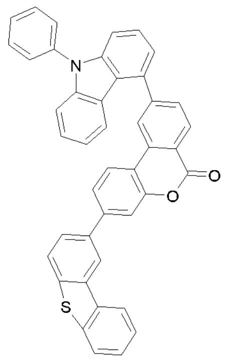

Formel 157

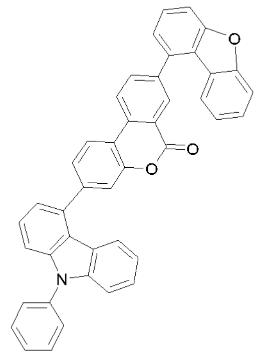

Formel 158

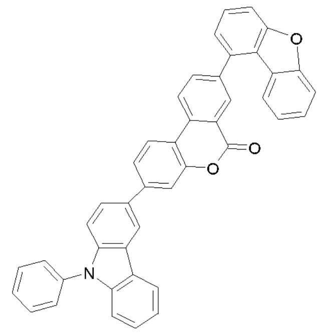

Formel 159

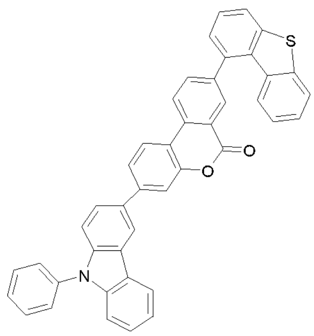

Formel 160

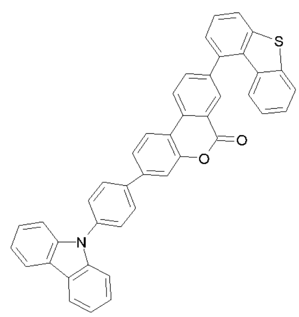

Formel 161

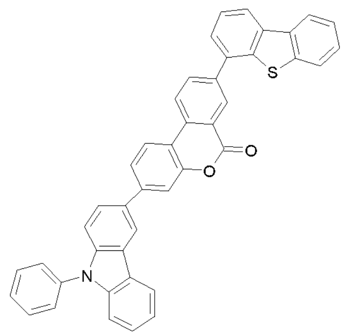

Formel 162

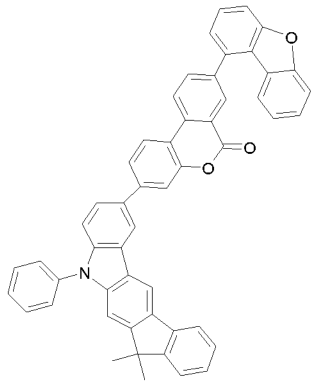

Formel 163

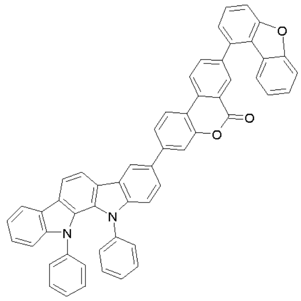

Formel 164

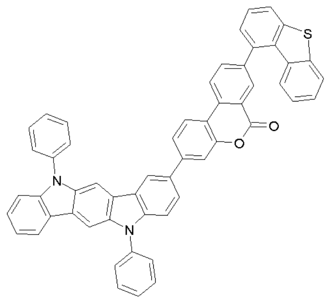

Formel 165

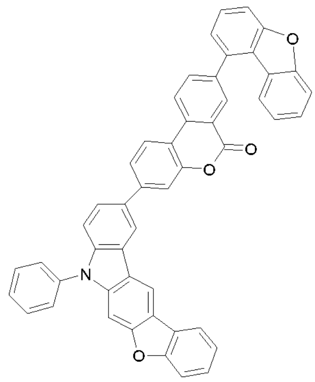

Formel 166

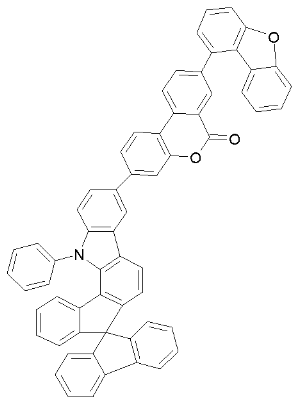

Formel 167

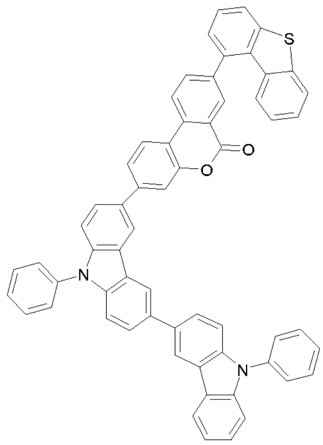

Formel 168

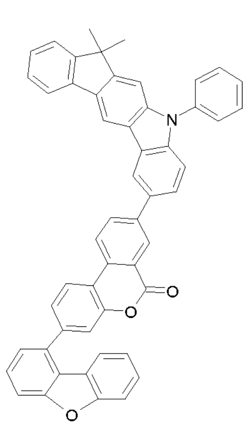

Formel 169

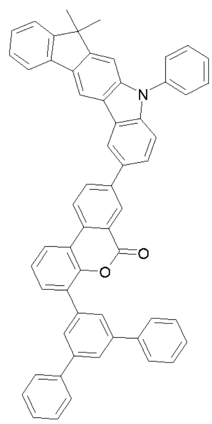

Formel 170

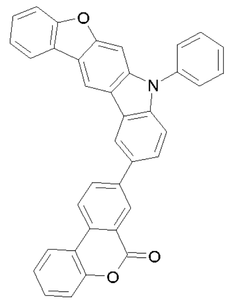

Formel 171

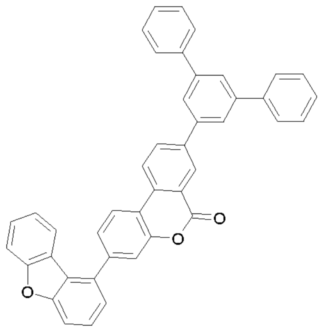

Formel 172

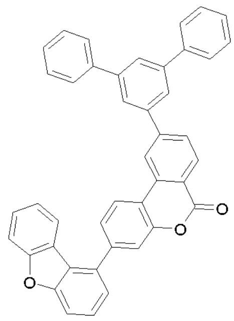

Formel 173

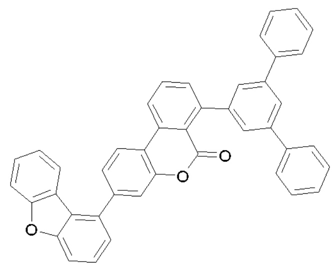

Formel 174

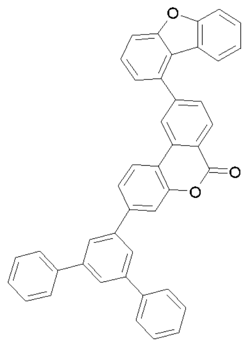

Formel 172

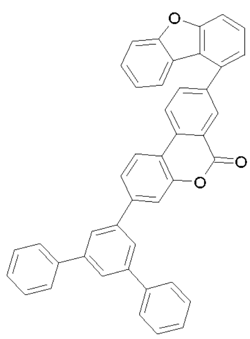

Formel 173

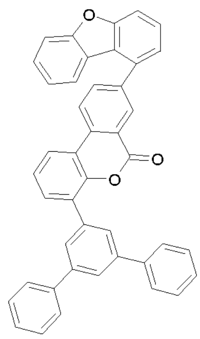

Formel 174

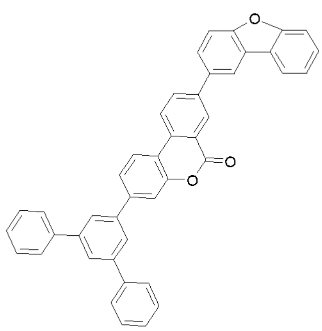

Formel 175

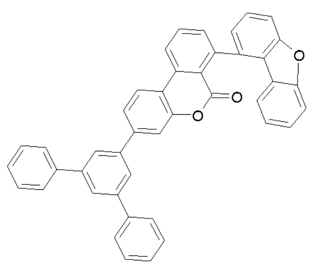

Formel 176

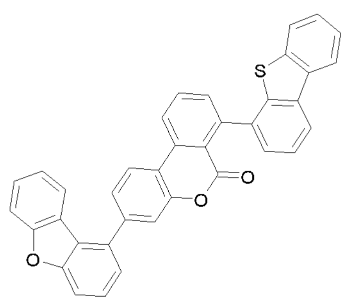

Formel 177

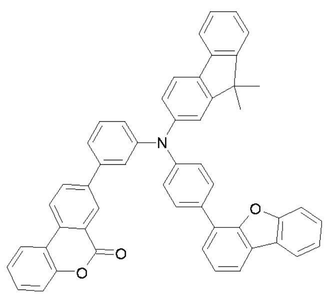

Formel 178

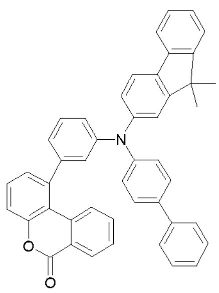

Formel 179

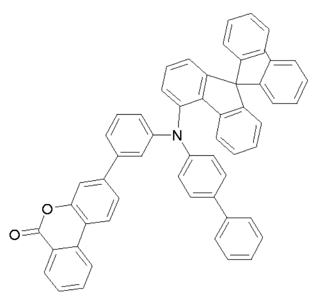

Formel 180

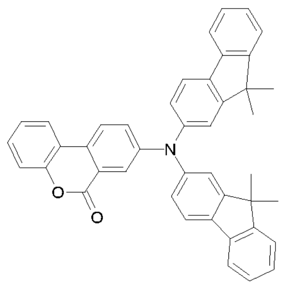

Formel 181

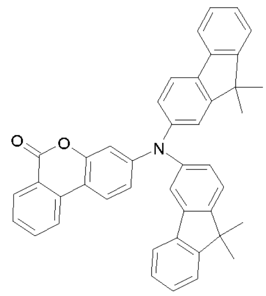

Formel 182

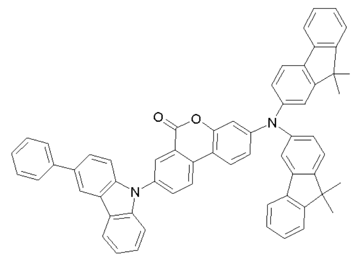

Formel 183

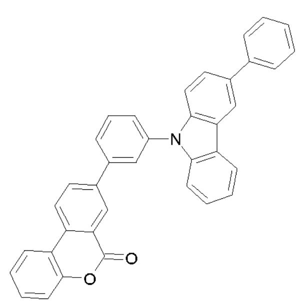

Formel 184

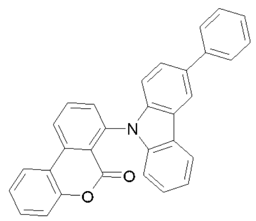

Formel 185

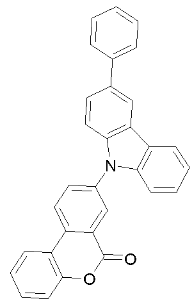

Formel 186

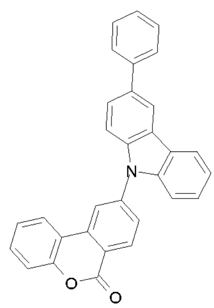

Formel 187

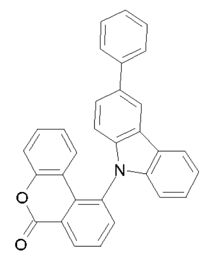

Formel 188

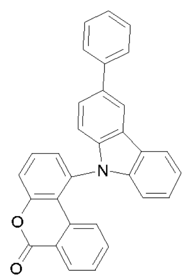

Formel 189

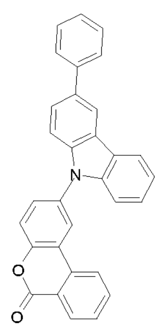

Formel 190

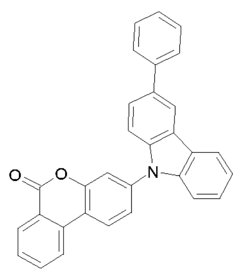

Formel 191

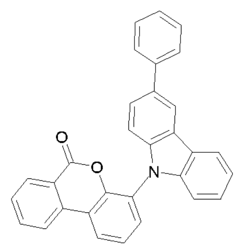

Formel 192

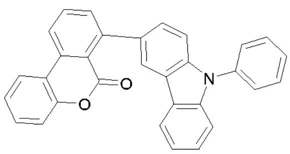

Formel 193

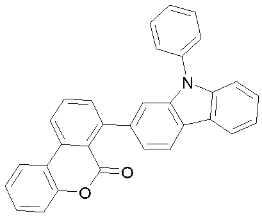

Formel 194

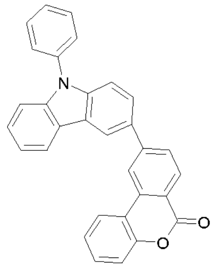

Formel 195

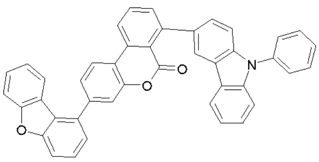

Formel 196

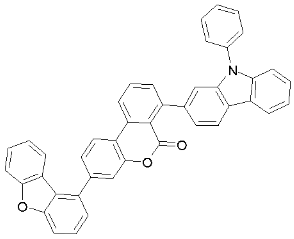

Formel 197

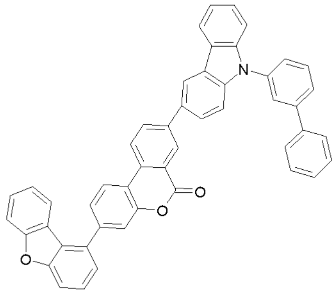

Formel 198

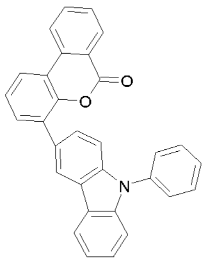

Formel 199

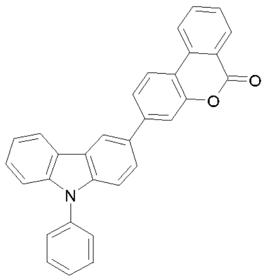

Formel 200

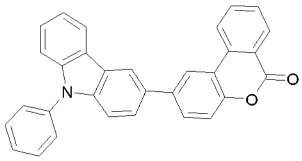

Formel 201

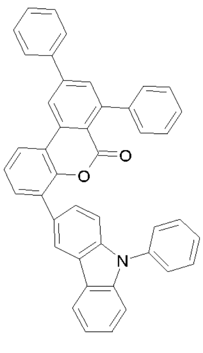

Formel 202

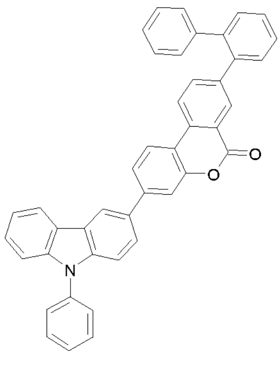

Formel 203

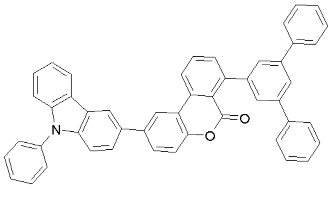

Formel 204

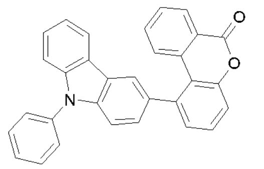

Formel 205

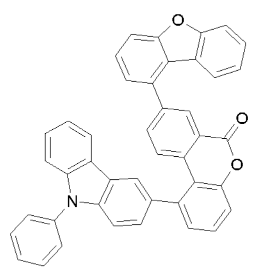

Formel 206

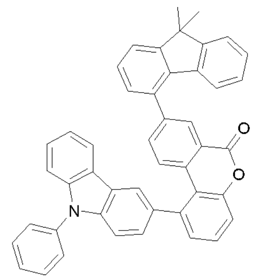

Formel 207

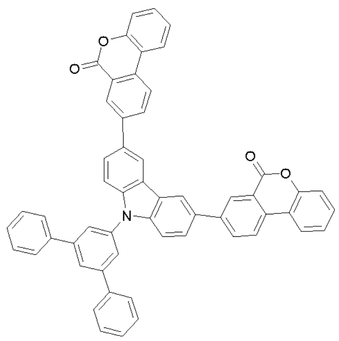

Formel 208

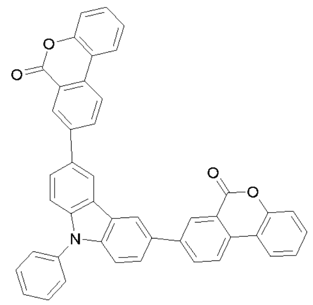

Formel 209

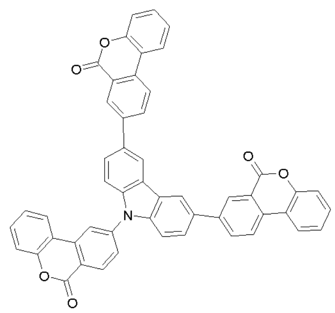

Formel 210

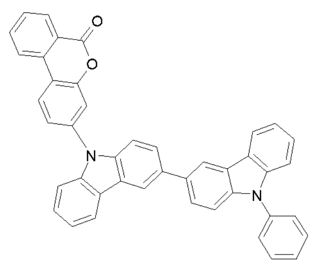

Formel 211

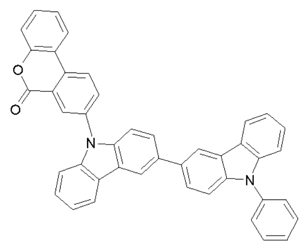

Formel 212

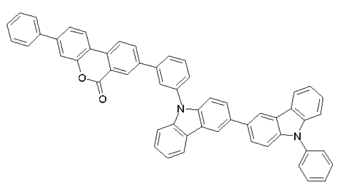

Formel 213

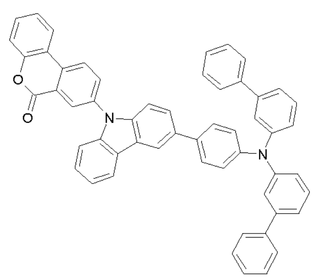

Formel 214

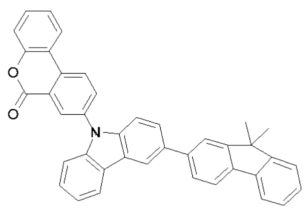

Formel 215

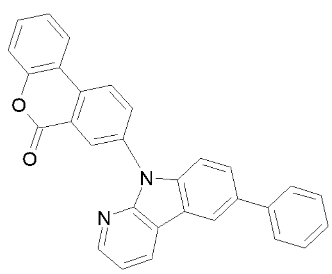

Formel 216

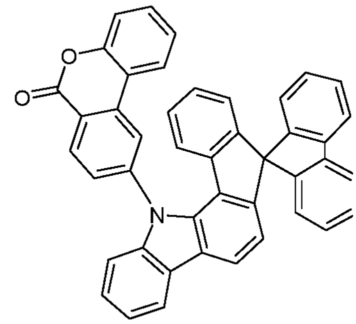

Formel 217

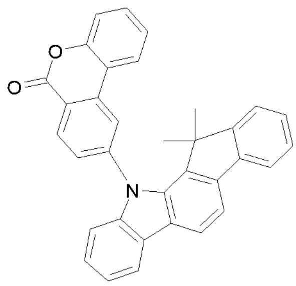

Formel 218

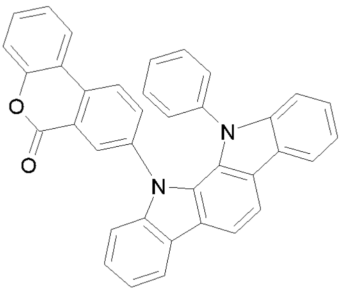

Formel 219

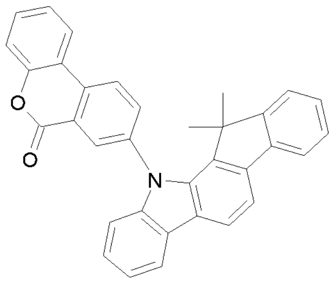

Formel 220

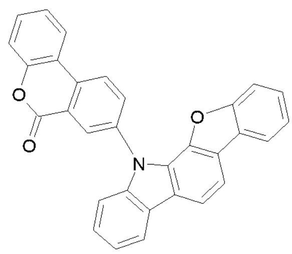

Formel 221

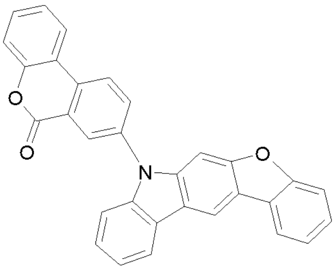

Formel 222

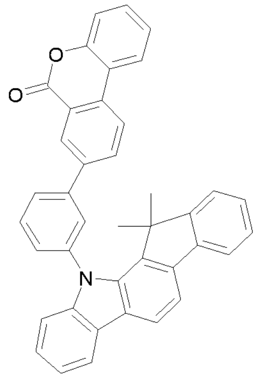

Formel 223

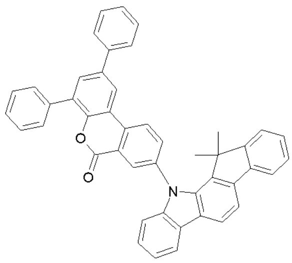

Formel 224

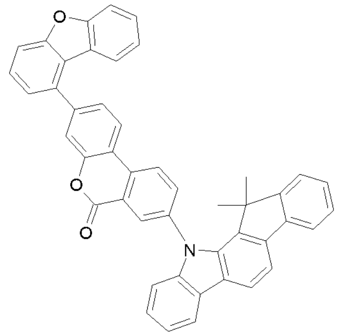

Formel 225

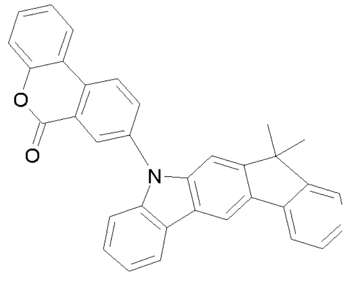

Formel 226

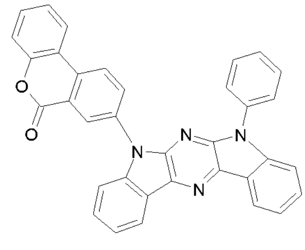

Formel 227

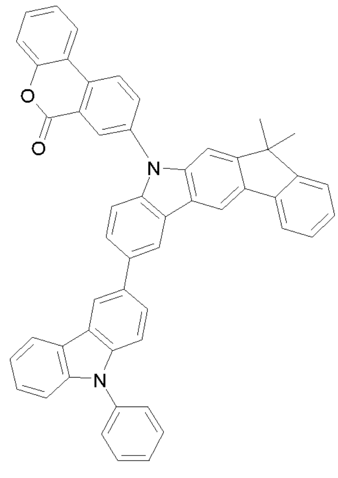

Formel 228

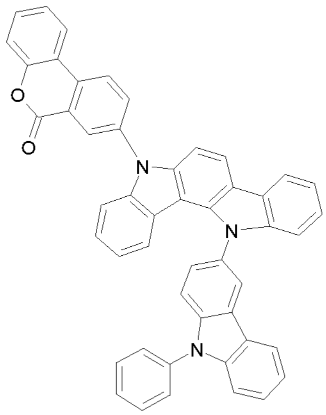

Formel 229

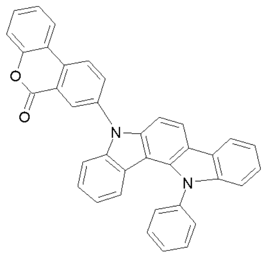

Formel 230

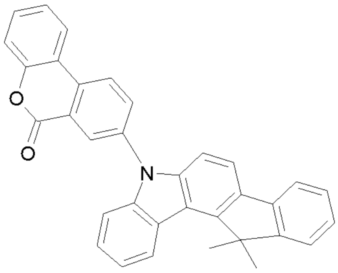

Formel 231

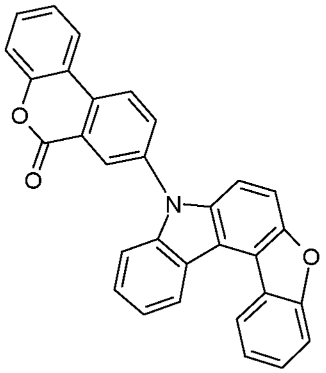

Formel 232

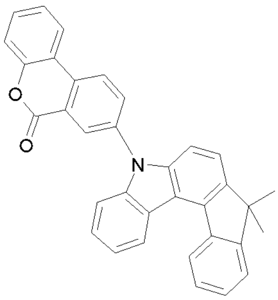

Formel 233

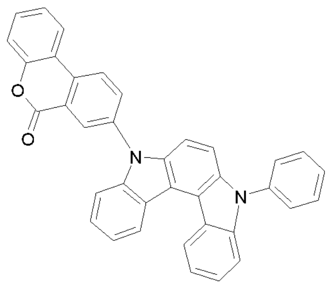

Formel 234

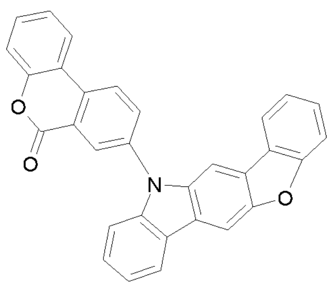

Formel 235

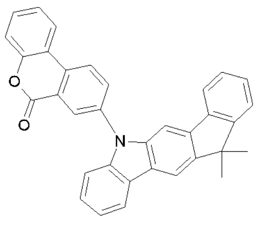

Formel 236

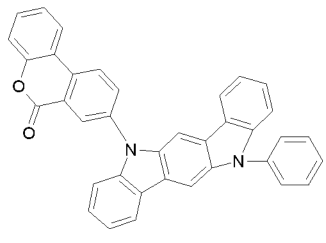

Formel 237

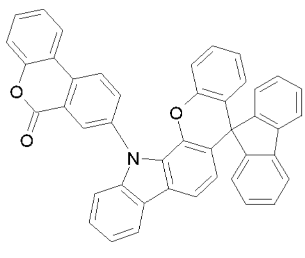

Formel 238

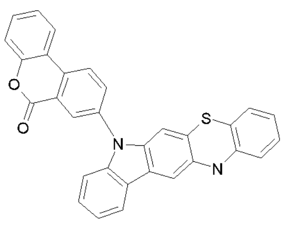

Formel 239

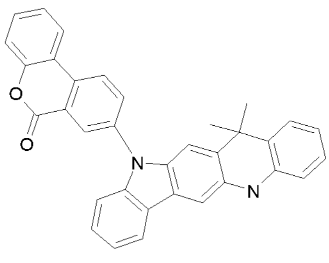

Formel 240

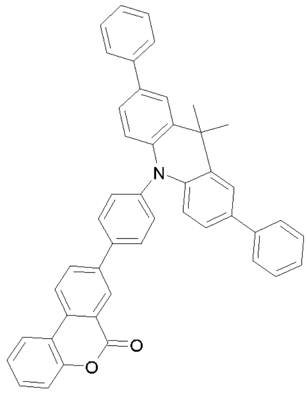

Formel 241

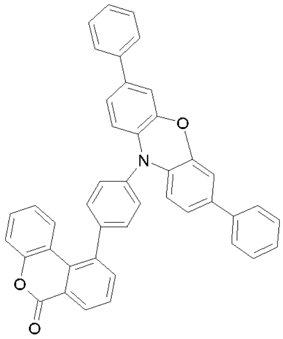

Formel 242

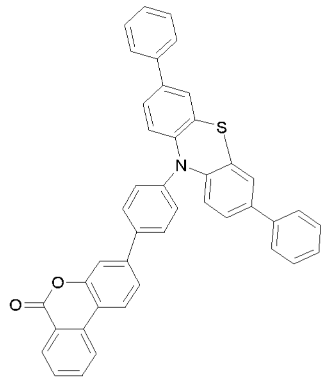

Formel 243

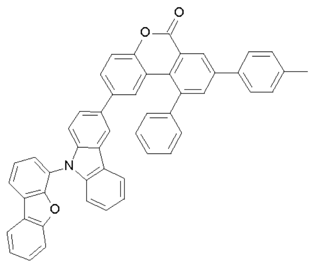

Formel 244

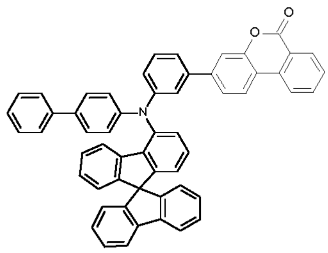

Formel 245

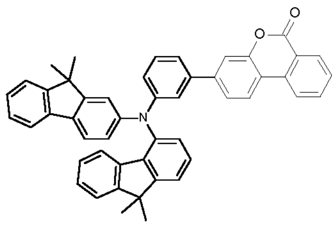

Formel 246

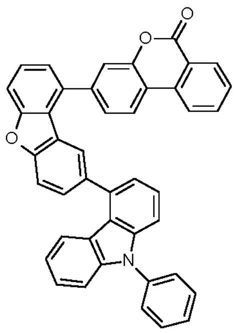

Formel 247.

Bevorzugte Ausführungsformen von Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die beschriebenen Verbindungen sind prinzipiell durch verschiedene Verfahren darstellbar. Es haben sich jedoch die im Folgenden beschriebenen Verfahren als besonders geeignet herausgestellt.

Es ist ein Verfahren zur Herstellung der Verbindungen, umfassend Strukturen gemäß Formel (I), bei dem eine Coumarin-Verbindung über eine Kupplungsreaktion mit einer Aryl- und/ oder Heteroaryl-Gruppe verbunden wird.

Die Coumarin-Verbindung, die vorzugsweise mindestens ein Benzo[c]-coumarin-Strukturelement umfasst kann unter anderem durch Oxidation aus einer entsprechenden Dibenzofuran-Verbindung oder durch eine Ringschlussreaktion, beispielsweise durch die Umsetzung einer aromatischen Carbonsäureverbindung, zum Beispiel einer Benzoesäureverbindung, mit einer Phenolverbindung erfolgen. Die notwendigen Bedingungen hierfür sind dem Fachmann bekannt, wobei die ausführlichen Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

In allen folgenden Syntheseschemata sind die Verbindungen zur Vereinfachung der Strukturen mit einer geringen Anzahl an Substituenten gezeigt gezeigt. Dies schließt das Vorhandensein von beliebigen weiteren Substituenten in den Verfahren nicht aus.

Eine Umsetzung ergibt sich beispielhaft gemäß folgenden Schemta, ohne dass hierdurch eine Beschränkung erfolgen soll. Die Teilschritte der einzelnen Schemata können hierbei beliebig kombiniert werden.

Beispielsweise kann gemäß Schema 1, ausgehend von einer Dibenzofuranverbindung eine reaktive Benzo[c]coumarin-Verbindung durch eine Oxidation hergestellt werden, wobei die Oxidation beispielsweise durch Natriumpercarbonat erfolgen kann. Alternativ kann ausgehend von einer reaktiven Brombenzoesäure durch eine Ringschlussreaktion mit einer Phenolborsäureverbindung eine reaktive Benzo[c]coumarin-Verbindung erhalten werden. Ausgehend von dieser reaktiven Benzo[c]coumarin-Verbindung kann über eine Buchwaldkupplung beispielsweise eine Carbazolstruktur angebunden werden. Ferner kann beispielsweise über eine Suzuki-Reaktion eine Arylgruppe an die Benzo[c]coumarin-Verbindung gebunden werden.

## Schema 1

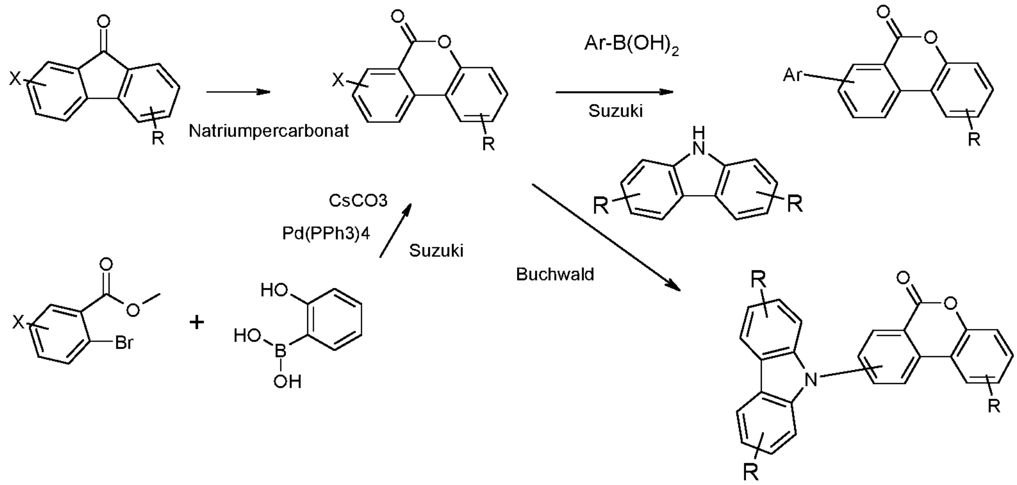


X = Halogen oder Triflat, wobei das Triflat auch in einer Zwischenreaktion aus einem Ether oder einer Hydroxygruppe erhalten werden kann, wie dies in den Beispielen näher erläutert wird.

In Umsetzungen nach Schema 2 wird, ausgehen von entsprechendem Dibromid, die Synthese von disubstituierten Derivaten dargestellt.

## Schema 2

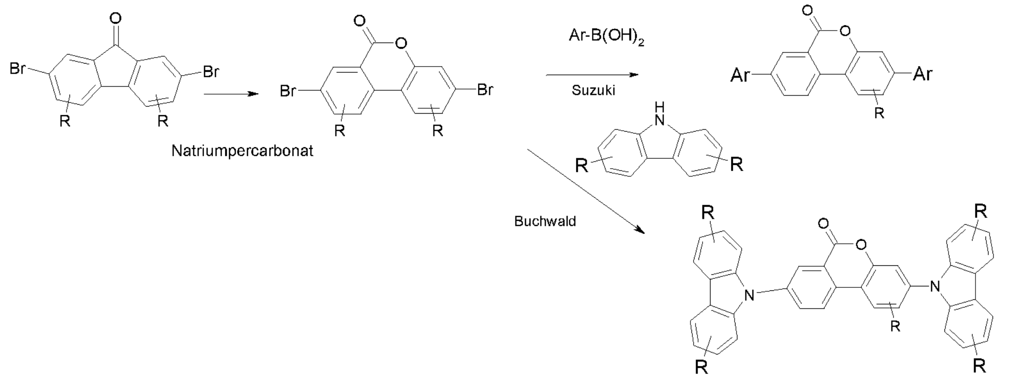


Gemäß Schema 3 können zwei oder mehr gleiche oder verschiedene Substituenten stufenweise mit einer

Benzo[c]coumarin-Verbindung mit entsprechend unterschiedlich reaktiven Gruppen verbunden werden. Vorzugsweise können Verbindungen mit Hydroxy- und/oder Ethergruppen hierzu eingesetzt werden. Die Position der Substitution kann durch das entsprechende Ausgangsmaterial festgelegt werden.

## Schema 3

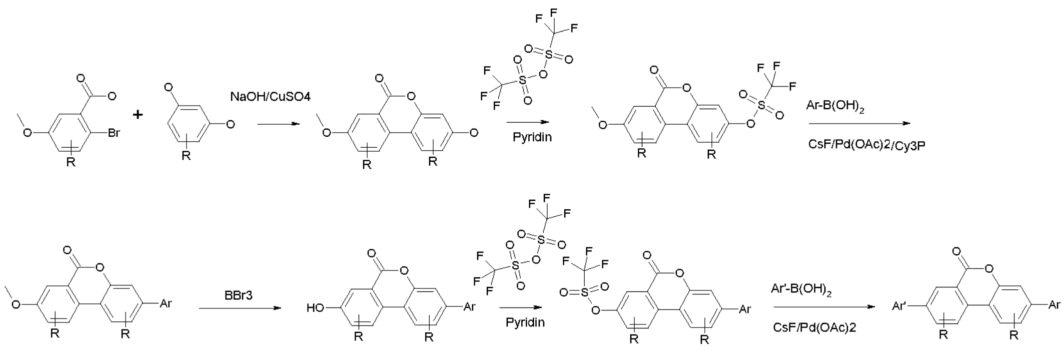


Die gezeigten Verfahren zur Synthese der beschriebenen Verbindungen sind exemplarisch zu verstehen. Der Fachmann kann alternative Synthesewege im Rahmen seines allgemeinen Fachwissens entwickeln.

Die Grundlagen der zuvor dargelegten Herstellungsverfahren sind im Prinzip aus der Literatur für ähnliche Verbindungen bekannt und können vom Fachmann leicht zur Herstellung der beschriebenen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die Verbindungen, umfassend Strukturen gemäß Formel (I) in hoher Reinheit, bevorzugt mehr als 99 % (bestimmt mittels $^1$H-NMR und/oder HPLC) erhalten.

Die beschriebenen Verbindungen können auch geeignete Substituenten aufweisen, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, die eine Löslichkeit in gängigen organischen Lösemitteln bewirken, wie beispielsweise Toluol oder Xylol bei Raumtemperatur in ausreichender Konzentration löslich, um die Komplexe aus Lösung verarbeiten zu können. Diese löslichen Verbindungen eignen sich besonders gut für die Verarbeitung aus Lösung, beispielsweise durch Druckverfahren. Weiterhin ist festzuhalten, dass die Verbindungen, umfassend mindestens eine Struktur der Formel (I) bereits eine gesteigerte Löslichkeit in diesen Lösungsmitteln besitzen.

Die beschriebenen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die Verbindungen und Polymere können als vernetzte oder unvernetzte Schicht eingesetzt werden.

Je nach Verknüpfung der Strukturen der Formel (I) bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der beschriebenen Verbindungen in Oligomeren, Dendrimeren und Polymeren gelten dieselben Bevorzugungen, wie oben beschrieben.

Zur Herstellung der Oligomere oder Polymere werden die beschriebenen Monomere homopolymerisiert oder mit weiteren Monomeren copolymerisiert. Bevorzugt sind Copolymere, wobei die Einheiten gemäß Formel (I) bzw. die oben ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten,

beispielsweise Lochtransporteinheiten, insbesondere solche basierend auf Triarylaminen, und/oder Elektronentransporteinheiten.

Die Verbindungen in den vorliegenden Vorrichtungen weisen ein Molekulargewicht von kleiner oder gleich 5000 g/mol, besonders bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1000 g/mol auf.

Weiterhin zeichnen sich bevorzugte Verbindungen in der erfindungsgemäßen Vorrichtung dadurch aus, dass diese sublimierbar sind. Diese Verbindungen weisen im Allgemeinen eine Molmasse von weniger als ca. 1200 g/mol auf.

Von besonderem Interesse sind des Weiteren Verbindungen, die sich durch eine hohe Glasübergangstemperatur auszeichnen. In diesem Zusammenhang sind insbesondere Verbindungen umfassend Strukturen der allgemeinen Formel (I) bevorzugt, die eine Glasübergangstemperatur von mindestens 70°C, besonders bevorzugt von mindestens 110°C, ganz besonders bevorzugt von mindestens 125°C und insbesondere bevorzugt von mindestens 150°C aufweisen, bestimmt nach DIN 51005.

Es kann eine Formulierung, enthaltend eine Verbindung bzw. ein Oligomer, Polymer oder Dendrimer und mindestens eine weitere Verbindung verwendet werden. Die weitere Verbindung kann vorzugsweise ein Lösemittel sein. Die weitere Verbindung kann aber auch eine weitere organische oder anorganische Verbindung sein, die ebenfalls in der elektronischen Vorrichtung eingesetzt wird, beispielsweise ein Matrixmaterial. Diese weitere Verbindung kann auch polymer sein.

Geeignete und bevorzugte Lösungsmittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-tetramethylbenzol, 1-Methylnaphtalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzyl ether, Diethylenglycolbutylmethylether, Triethylenglycolbutylmethylether, Diethylenglycoldibutylether, Triethylenglycoldimethylether, Diethylen-glycolmonobutylether, Tripropylenglycoldimethylether, Tetraethylenglycol-dimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösungsmittel.

Nochmals ein weiterer Gegenstand der vorliegenden Beschreibung ist eine Zusammensetzung enthaltend eine beschriebene Verbindung und wenigstens ein weiteres organisch funktionelles Material Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, n-Dotanden, Wide-Band-Gap-Materialien, Elektronenblockiermaterialien und Lochblockiermaterialien.

Die vorliegende Beschreibung betrifft daher auch eine Zusammensetzung enthaltend wenigstens eine Verbindung umfassend Strukuren gemäß Formel (I) sowie wenigstens ein weiteres Matrixmaterial. Gemäß einem besonderen Aspekt weist das weitere Matrixmaterial lochtransportierende Eigenschaften auf.

Ein weiterer Gegenstand der vorliegenden Beschreibung stellt eine Zusammensetzung dar, enthaltend wenigstens eine Verbindung, umfassend mindestens eine Struktur gemäß Formel (I) sowie wenigstens ein Wide-Band-Gap-Material, wobei unter Wide-Band-Gap-Material ein Material im Sinne der Offenbarung von US 7,294,849 verstanden wird. Diese Systeme zeigen besondere vorteilhafte Leistungsdaten in elektrolumineszierenden Vorrichtungen.

Vorzugsweise kann die zusätzliche Verbindung eine Bandlücke (band gap) von 2,5 eV oder mehr, bevorzugt 3,0 eV oder mehr, ganz bevorzugt von 3,5 eV oder mehr aufweisen. Die Bandlücke kann unter anderem durch die Energieniveaus des highest occupied molecular orbital (HOMO) und des lowest unoccupied molecular orbital (LUMO) berechnet werden.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden über quantenchemische Rechnungen bestimmt. Zur Berechnung organischer Substanzen ohne Metalle wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31 G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31 G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$HOMO(eV) = ((HEh*27.212)-0.9899)/1.1206$$

$$LUMO(eV) = ((LEh*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Die vorliegende Beschreibung betrifft auch eine Zusammensetzung umfassend wenigstens eine Verbindung umfassend Strukuren gemäß Formel (I) sowie wenigstens einen phosphoreszierende Emitter, wobei unter dem Begriff phosphoreszierende Emitter auch phosphoreszierende Dotanden verstanden werden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Anmeldung alle lumineszierenden Iridium-, Platin- oder Kupferkomplexe als phosphoreszierende Verbindungen angesehen. Beispiele für phosphoreszierende Dotanden sind in einem folgenden Abschnitt aufgeführt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Bevorzugte phosphoreszierende Dotanden zur Verwendung in Mixed-Matrix-Systemen sind die im Folgenden angebenen bevorzugten phosphoreszierenden Dotanden.

Beispiele für phosphoreszierende Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen.

Explizite Beispiele für phosphoreszierende Dotanden sind in der folgenden Tabelle aufgeführt.

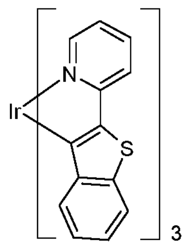

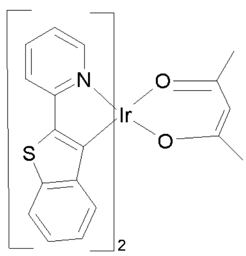

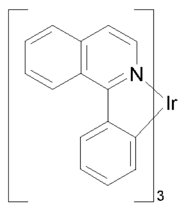

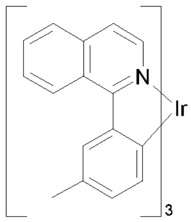

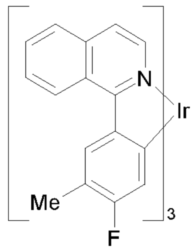

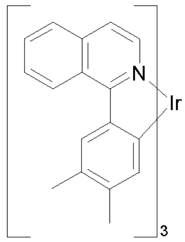

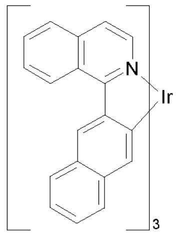

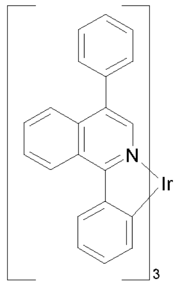

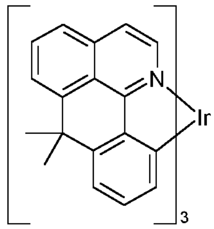

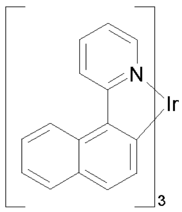

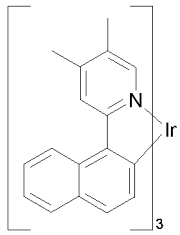

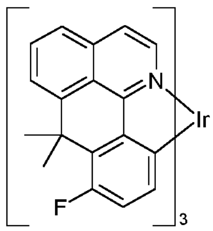

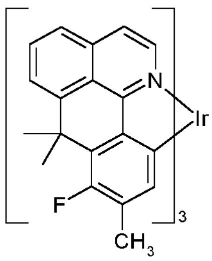

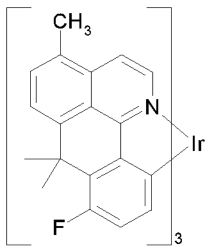

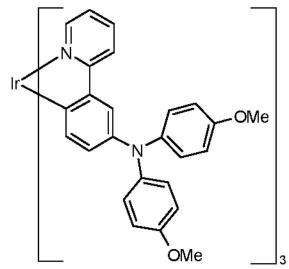

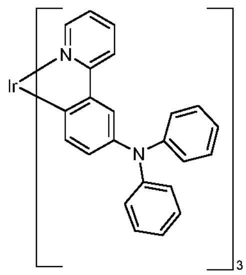

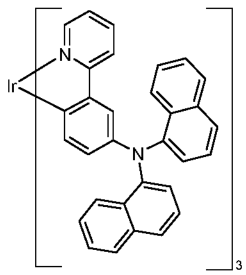

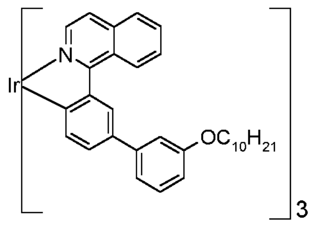

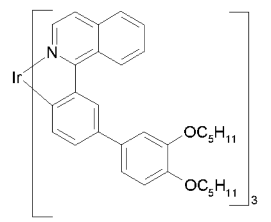
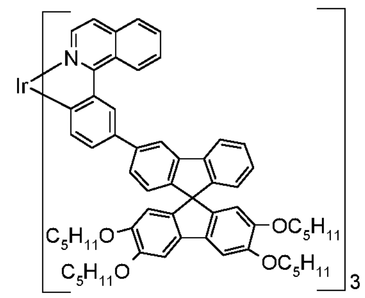
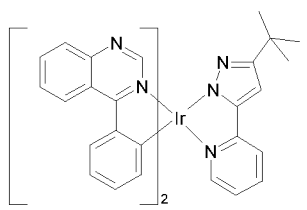

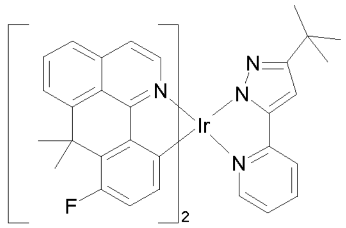
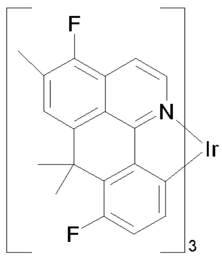
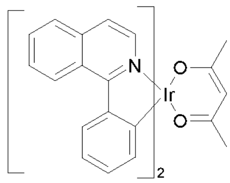

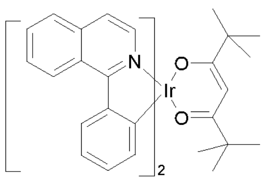
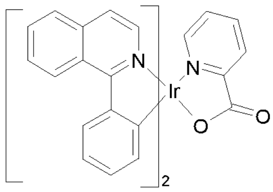
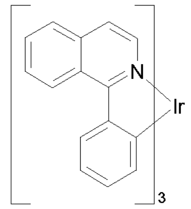

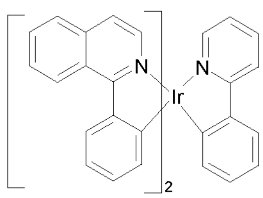
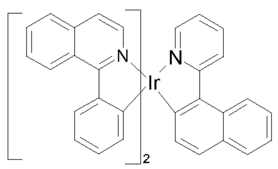
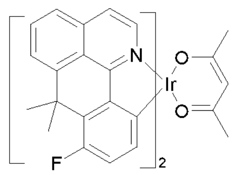

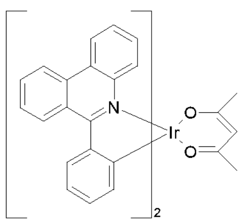
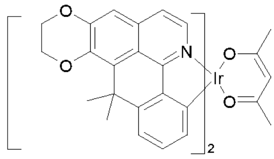
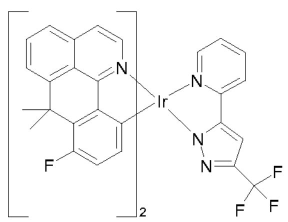

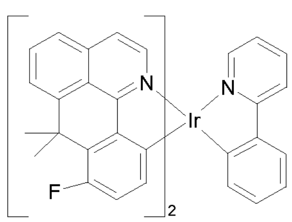
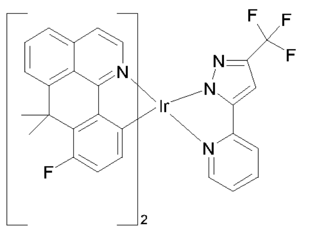
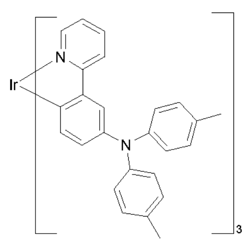

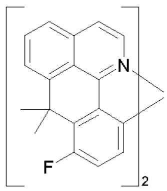
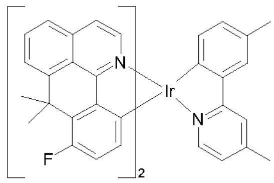
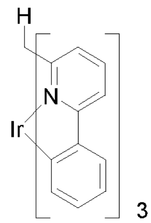

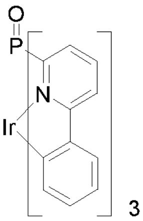
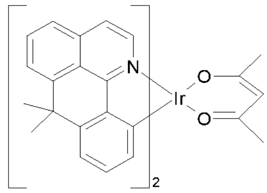
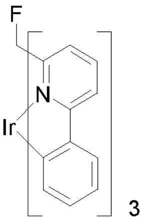

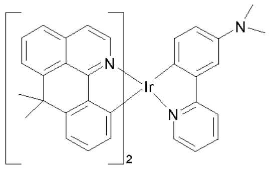
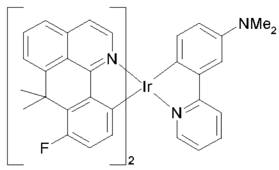
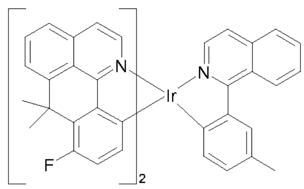

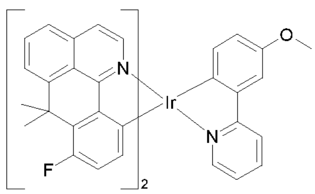
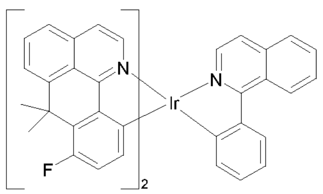
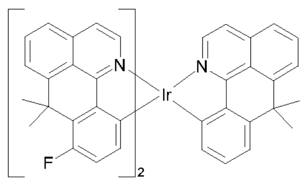

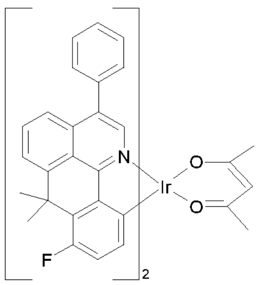
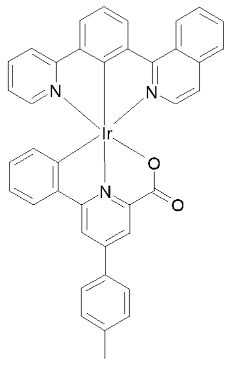
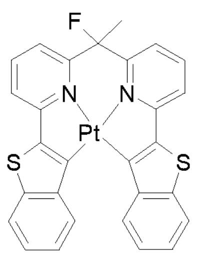

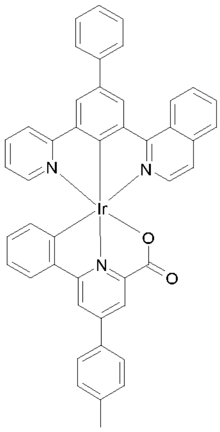
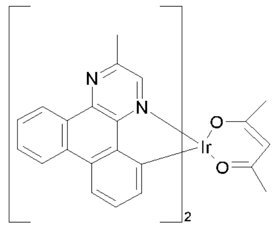
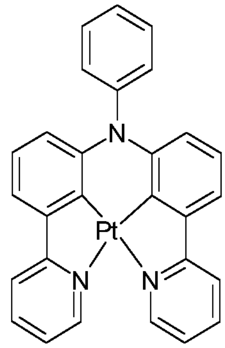
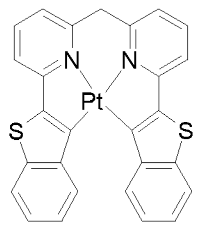
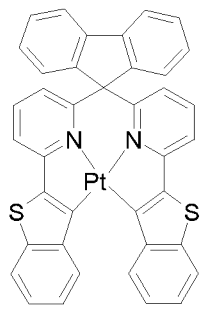
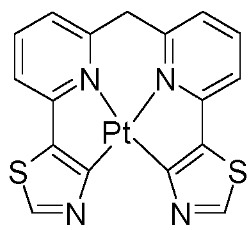
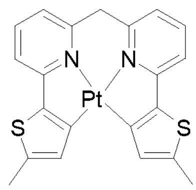
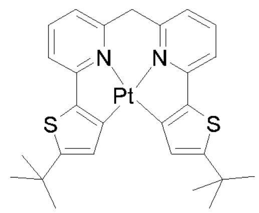
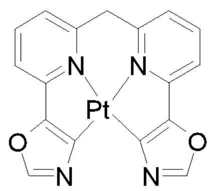
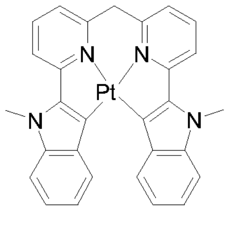
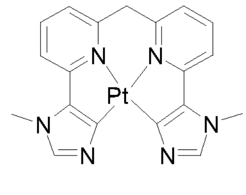
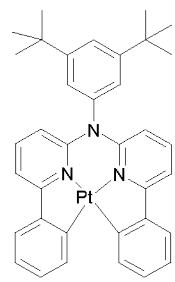
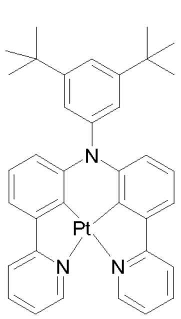
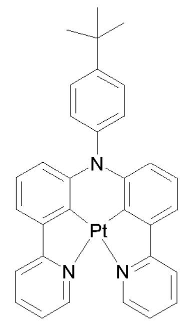
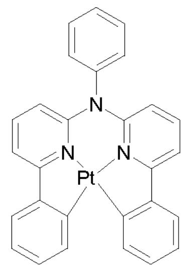

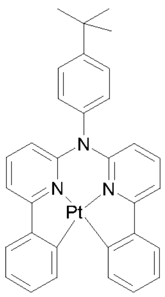
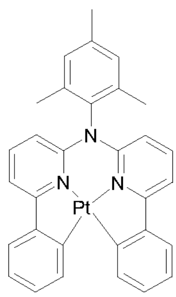
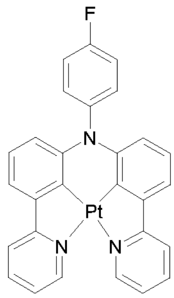

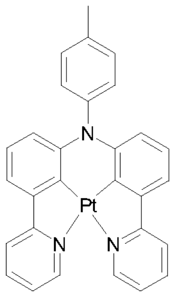
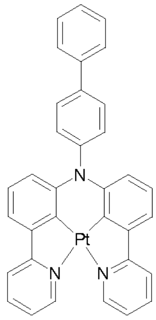
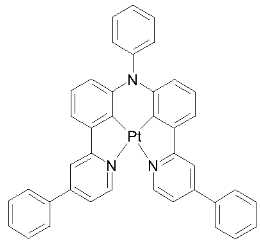

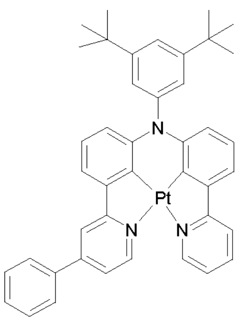
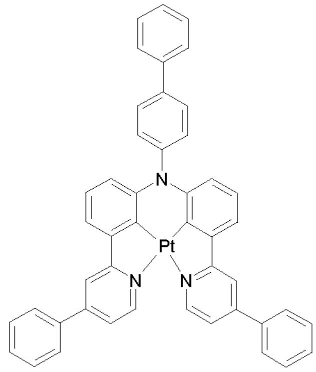
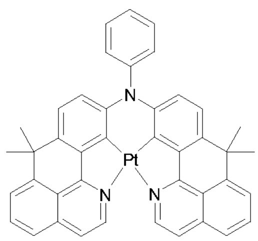

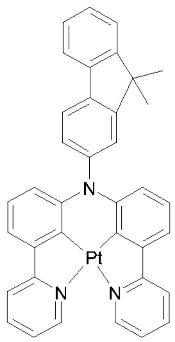
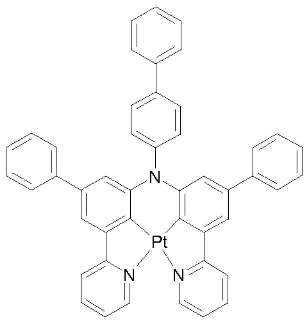
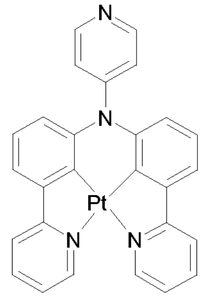

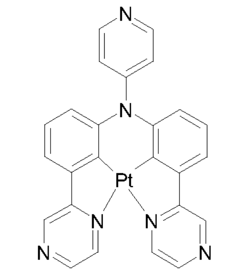
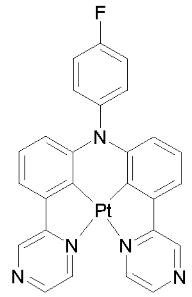
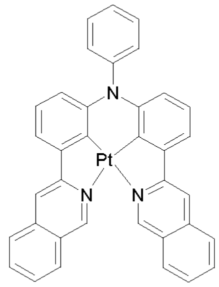

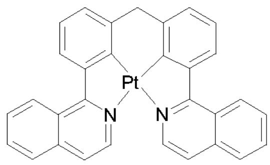
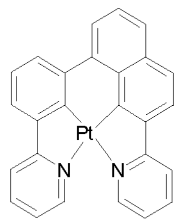
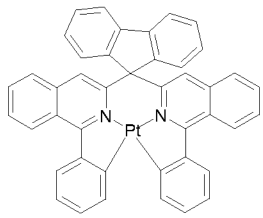

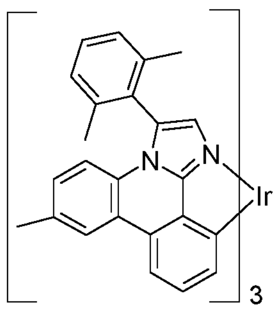
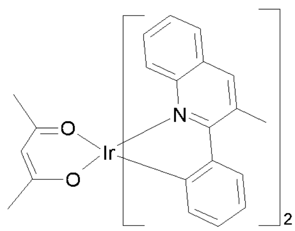
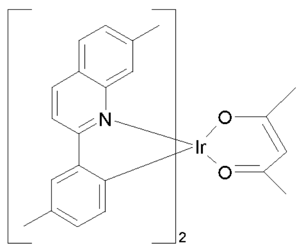

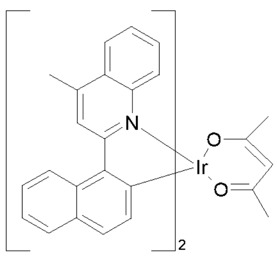
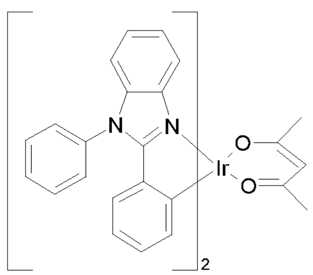
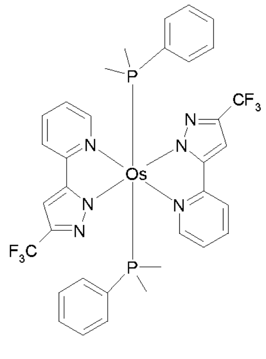

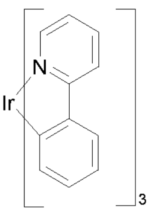
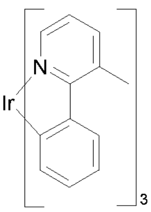
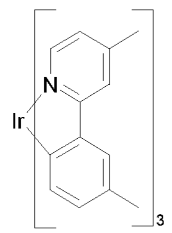

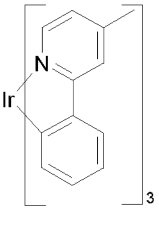
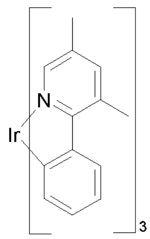
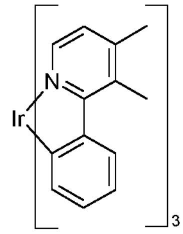

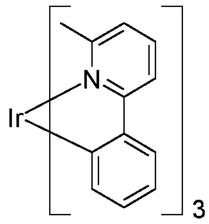
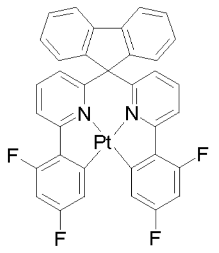
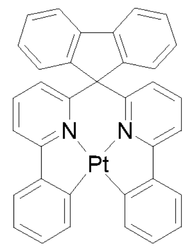

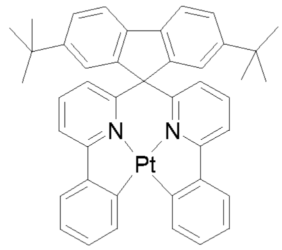
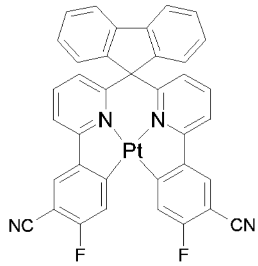
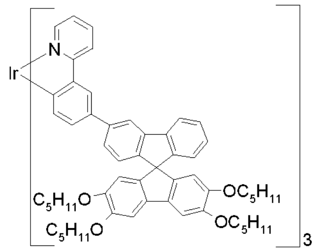

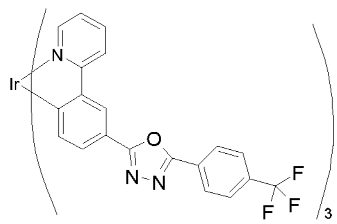
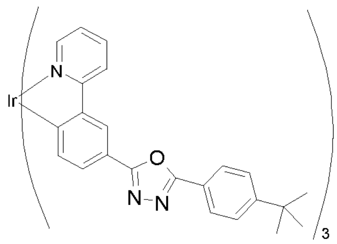
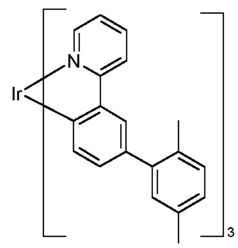

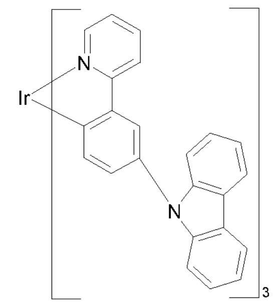
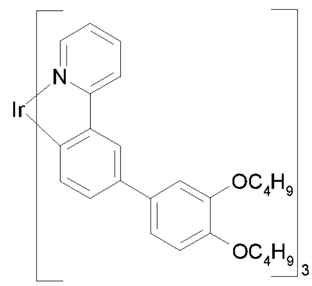
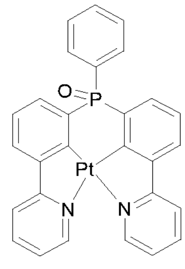

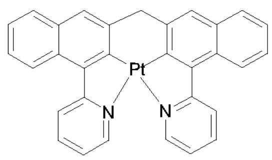
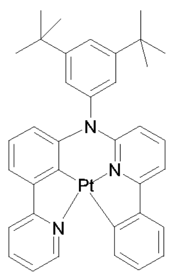
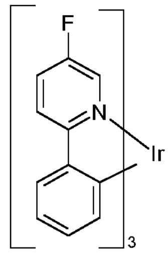

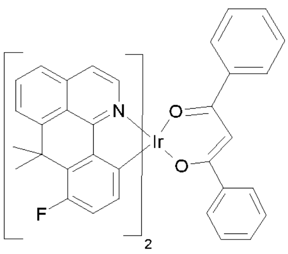
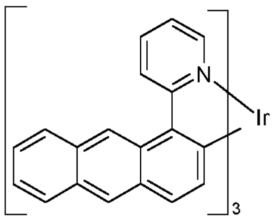
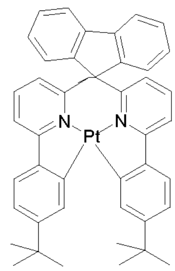

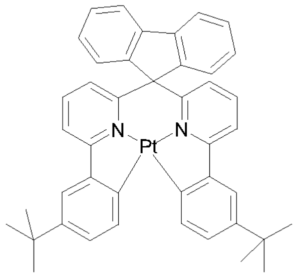
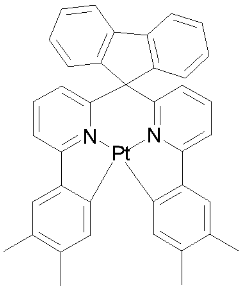
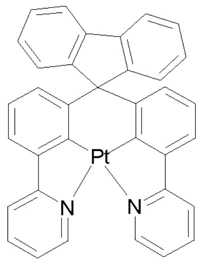

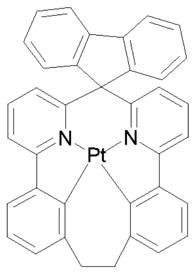
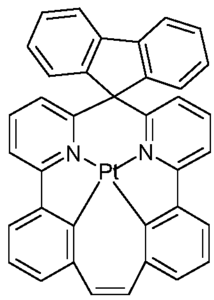
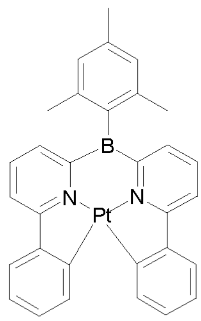

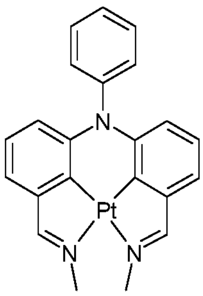
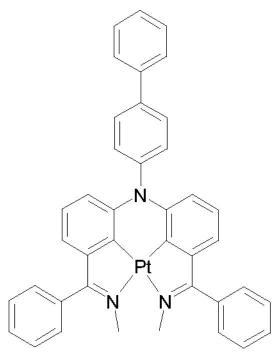
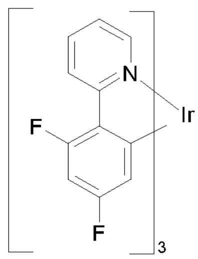

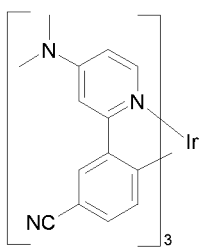
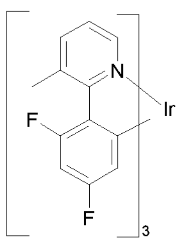
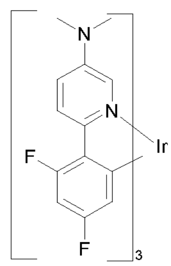

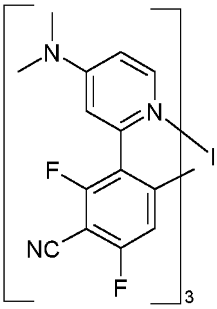
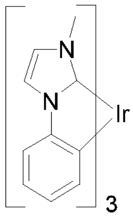
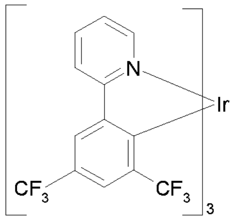

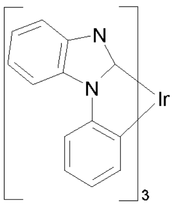
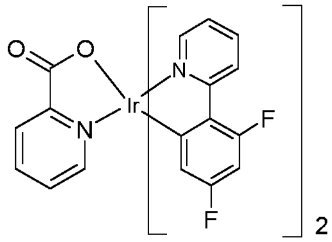
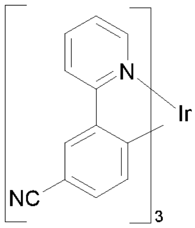

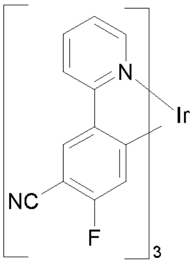
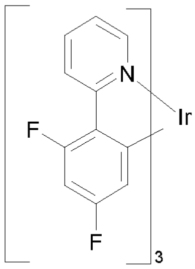
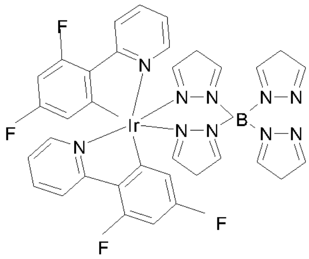

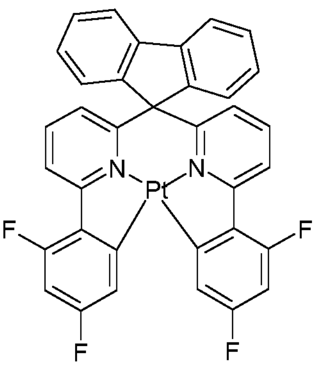
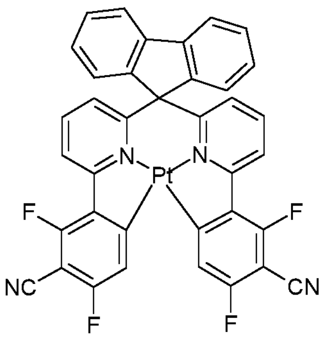
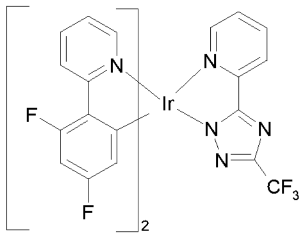

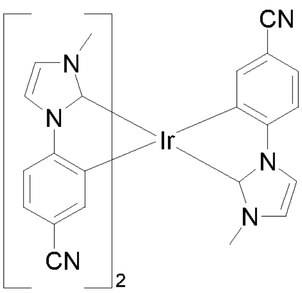
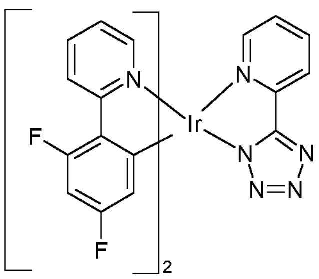
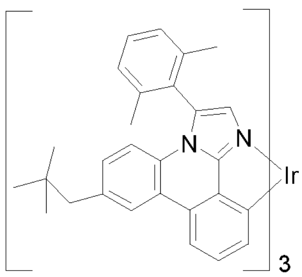

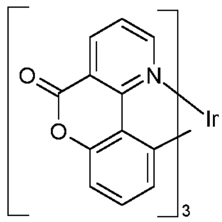
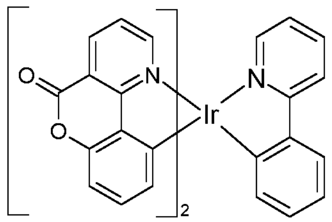
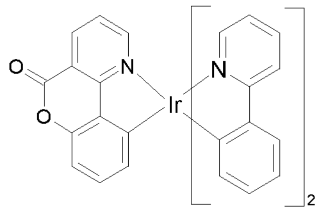

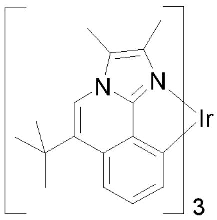
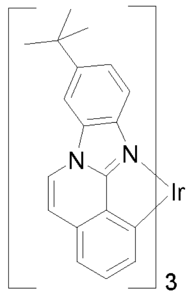
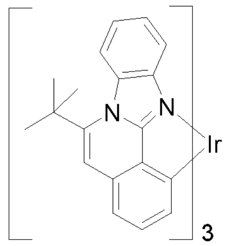

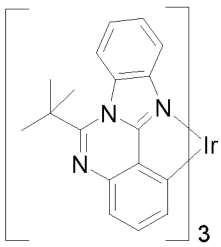
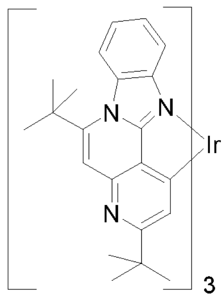
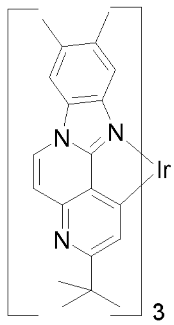

Die oben beschriebenen Verbindung, umfassend Strukturen der Formel (I), bzw. die oben aufgeführten bevorzugten Ausführungsformen können in einer elektronischen Vorrichtung bevorzugt als aktive Komponente verwendet werden. Unter einer elektronischen Vorrichtung wird eine Vorrichtung verstanden, welche Anode, Kathode und mindestens eine Schicht enthält, wobei diese Schicht mindestens eine organische bzw. metallorganische Verbindung enthält. Die erfindungsgemäße elektronische Vorrichtung enthält also Anode, Kathode und mindestens eine Schicht, welche mindestens eine Verbindung, umfassend Strukturen der Formel (I), enthält, wie in Anspruch 1 beschrieben. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs) oder organischen Laserdioden (O-Laser), enthaltend in mindestens einer Schicht mindestens eine Verbindung, umfassend Strukturen der Formel (I) nach Anspruch 1. Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien.

Eine bevorzugte Ausführungsform der Erfindung sind organische Elektrolumineszenzvorrichtungen. Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie $MoO_3$ oder $WO_3$ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser

Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Es kann sich auch um ein HybridSystem handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die Verbindung umfassend Strukturen gemäß Formel (I) nach Anspruch 1 als Matrixmaterial, vorzugsweise als elektronenleitendes Matrixmaterial in einer oder mehreren emittierenden Schichten, bevorzugt in Kombination mit einem weiteren Matrixmaterial, vorzugsweise einem lochleitenden Matrixmaterial. Eine emittierende Schicht umfasst mindestens eine emittierende Verbindung.

Als Matrixmaterial können generell alle Materialien eingesetzt werden, die gemäß dem Stand der Technik hierfür bekannt sind. Bevorzugt ist das Triplett-Niveau des Matrixmaterials höher als das Triplett-Niveau des Emitters.

Geeignete Matrixmaterialien für die beschriebenen Verbindungen sind Ketone, Phosphinoxide, Sulfoxide und Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl), m-CBP oder die in WO 2005/039246, US 2005/0069729, JP 2004/288381, EP 1205527, WO 2008/086851 oder US 2009/0134784 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazole, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Diazasilolderivate, z. B. gemäß WO 2010/054729, Diazaphospholderivate, z. B. gemäß WO 2010/054730, Triazinderivate, z. B. gemäß WO 2010/015306, WO 2007/063754 oder WO 2008/056746, Zinkkomplexe, z. B. gemäß EP 652273 oder WO 2009/062578, Dibenzofuranderivate, z. B. gemäß WO 2009/148015, oder verbrückte Carbazolderivate, z. B. gemäß US 2009/0136779, WO 2010/050778, WO 2011/042107 oder WO 2011/088877.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum.

Besonders bevorzugt kann eine beschriebene Verbindung umfassend Strukuren gemäß Formel (I) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt werden. Dabei ist das Matrixmaterial enthaltend Verbindung umfassend Strukuren gemäß Formel (I) in der elektronischen Vorrichtung in Kombination mit einem oder mehreren Dotanden, vorzugsweise phosphoreszierenden Dotanden, vorhanden.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform werden die Verbindung umfassend Strukuren gemäß Formel (I) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Ferner ist eine elektronische Vorrichtung, vorzugsweise eine organische Elektrolumineszenzvorrichtung Gegenstand der vorliegenden Erfindung, die eine oder mehrere Verbindungen nach Anspruch 1 in einer oder mehreren elektronenleitenden Schichten umfasst, als elektronenleitende Verbindung.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise $MoO_3$ oder $WO_3$, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine elektronischen Vorrichtung, insbesondere eine organische Elektrolumineszenzvorrichtung, welche dadurch gekennzeichnet ist, dass eine oder mehrere Schichten mit einem Sublimationsverfahren beschichtet werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine elektronischen Vorrichtung, insbesondere eine organische Elektrolumineszenzvorrichtung, welche dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronischen Vorrichtung, insbesondere eine organische Elektrolumineszenzvorrichtung, welche dadurch gekennzeichnet ist, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronischen Vorrichtung, insbesondere die organische Elektrolumineszenzvorrichtung kann auch als Hybridsystem hergestellt werden, indem eine oder mehrere Schichten aus Lösung aufgebracht werden und eine oder

mehrere andere Schichten aufgedampft werden. So ist es beispielsweise möglich, eine emittierende Schicht enthaltend eine efindungsgemäße Verbindung umfassend Strukturen gemäß Formel (I) und ein Matrixmaterial aus Lösung aufzubringen und darauf eine Lochblockierschicht und/oder eine Elektronentransportschicht im Vakuum aufzudampfen. Diese Verfahren sind dem Fachmann generell bekannt und können von ihm ohne Probleme auf elektronischen Vorrichtungen, insbesondere organische Elektrolumineszenzvorrichtungen enthaltend Verbindungen umfassend Strukturen gemäß Formel (I) nach Anspruch 1 angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:

1. Elektronischen Vorrichtungen, insbesondere organische Elektrolumineszenzvorrichtungen enthaltend Verbindungen mit Strukturen gemäß Formel (I), insbesondere als elektronenleitende und/oder als lochleitende Materialien, weisen eine sehr gute Lebensdauer auf.

2. Elektronischen Vorrichtungen, insbesondere organische Elektrolumineszenzvorrichtungen enthaltend Verbindungen mit Strukturen gemäß Formel (I) als elektronenleitende und/oder als lochleitende Materialien weisen eine hervorragende Effizienz auf. Insbesondere ist die Effizienz deutlich höher gegenüber analogen Verbindungen, die keine Struktureinheit gemäß Formel (I) enthalten.

3. Die Verbindungen mit Strukturen gemäß Formel (I) zeigen eine sehr hohe Stabilität und führen zu Verbindungen mit einer sehr hohen Lebensdauer.

4. Mit Verbindungen mit Strukturen gemäß Formel (I) kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.

5. Die Verwendung von Verbindungen mit Strukturen gemäß Formel (I) in Schichten elektronischer Vorrichtungen, insbesondere organischer Elektroumineszenzvorrichtungen führt zu einer hohen Mobilität der Elektron-Leiterstrukturen und/oder der Loch-Leiterstrukturen.

6. Verbindungen mit Strukturen gemäß Formel (I) zeichnen sich durch eine ausgezeichnete thermische Stabilität aus, wobei Verbindungen mit einer Molmasse von weniger als ca. 1200 g/mol gut sublimierbar sind

7. Verbindungen mit Strukturen gemäß Formel (I) weisen eine ausgezeichnete Glasfilmbildung auf.

8. Verbindungen mit Strukturen gemäß Formel (I) bilden aus Lösungen sehr gute Filme.

9. Die Verbindungen umfassend Strukturen gemäß Formel (I) weisen ein überraschend hohes Triplett-Niveau $T_1$ auf, wobei dies insbesondere Verbindungen gilt, die als elektronenleitende Materialien eingesetzt werden.

Diese oben genannten Vorteile gehen nicht mit einer Verschlechterung der weiteren elektronischen Eigenschaften einher.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

Der Fachmann kann aus den Schilderungen ohne erfinderisches Zutun weitere erfindungsgemäße elektronische Vorrichtungen herstellen und somit die Erfindung im gesamten beanspruchten Bereich ausführen.

## Beispiele

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von Sigma-ALDRICH bzw. ABCR bezogen

werden. Die jeweiligen Angaben in eckigen Klammern bzw. die zu einzelnen Verbindungen angegebenen Nummern beziehen sich auf die CAS-Nummern der literaturbekannten Verbindungen.

**Hestellungsbeispiele:**

**a) 1-Brom-dibenzofuran**

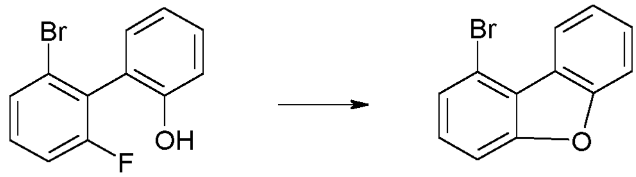

111 g (416 mmol) 6'-Bromo-2'-fluoro-biphenyl-2-ol werden in 2 L DMF (max 0,003% $H_2O$) SeccoSolv® gelöst und auf 5°C gekühlt. Zu dieser Lösung wird portionsweise 20 g (449 mmol) Natriumhydrid (60% Suspension in Paraffinöl)zugegeben und nach beendeter Zugabe 20 min. nachgerührt und dann für 45 min. auf 100 °C erhitzt. Das Gemisch wird im Anschluss nach dem Abkühlen langsam mit 500 ml Ethanol versetzt, und komplett einrotiert und dann chromatographisch gereinigt. Ausbeute: 90g (367 mmol), 88,5 % der Theorie.

Analog dazu werden die folgenden Verbindungen hergestellt:

| | Edukt 1 | Produkt | Ausbeute |
|---|---|---|---|
| a1 | 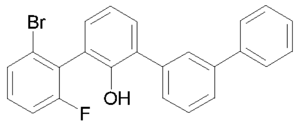 | 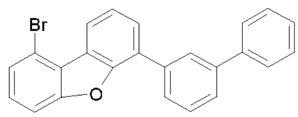 | 73% |
| a2 | 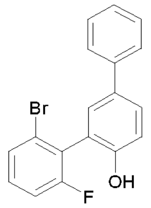 | 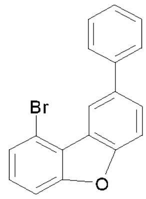 | 79% |

**b) Dibenzofuran-1-boronsäure**

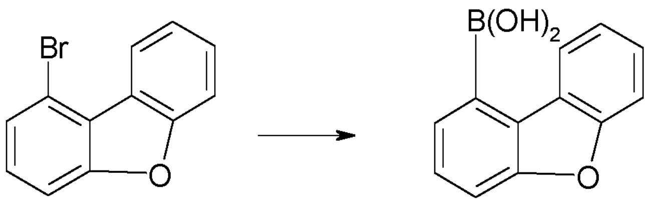

180 g (728 mmol) 1-Brom-dibenzofuran werden in 1500 mL trockenem THF gelöst und auf 78°C gekühlt. Bei dieser Temperatur wird mit 305 mL (764 mmol / 2.5 M in Hexan) n-BuLi innerhalb von ca. 5 min. versetzt und anschließend für 2.5h bei -78°C nachgerührt. Bei dieser Temperatur wird mit 151 g (1456 mmol) Borsäure-trimethylester möglichst zügig versetzt und die Reaktion langsam auf RT kommen gelassen (ca. 18h). Die Reaktionslösung wir mit Wasser gewaschen und der ausgefallene Feststoff und die organische Phase mit Toluol azeotrop getrocknet. Das Rohprodukt wird aus Toluol/methylenchlorid bei ca. 40°C ausgerührt und abgesaugt. Ausbeute: 146 g (690 mmol), 95 % der Theorie.

Analog dazu werden die folgenden Verbindungen hergestellt:

| | Edukt 1 | Produkt | Ausbeute |
|---|---|---|---|
| b1 | 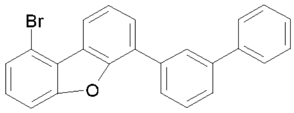 | 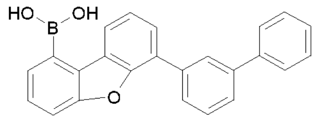 | 73% |
| b2 | 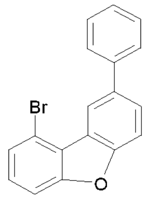 | 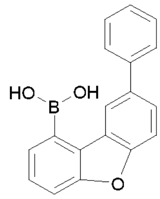 | 79% |
| b3 | 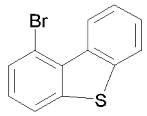 [65642-94-6] | 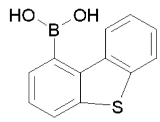 | 73% |

**c) Synthese von 5"-Chloro-[1,3';1',1";3",1"';3"',1""]quinquephenyl**

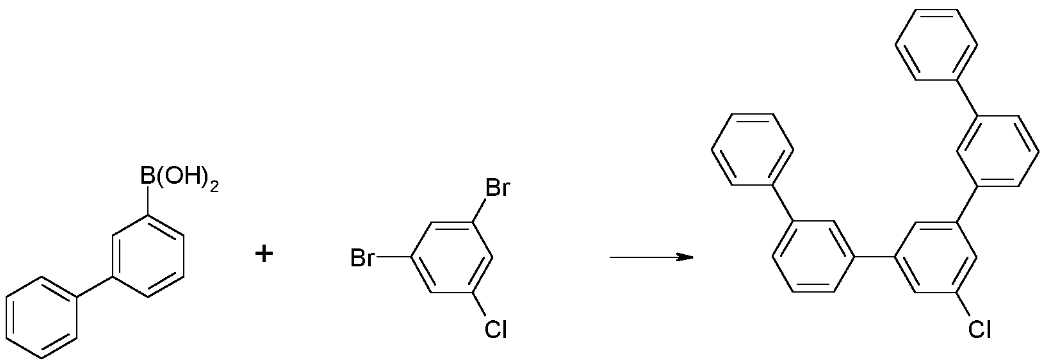

29 g (148 mmol) 3-Bipheny-boronsäure, 20 g (74 mmol) 1-Chlor-3,5-dibrombenzol und 60 g (596 mmol) Natriumcarbonat werden in 500 mL THF und 300 mL Wasser suspendiert. Zu dieser Suspension werden 6,5 g(5,6 mmol) Tetrakis(triphenylphosphin)-palladium(0) gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol umkristallisiert. Die Ausbeute beträgt 22 g (53 mmol), entsprechend 71 % der Theorie.

Analog können folgende Verbindungen erhalten werden:

| Beispiel | Edukt 1 | Edukt 2 | Produkt | Ausbeute |
|---|---|---|---|---|
| **c1** | 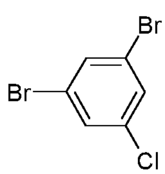 | 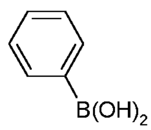 | 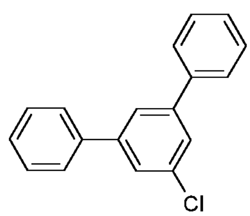 | 95% |

(fortgesetzt)

| Beispiel | Edukt 1 | Edukt 2 | Produkt | Ausbeute |
|---|---|---|---|---|
| **c2** | 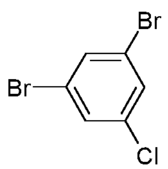 | 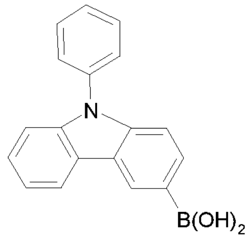 [854952-58-2] | 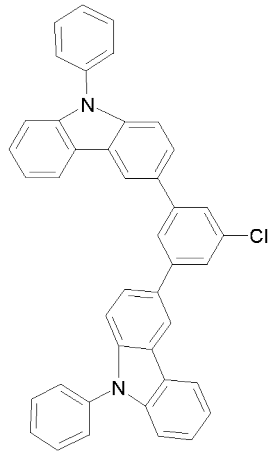 | 73% |
| **c3** | 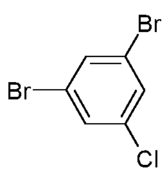 | 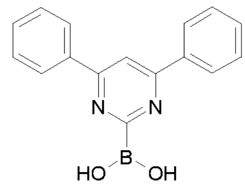 [1314221-56-1 ] | 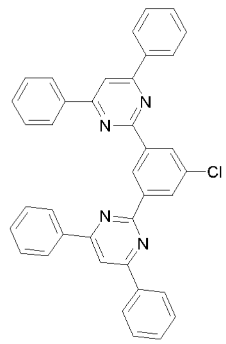 | 83% |

**d) Synthese von 4,4,5,5-Tetramethyl-2-[1,3’;1’,1";3",1'";3"',1""] quinquephenyl-5"-yl-[1,3,2]dioxaborolan**

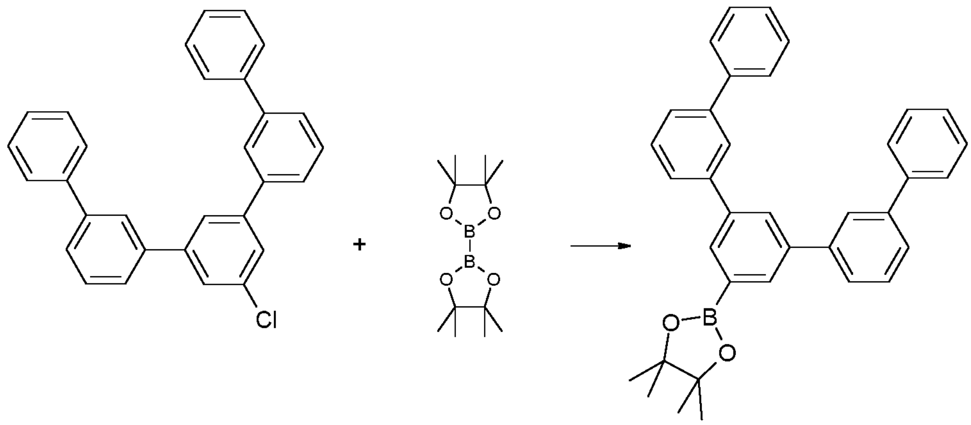

75 g (126 mmol) 5"-Chloro-[1,3’;1’,1";3",1’";3’",1""]quinquephenyl, 41,6 g Bis(pinacolato)diboran (163 mmol), 21 g (214 mmol) Kaliumacetat und 18 g (25 mmol) Palladiumdichlor-tricyclohexylphosphin werden in 1 l 1,4-Dioxan unter kräftigem Rühren für 2 Tage zum Rückfluß erhitzt. Die Reaktionsmischung wird bei Raumtemperatur über Celite filtriert. Das Lösungsmittel wird im Vakuum entfernt, der verbleibende Feststoff in Acetonitril umkristallisiert. Der dabei entstehende Feststoff wird abfiltriert und mit Heptan gewaschen. Die Ausbeute beträgt 82 g (119 mmol), entsprechend 95 % der Theorie.

Analog können folgende Verbindungen erhalten werden:

| Beispiel | Edukt 1 | Produkt | Ausbeute |
|---|---|---|---|
| d1 | 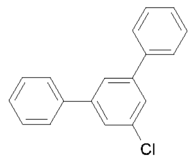 | 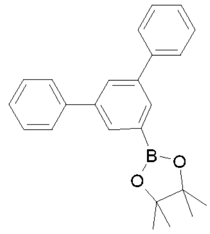 | 90% |
| d2 | 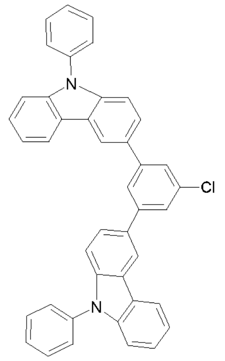 | 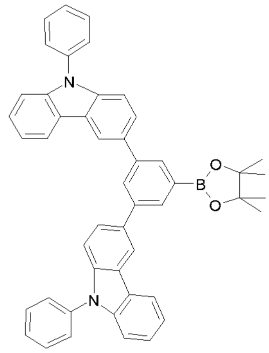 | 84% |
| d3 | 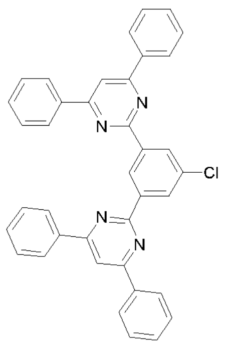 | 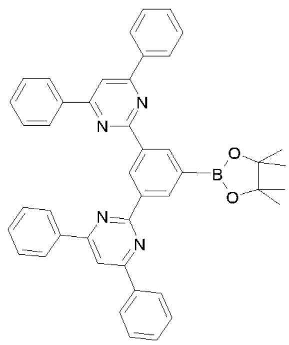 | 75% |

**e) Synthese von 8-Bromo-dibenzo[b,d]pyran-6-one**

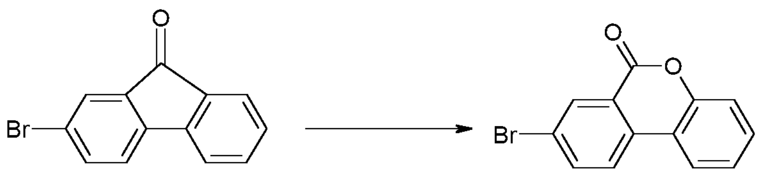

[3096-56-8]

100 g (386 mmol) 2-Bromfluorenon werden in 1000 mL Trifluoressigsäure, vorgelegt und auf 0°C gekühlt. Zu dieser Lösung werden langsam 100 g (637 mmol) Natriumpercarbonat (13-14% activer Sauerstoff) gegeben, und die Reaktionsmischung wird 1 h auf 10-15 °C gerührt. Anschließen wirde weiter über Nacht beim Raumtemperatur gerührt.Die Reaktion wird mit 1000 ml Wasser versetzt, die organische Phase abgetrennt und anschließend zur Trockene eingeengt. Der Rückstand wird mit Heptan verrieben, abgesaugt und in Vakuum bei 50°c getrocknet.

Ausbeute: 92 g (334 mmol) 86% d.Th.

Analog kann die folgende Verbindung hergestellt werden.

| Beispiel | Edukt 1 | Produkt | Ausbeute |
|---|---|---|---|
| e1 | 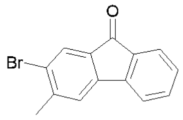 [52086-14-3] | 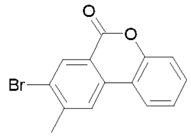 [52086-14-3] | 50% |
| e2 | 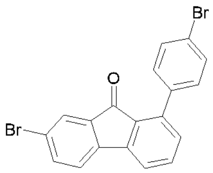 [171669-72-0] | 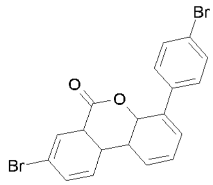 [171669-72-0] | 63% |
| e3 | 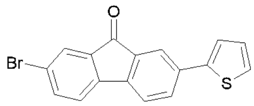 [1470370-96-7] | 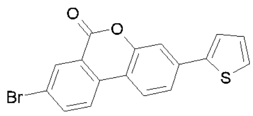 [1470370-96-7] | 61% |

**f) 8-(9-Phenyl-9H-carbazol-3-yl)-benzo[c]chromen-6-on**

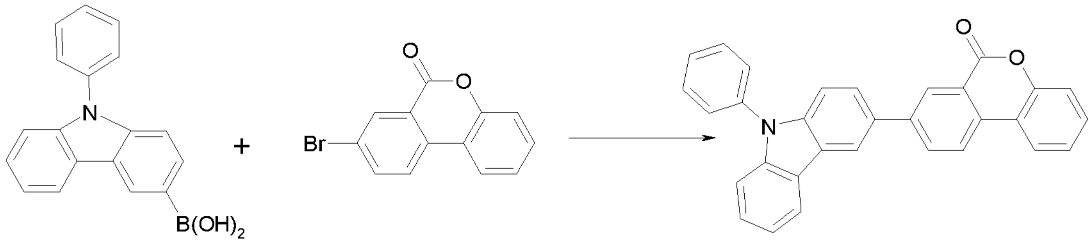

[854952-58-2]

17 g (67 mmol) 9-Phenyl-9H-carbazol-3 boronsäure, 22,2 g (81 mmol) 8-Brom-6h-dibenzo[b,d]pyran-6-on und 136 g (980 mmol) Trikaliumphosphat werden in 1000 mL THF, 300 mL Wasser suspendiert. Zu dieser Suspension werden 178mg (0,67 mmol) Triphenylphosphin und dann 152 mg (0.67 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rück¬stand wird aus Toluol/Heptan umkristallisiert. Der Rückstand wird aus Toluol/Heptan umkristallisiert und abschließend im Hochvakuum (p = 5 x 10-5 mbar, T = 377 °C) sublimiert. Die Ausbeute beträgt 23 g (54 mmol), entsprechend 82 % der Theorie.

Analog können folgende Verbindungen erhalten werden:

| | Edukt 1 | Edukt 2 | Produkt | Ausbeute |
|---|---|---|---|---|
| f1 | 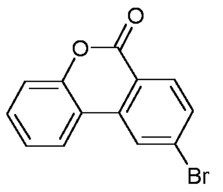 [1469912-47-7] | 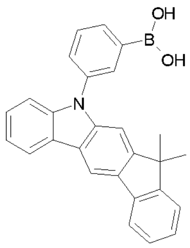 [1369587-64-3] | 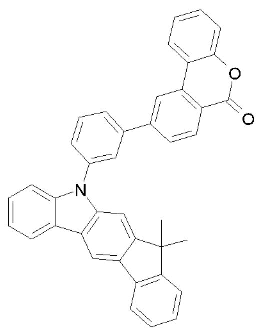 | 78% |
| f2 | 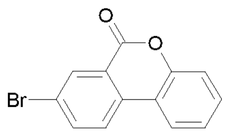 | 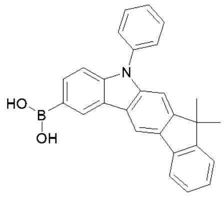 [1379585-25-7] | 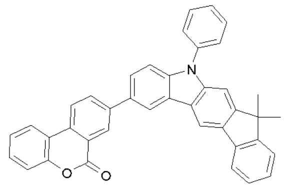 | 70% |
| f3 | 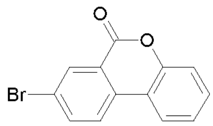 | 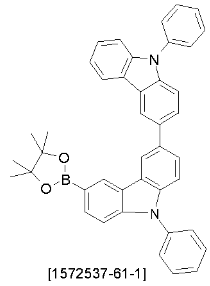 [1572537-61-1] | 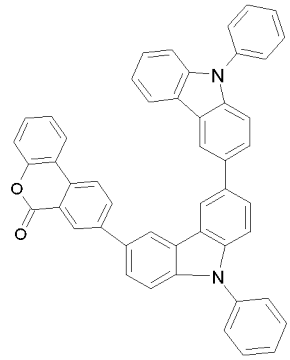 | 75% |
| f4 | 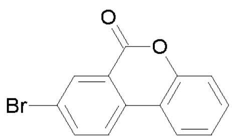 | 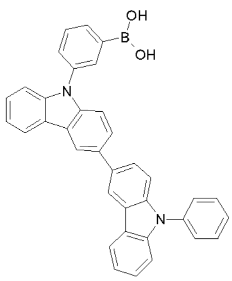 [1398394-64-3] | 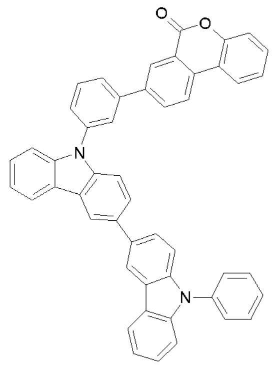 | 77% |
| f5 | 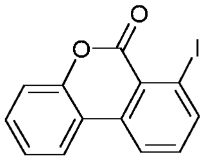 [1443434-82-9] | 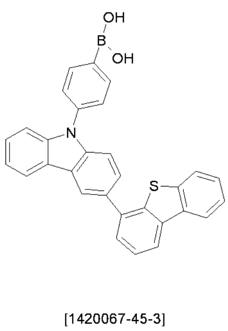 [1420067-45-3] | 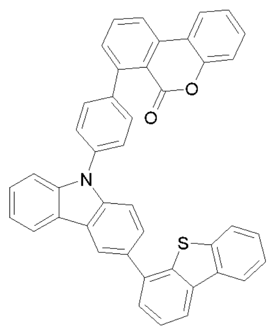 | 75% |

(fortgesetzt)

| | Edukt 1 | Edukt 2 | Produkt | Ausbeute |
|---|---|---|---|---|
| **f6** | 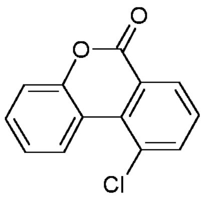 [1000391-25-2] | 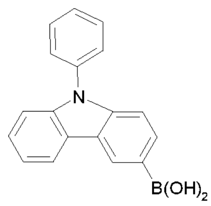 [854952-58-2] | 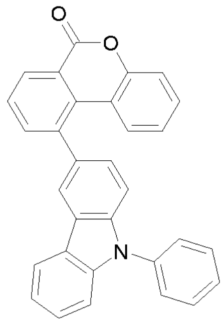 | 62% |
| f7 | 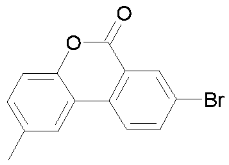 [100527-53-5] | 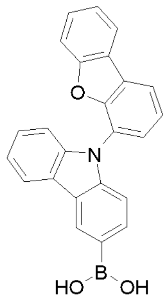 [1547397-15-8] | 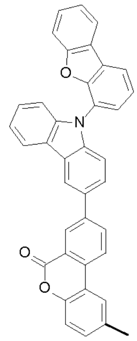 | 83% |
| **f8** | 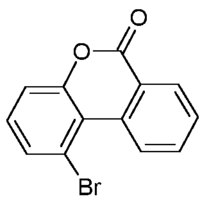 [928307-80-6] | 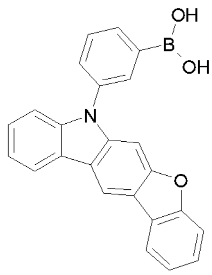 [1369587-56-3] | 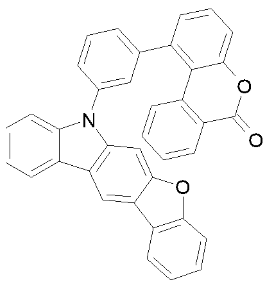 | 84% |
| **f9** | 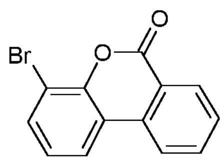 [158097-94-0] | 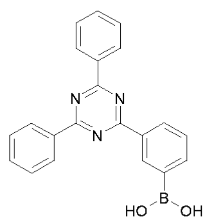 [1269508-31-7 ] | 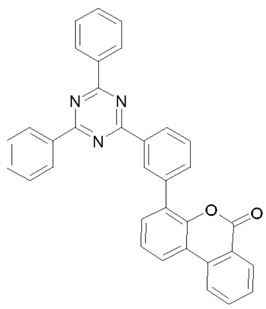 | 79% |
| f10 | 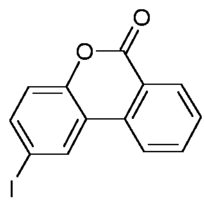 [151648-54-3] | 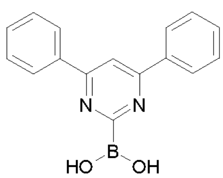 [1314221-56-1 ] | 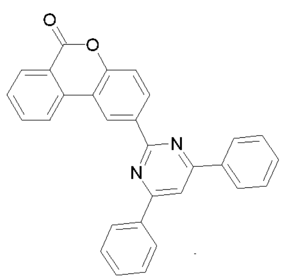 | 83% |

(fortgesetzt)

| | Edukt 1 | Edukt 2 | Produkt | Ausbeute |
|---|---|---|---|---|
| f11 | 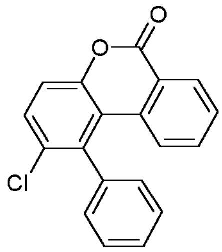 [1130141-14-8] | 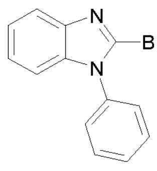 [1214723-25-7] | 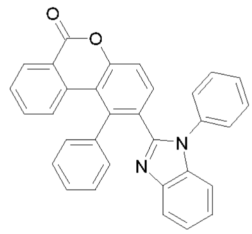 | 75% |
| f12 | 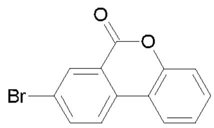 | 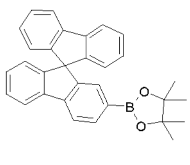 [1557257-88-1] | 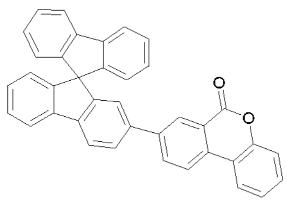 | 77% |
| f13 | 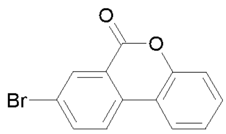 | 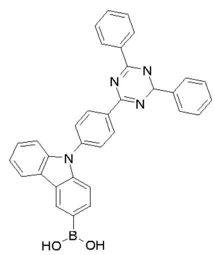 | 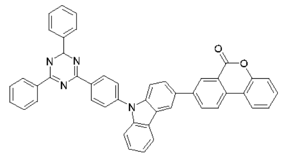 | 74% |
| f14 | 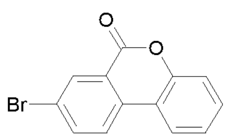 | 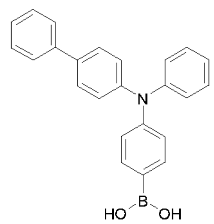 [1084334-86-0 ] | 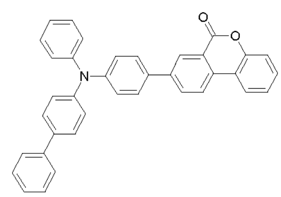 | 69% |
| f15 | 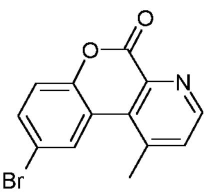 [585529-39-1] | 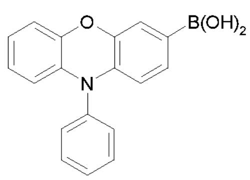 | 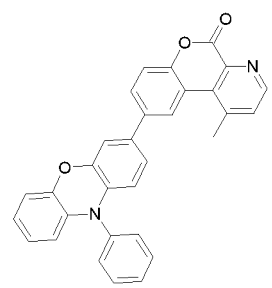 | 68% |
| f16 | 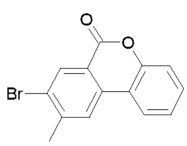 [52086-14-3] | 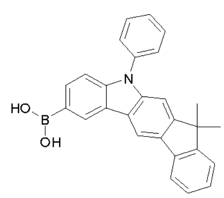 [1379585-25-7] | 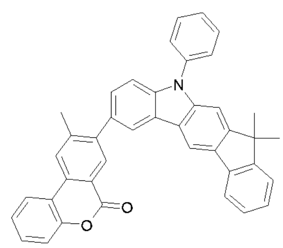 | 80% |

(fortgesetzt)

| | Edukt 1 | Edukt 2 | Produkt | Ausbeute |
|---|---|---|---|---|
| f17 | 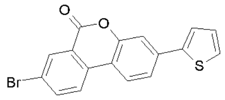 [1470370-96-7] | 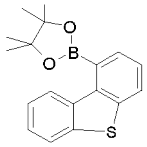 [1434286-69-7] | 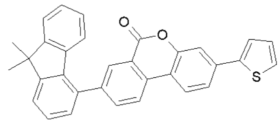 | 71% |
| f18 | 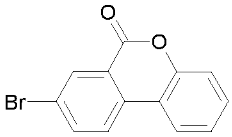 | 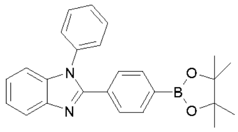 [1146340-38-6] | 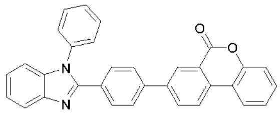 | 69% |
| f19 | 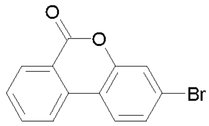 [1433988-13-6 ] | 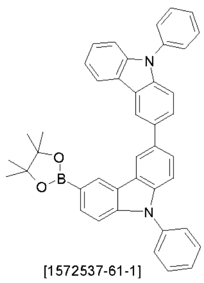 [1572537-61-1] | 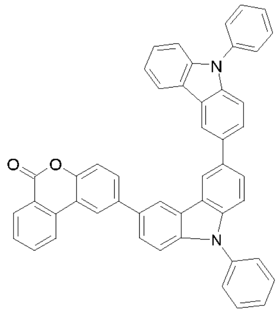 | 71% |
| f20 | 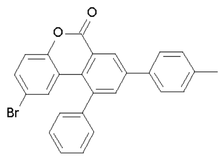 | 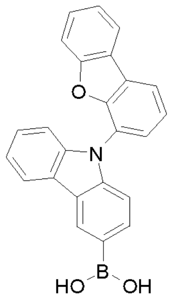 [1547397-15-8] | 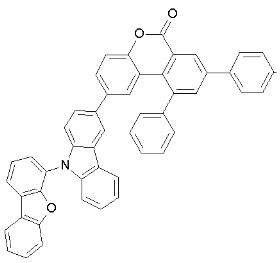 | 71% |

Analog kann die folgende Verbindung mit 0,5 eq. Boronsäure oder Bromid hergestellt werden.

| | Edukt 1 | Edukt 2 | Produkt | Ausbeute |
|---|---|---|---|---|
| f21 | 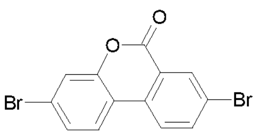 [ 18102-99-3] | 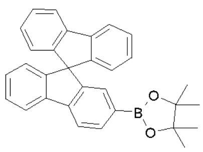 [1557257-88-1] | 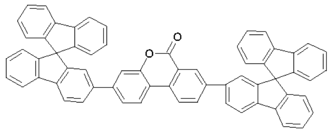 | 74% |

(fortgesetzt)

| | Edukt 1 | Edukt 2 | Produkt | Ausbeute |
|---|---|---|---|---|
| f22 | 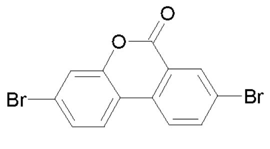 [ 18102-99-3] | 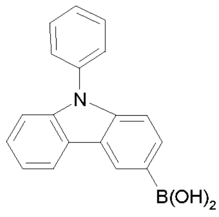 [854952-58-2] | 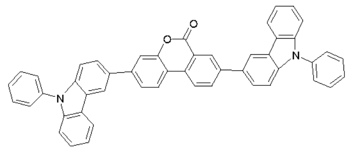 | 76% |
| f23 | 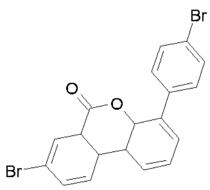 [171669-72-0] | 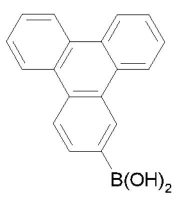 [654664-63-8] | 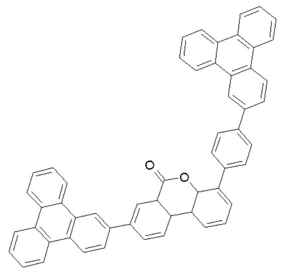 | 70% |
| f24 | 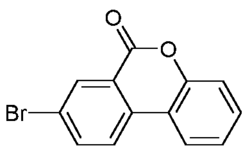 | 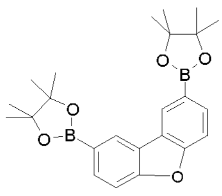 [1197989-83- | 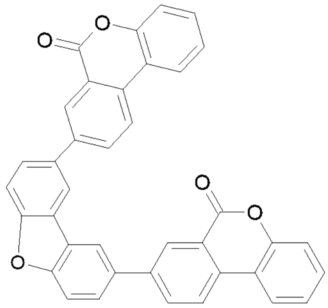 | 72% |

**g) 3-Hydroxy-8-methoxy-benzo[c]chromen-6-on**

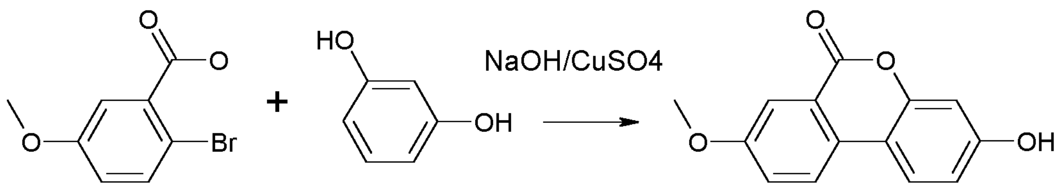


[22921-68-2 ]

83g (343 mmol) Natriumhydroxid werden in 1000 mL Wasser gelöst. 80 g (346 mmol) 2-Brom-5-methoxy-benzoesäure, 76,5 g (695 mmol) Benzen-1,3-diol werden zugegeben, 30 min. unter Rückfluss gekocht. Zu dieser Suspension wird eine Lösung aus 7g (28 mmol) Kupfersulfatpentahydrid gelöst in 300 mL Wasser gegeben, und die Reaktionsmischung wird 1 h unter Rückfluss erhitzt. Nach Erkalten wird der Feststoff abfiltrier, einmal mit 200 mL Heptan gemischt gewaschen und anschließend zur Trockene eingeengt. Die Ausbeute beträgt 50 g (165 mmol), entsprechend 80 % der Theorie.

Analog können folgende Verbindungen erhalten werden:

| | Edukt 1 | Edukt 2 | Produkt | Ausbeute |
|---|---|---|---|---|
| g1 | 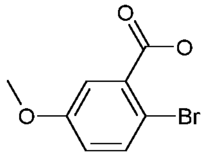 [22921-68-2 ] | 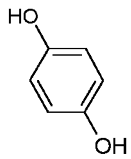 | 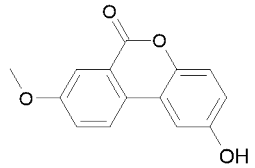 | 80% |
| g2 | 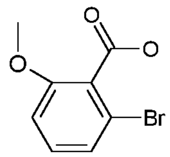 [72135-36-5] | 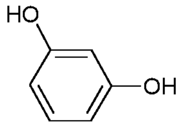 | 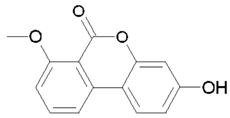 | 87% |
| g3 | 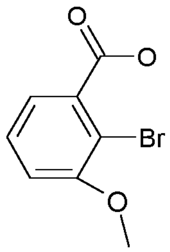 [88377-29-1] | 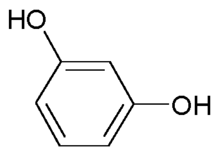 | 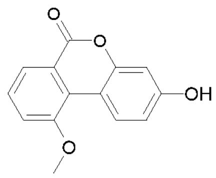 | 85% |

**h) Trifluor-methanesulfonsäure 8-methoxy-6-oxo-6H-benzo[c]chromen-3-yl -ester**

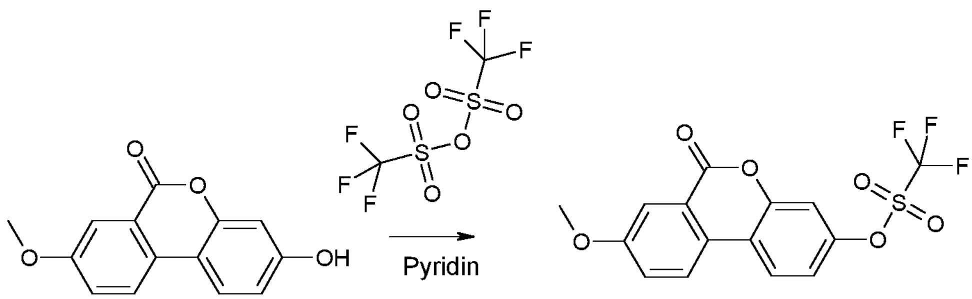


83 g (343 mmol) 3-Hydroxy-8-methoxy-benzo[c]chromen-6-on wird in 1200 mL Dichlormethan bei 0°C suspendiert. 37 mL (267 mmol) Triethylamin, 57 mL (343 mmol) Trifluoromethansulfonat in 250 mL Dichlormethan so das die Temperatur nicht über 5 °C steigt. Es wird weiter 1 h bei 0°C gerührt, dann über Nacht bei Raumtemperatur weiter gerührt. Der Feststoff wird abfiltriert, einmal mit 200 mL Wasser /MeOH gemischt gewaschen und anschließend zur Trockene eingeengt. Die Ausbeute beträgt 102g (275 mmol), entsprechend 80 % der Theorie.

Analog können folgende Verbindungen erhalten werden:

| | Edukt 1 | Produkt | Ausbeute |
|---|---|---|---|
| h1 | 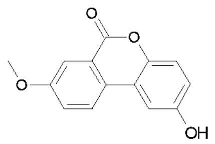 | 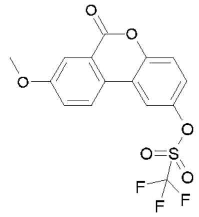 | 81% |
| h2 | 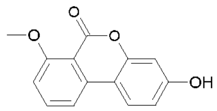 | 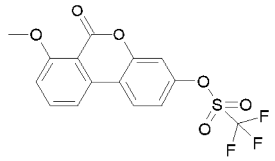 | 82% |
| h3 | 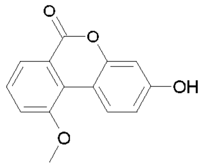 | 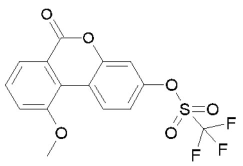 | 79% |

**j) 8-Methoxy-3-(9-phenyl-9H-carbazol-3-yl)-benzo[c]chromen-6-on**

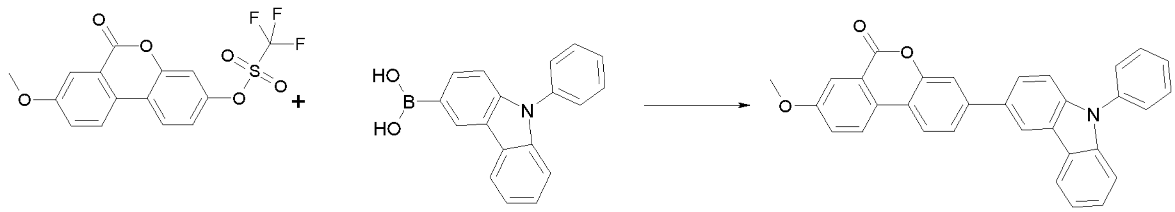

27 g (72mmol) Trifluor-methanesulfonsäure 8-methoxy-6-oxo-6H-benzo[c]chromen-3-yl-ester, 31 g (108 mmo) (9-Phenylcarbazol-3-yl)boronsäure und 8,3 g (7,2 mmol) Tetrakies(triphenylphosphin)palladium (0) und 25 mL(180 mmol) Triethylamin werden in 700 mL 1,2-Dimethoxyethan suspendiert und die Reaktionsmischung wird 16 h unter Rückflussbei 110 °C erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol/Heptan umkristallisiert. Die Ausbeute beträgt 20 g (42 mmol), entsprechend 60 % der Theorie.

Analog können folgende Verbindungen erhalten werden:

| | Edukt 1 | Edukt 2 | Produkt | Ausbeute |
|---|---|---|---|---|
| j1 | 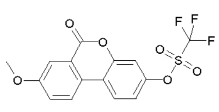 | 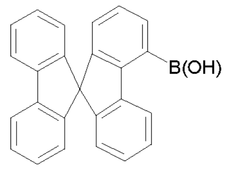 [1421789-05-0] | 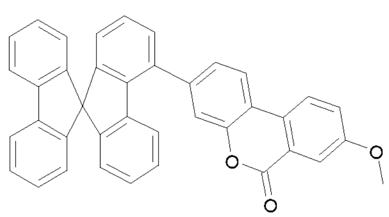 | 63% |

(fortgesetzt)

| | Edukt 1 | Edukt 2 | Produkt | Ausbeute |
|---|---|---|---|---|
| j2 | 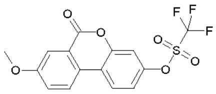 | 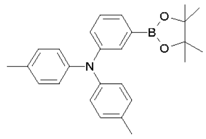 [1162753-18-5] | 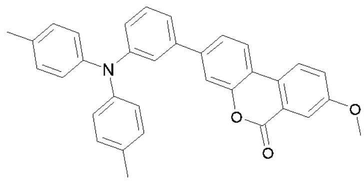 | 68% |
| j3 | 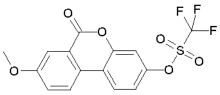 | 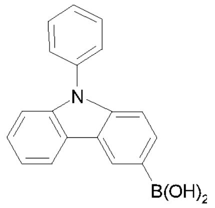 [854952-58-2] | 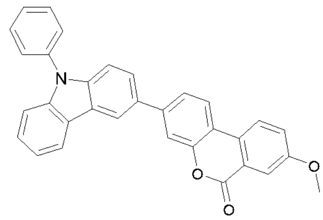 | 72% |
| j4 | 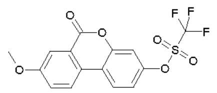 | 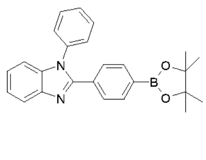 [1146340-38-6] | 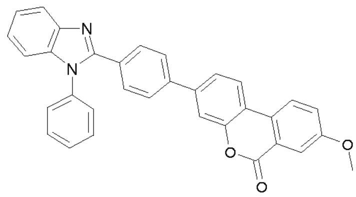 | 75% |
| j5 | 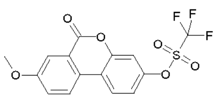 | 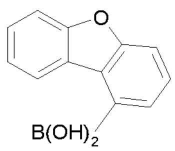 | 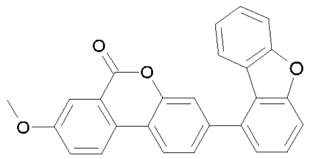 | 70% |
| j6 | 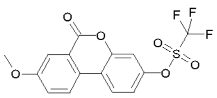 | 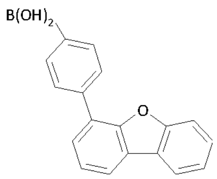 | 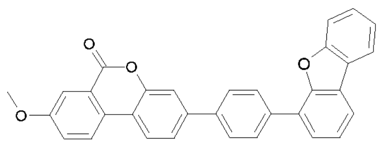 | 76% |
| j7 | 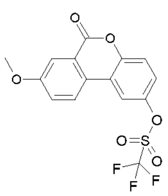 | 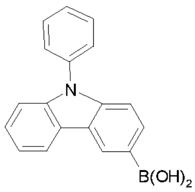 [854952-58-2] | 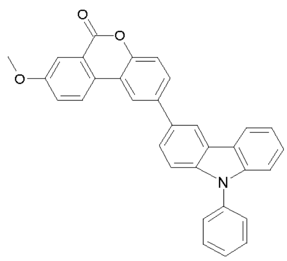 | 81% |
| j8 | 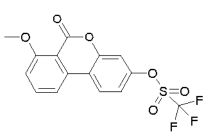 | 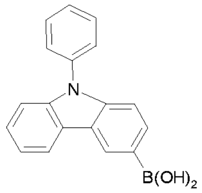 [854952-58-2] | 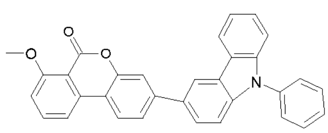 | 82% |

(fortgesetzt)

| | Edukt 1 | Edukt 2 | Produkt | Ausbeute |
|---|---|---|---|---|
| j9 | 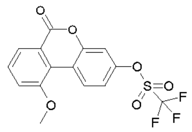 | 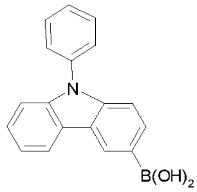 [854952-58-2] | 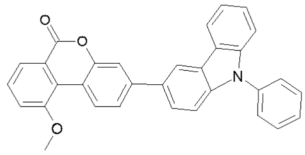 | 79% |
| j10 | 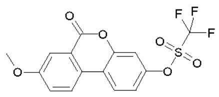 | 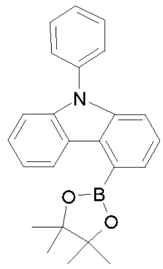 [1547492-13-6 ] | 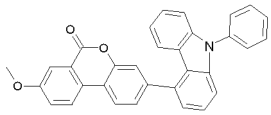 | 78% |

**i) 8-Hydroxy-3-(9-phenyl-9H-carbazol-3-yl)-benzo[c]chromen-6-on**

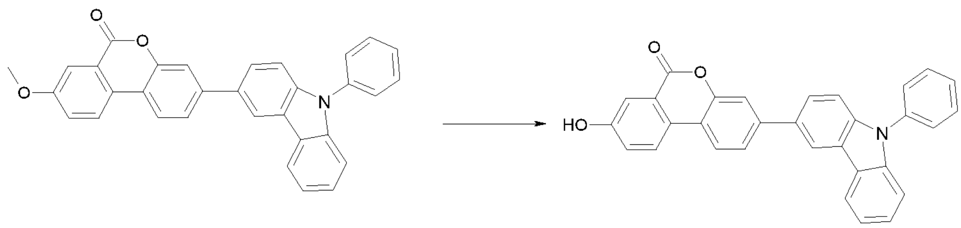

83 g (178 mmol) 8-Methoxy-3-(9-phenyl-9H-carbazol-3-yl)-benzo[c]chromen-6-on und 1,500 mLDichlromethan werden auf 0° C gekühlt und langsam mit 100 ml (1054 mmo) Tribromboran versetzt wird 16 h bei Raumtemoeratur gerührt. Danach wird mit 100 ml Methanol hydroliesiert und der Feststoff abgesaugt. Der Rückstand wird aus Heptan umkristallisiert. Die Ausbeute beträgt 46 g (103 mmol), entsprechend 57 % der Theorie.

Analog können folgende Verbindungen erhalten werden:

| | Edukt 1 | Produkt | Ausbeute |
|---|---|---|---|
| i1 | 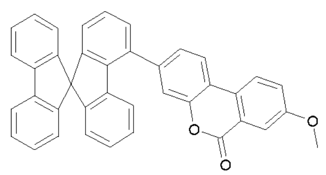 | 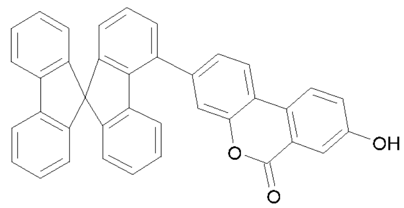 | 65% |
| i2 | 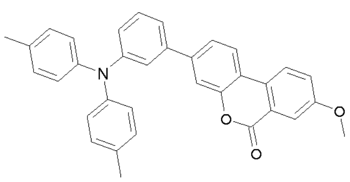 | 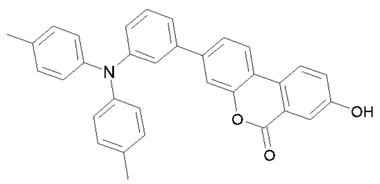 | 64% |

(fortgesetzt)

| | Edukt 1 | Produkt | Ausbeute |
|---|---|---|---|
| i3 | 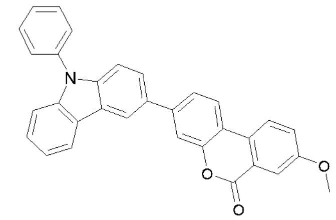 | 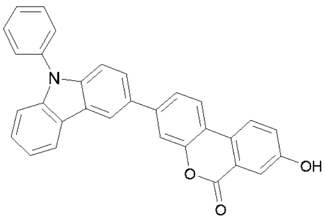 | 70% |
| i4 | 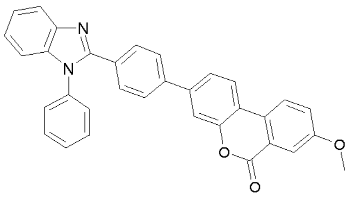 | 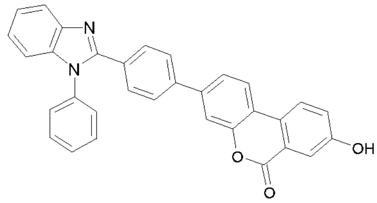 | 65% |
| i5 | 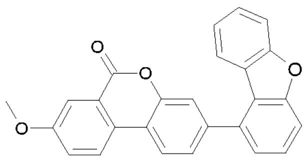 | 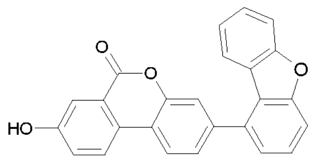 | 60% |
| i6 | 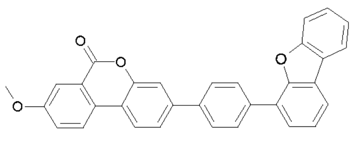 | 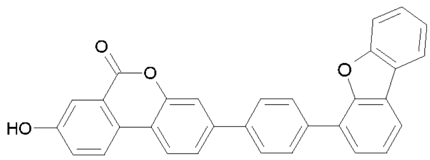 | 71% |
| i7 | 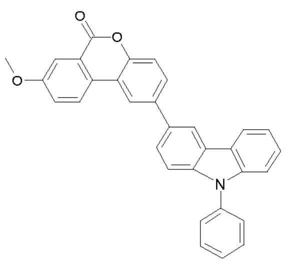 | 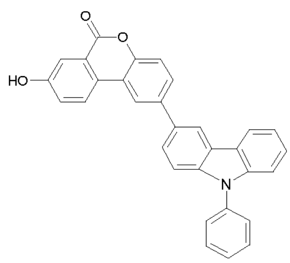 | 69% |
| i8 | 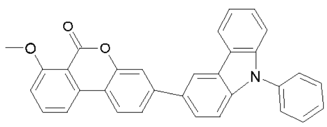 | 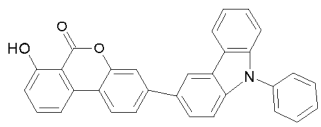 | 78% |
| i9 | 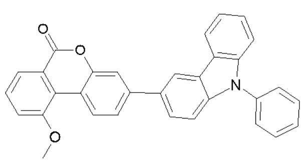 | 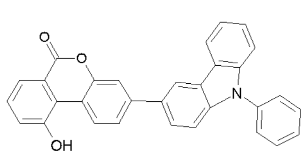 | 73% |
| i10 | 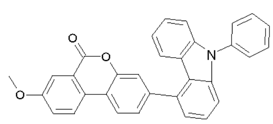 | 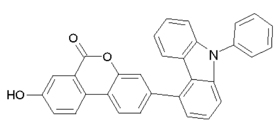 | 67% |

**k) Trifluoro-methanesulfonsäure 6-oxo-3-(9-phenyl-9H-carbazol-3-yl)-6H-benzo[c]chromen-8-yl ester**

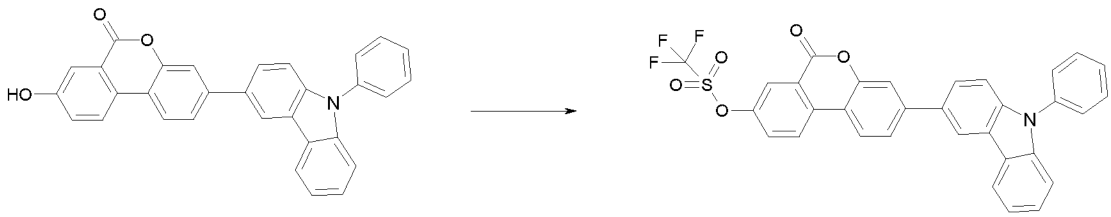

Analog können folgende Verbindungen nach Vorschrift (h) erhalten werden. Der Rückstand wird aus Toluol umkristallisiert. Die Ausbeute beträgt 81 % der Theorie.

Analog können folgende Verbindungen erhalten werden:

| | Edukt 1 | Produkt | Ausbeute |
|---|---|---|---|
| k1 | 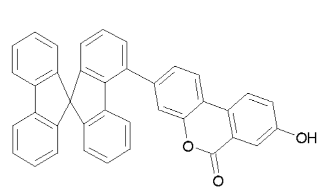 | 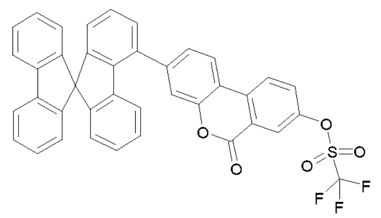 | 80% |
| k2 | 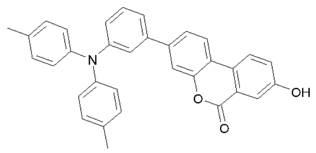 | 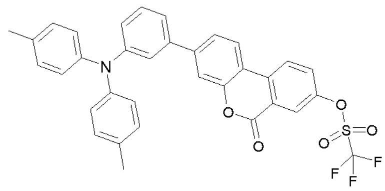 | 78% |
| k3 | 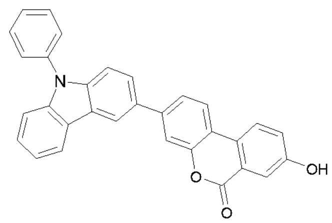 | 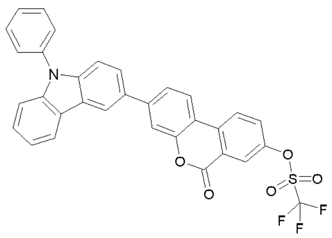 | 84% |
| k4 | 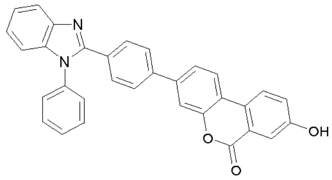 | 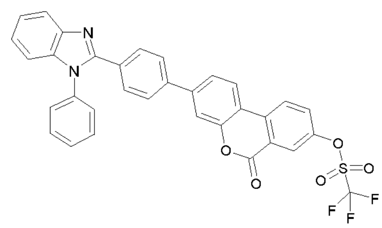 | 73% |
| k5 | 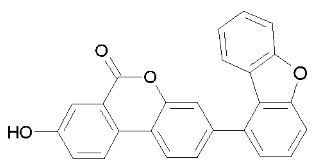 | 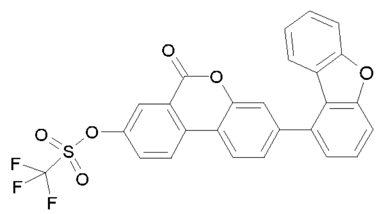 | 72% |

(fortgesetzt)

| | Edukt 1 | Produkt | Ausbeute |
|---|---|---|---|
| k6 | 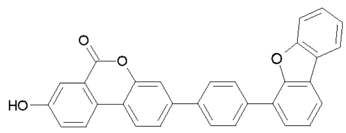 | 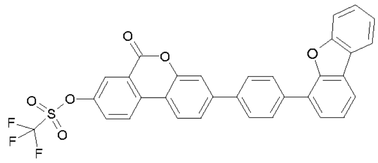 | 71% |
| k7 | 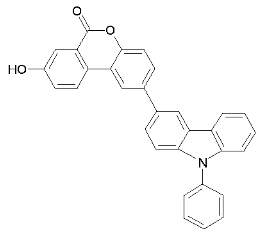 | 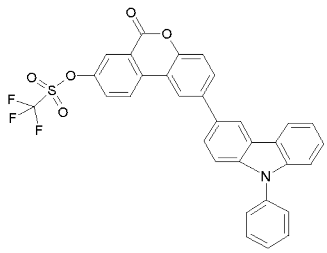 | 79% |
| k8 | 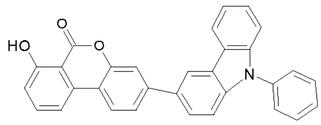 | 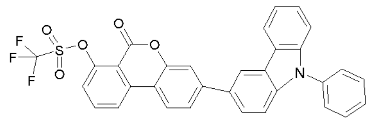 | 72% |
| k9 | 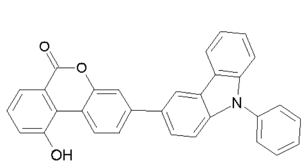 | 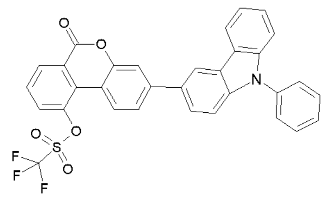 | 75% |
| k10 | 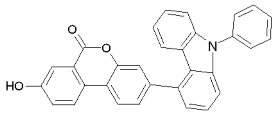 | 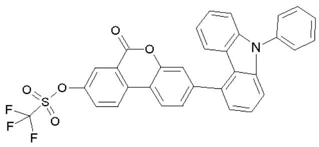 | 74% |

**l) Dibenzofuran-1-yl-3-(9-phenyl-9H-carbazol-3-yl)-benzo[c]chromen-6-on**

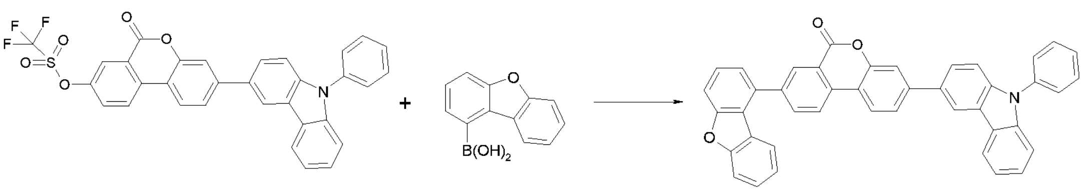

Analog können folgende Verbindungen nach Vorschrift (j) erhalten werden. Der Rückstand wird aus Toluol/Heptan umkristallisiert. Der Rückstand wird aus Toluol/Heptan umkristallisiert und abschließend im Hochvakuum (p = 5 x $10^{-5}$ mbar, T = 377 °C) sublimiert. Die Ausbeute beträgt 75 % der Theorie.

Analog können folgende Verbindungen erhalten werden:

| | Edukt 1 | | Produkt | Ausbeute |
|---|---|---|---|---|
| I1 | 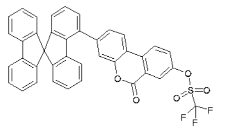 | 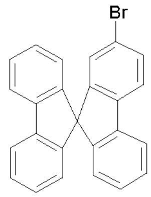 [171408-76-7] | 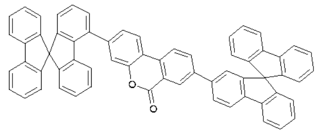 | 80% |
| I2 | 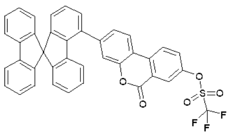 | 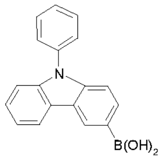 [854952-58-2] | 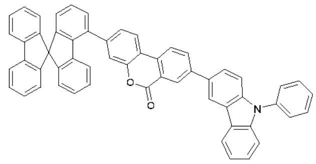 | 75% |
| I3 | 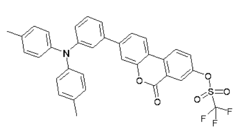 | 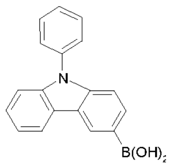 [854952-58-2] | 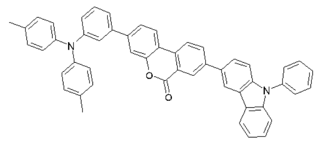 | 78% |
| I4 | 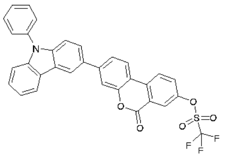 | 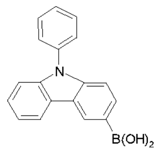 [854952-58-2] | 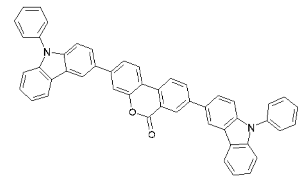 | 84% |
| I5 | 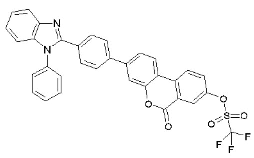 | 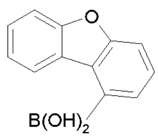 | 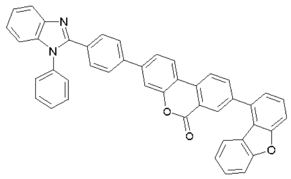 | 73% |
| I6 | 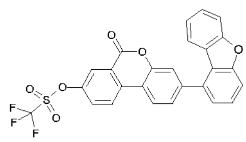 | 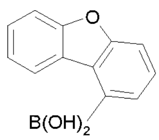 | 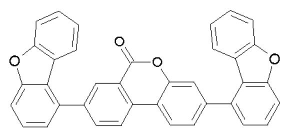 | 72% |
| I7 | 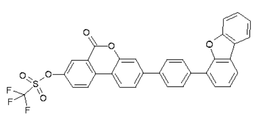 | 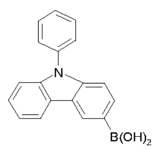 [854952-58-2] | 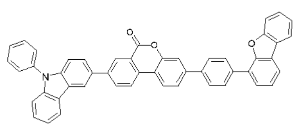 | 71% |
| I8 | 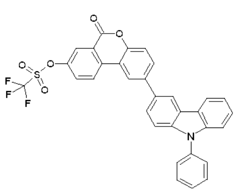 | 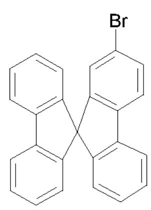 [171408-76-7] | 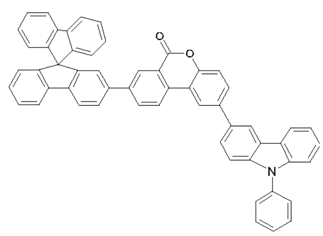 | 79% |

(fortgesetzt)

| | Edukt 1 | | Produkt | Ausbeute |
|---|---|---|---|---|
| I9 | 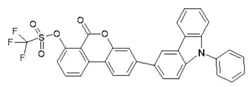 | 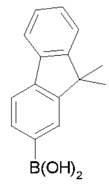 333432-28-3 | 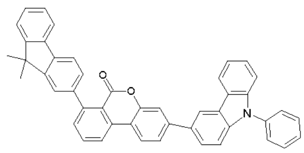 | 72% |
| I10 | 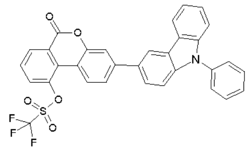 | 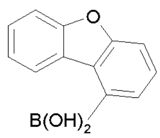 | 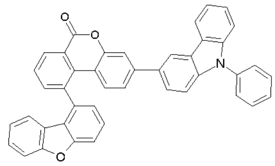 | 75% |
| I11 | 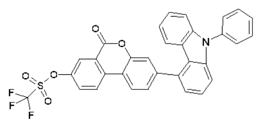 | 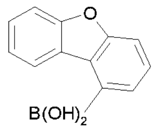 | 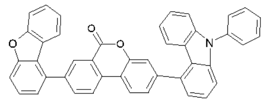 | 74% |
| I12 | 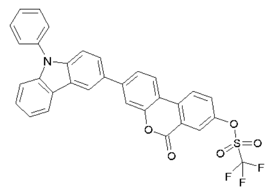 | 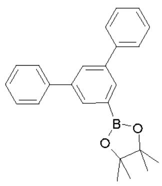 | 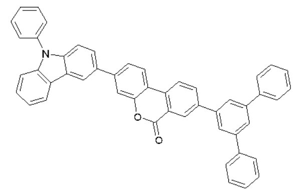 | 73% |
| I13 | 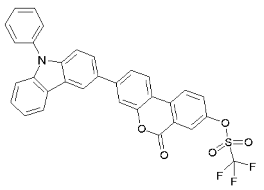 | 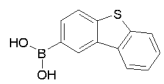 | 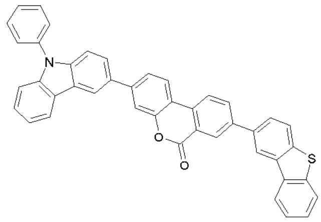 | 74% |

**m) 8-(12,12-Dimethyl-12H-10-aza-indeno[2,1-b]fluoren-10-yl)-benzo[c]chromen-6-on**

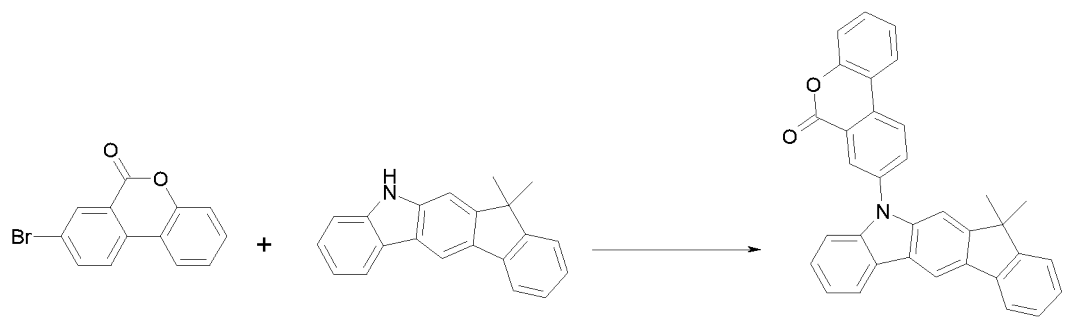

31 g (115 mmol) 8-Brom-6h-dibenzo[b,d]pyran-6-on, 27,7 g (98 mmol) 12,12-Dimethyl-10,12-dihydro-10-aza-indeno[2,1-b]fluoren , 30.5 g NaOtBu werden in 1.5 L p-Xylol suspendiert. Zu dieser Suspension werden 0.5 g (2.11 mmol) $Pd(OAc)_2$ und 6 ml einer 1M Tri-tert-butylphosphin (1M Lösung in Toluol) gegeben. Die Reaktionsmischung wird

16 h unter Rückfluss erhitzt. Nach Erkalten wird die organische Phase abgetrennt, dreimal mit je 200 mL Wasser gewaschen und anschließend zur Trockene eingeengt. Der Rückstand wird mit Toluol heiß extrahiert, aus Toluol umkristallisiert und abschließend im Hochvakuum sublimiert, Die Reinheit Beträgt 99,9% nach HPLC. Die Ausbeute beträgt 33 g (69 mmol), entsprechend 71 % der Theorie.

Analog können folgende Verbindungen hergestellt werden.

| | Edukt 1 | Edukt 2 | Produkt | Ausbeute |
|---|---|---|---|---|
| m1 | 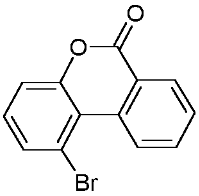 [928307-80-6] | 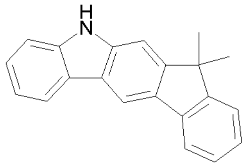 [1257220-47-5] | 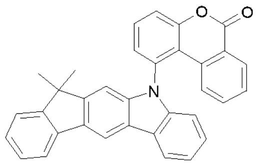 | 70% |
| m2 | 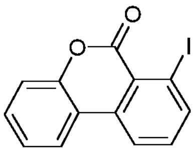 [1443434-82-9] | 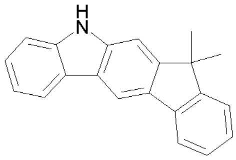 [1257220-47-5] | 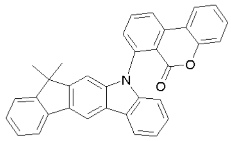 | 74% |
| m3 | 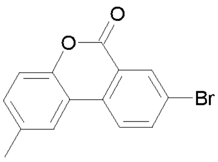 [100527-53-5] | 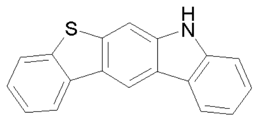 [1255309-04-6] | 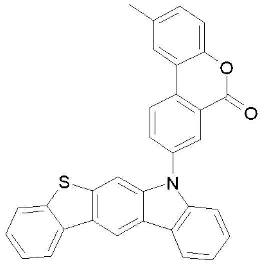 | 81% |
| m4 | 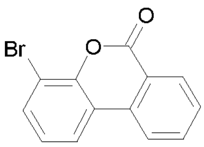 [158097-94-0] | 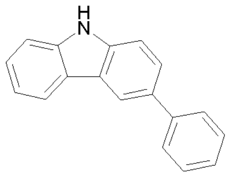 [103012-26-6 ] | 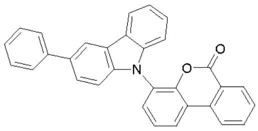 | 80% |
| m5 | 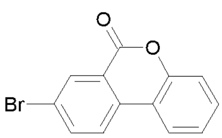 | 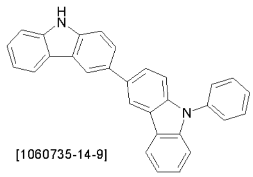 [1060735-14-9] | 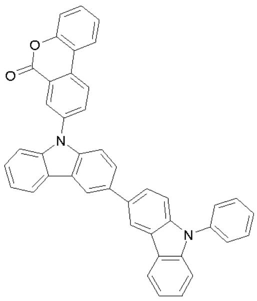 | 79% |

(fortgesetzt)

| | Edukt 1 | Edukt 2 | Produkt | Ausbeute |
|---|---|---|---|---|
| m6 | 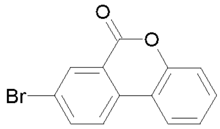 | 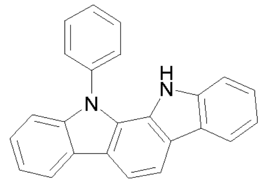 [1024598-06-8] | 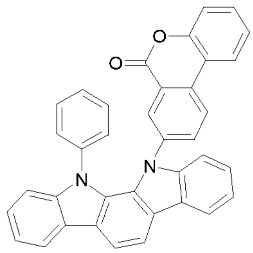 | 62% |
| m7 | 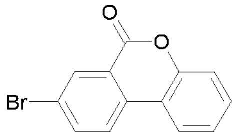 | 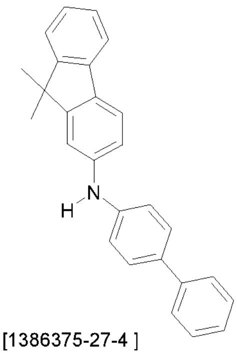 [1386375-27-4 ] | 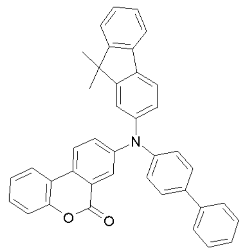 | 75% |
| m8 | 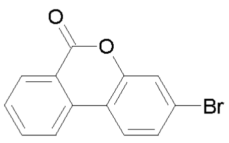 [1433988-13-6 ] | 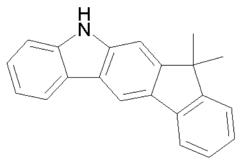 [1257220-47-5] | 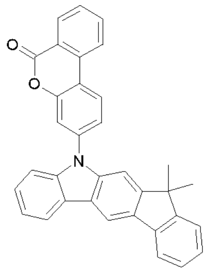 | 79% |
| m9 | 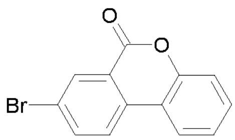 | 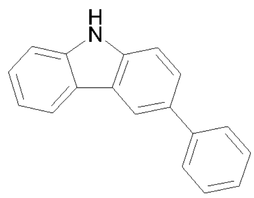 [103012-26-6 ] | 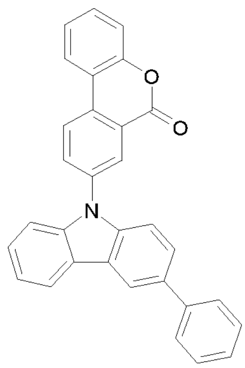 | 81% |
| m10 | 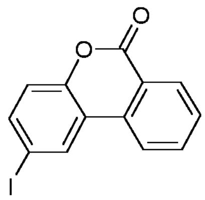 [151648-54-3] | 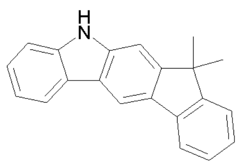 [1257220-47-5] | 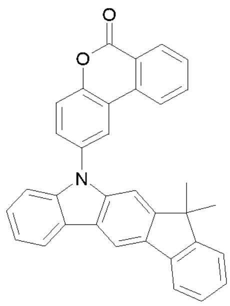 | 78% |

**Herstellung der OLEDs**

In den folgenden Anwendungsbeispielen Beispielen R1 bis R16 (siehe Tabellen 1 und 2) werden die Daten verschiedener OLEDs dargelegt.

**Vorbehandlung für die Beispiele R1-R16:** Glasplättchen, die mit strukturiertem ITO (Indium Zinn Oxid) der Dicke 50 nm beschichtet sind, werden zur verbesserten Prozessierung mit 20 nm PEDOT:PSS beschichtet (Poly-(3,4-ethylendioxythiophen) poly(styrolsulfonat), bezogen als CLEVIOS™ P VP AI 4083 von Heraeus Precious Metals GmbH Deutschland, aus wässriger Lösung aufgeschleudert). Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / optionale Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:IC3:TEG1 (55%:35%:10%) bedeutet hierbei, dass das Material IC1 in einem Volumenanteil von 55%, IC3 in einem Anteil von 35% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/$m^2$ bestimmt und daraus die CIE 1931 x und y Farbkoordinaten berechnet. Die Angabe U1000 in Tabelle 2 bezeichnet die Spannung, die für eine Leuchtdichte von 1000 cd/$m^2$ benötigt wird. SE1000 und LE1000 bezeichnen die Strom- bzw. Leistungseffizienz, die bei 1000 cd/$m^2$ erreicht werden. EQE1000 schließlich bezeichnet die externe Quanteneffizienz bei einer Betriebsleuchtdichte von 1000 cd/$m^2$.

Die Daten der verschiedenen OLEDs sind in Tabelle 2 zusammengefasst.

Hierbei werden die erfindungsgemäßen Strukturen in der Emissionschicht (EML) als Matrixmaterial und in der Elektronentransportschicht (ETL) zur Leitung von Elektronen eingesetzt. Zusätzlich können die erfindungsgemäßen Strukturen als Lochblockierschicht (HBL) verwendet werden.

Tabelle 1: Aufbau der OLEDs - *nicht erfindungsgemäß

| Bsp | HTL Dicke | IL Dicke | EBL Dicke | EML Dicke | HBL Dicke | ETL Dicke | EIL Dicke |
|---|---|---|---|---|---|---|---|
| R1* | SpA1 70nm | HATCN 5nm | SpMA1 90nm | EG1:TEG1 (90%:10%) 30nm | IC1 10nm | ST2:LiQ (50%: 50%) 30nm | --- |
| R2* | SpA1 70nm | HATCN 5nm | SpMA1 90nm | EG2:TEG1 (90%:10%) 30nm | IC1 10nm | ST2:LiQ (50%: 50%) 30nm | --- |
| R3* | SpA1 70nm | HATCN 5nm | SpMA1 90nm | EG3:TEG1 (90%:10%) 30nm | IC1 10nm | ST2:LiQ (50%: 50%) 30nm | --- |
| R4* | SpA1 70nm | HATCN 5nm | SpMA1 90nm | EG4:TEG1 (90%:10%) 30nm | IC1 10nm | ST2:LiQ (50%: 50%) 30nm | --- |
| R5* | SpA1 70nm | HATCN 5nm | SpMA1 90nm | IC1:TEG1 (90%:10%) 30nm | IC1 10nm | EG5:LiQ (50%: 50%) 30nm | --- |
| R6* | SpA1 70nm | HATCN 5nm | SpMA1 90nm | IC1:TEG1 (90%:10%) 30nm | IC1 10nm | EG6:ST2 (60%: 40%) 30nm | LiF 1nm |
| R7 | SpA1 70nm | HATCN 5nm | SpMA1 90nm | EG7:IC3:TEG1 (65%: 30%:5%) 30nm | IC1 10nm | ST2:LiQ (50%: 50%) 30nm | --- |
| R8* | SpA1 70nm | HATCN 5nm | SpMA1 90nm | EG8:TEG1 (90%:10%) 30nm | IC1 10nm | ST2:LiQ (50%: 50%) 30nm | --- |

(fortgesetzt)

| Bsp | HTL Dicke | IL Dicke | EBL Dicke | EML Dicke | HBL Dicke | ETL Dicke | EIL Dicke |
|---|---|---|---|---|---|---|---|
| R9* | SpA1 70nm | HATCN 5nm | SpMA1 90nm | IC1:TEG1 (90%:10%) 30nm | EG9 10nm | ST2:LiQ (50%: 50%) 30nm | --- |
| R10 | SpA1 70nm | HATCN 5nm | SpMA1 90nm | IC1:TEG1 (90%:10%) 30nm | IC1 10nm | EG10:LiQ (50%: 50%) 30nm | --- |
| R11* | HATCN 5nm | SpMA1 70nm | SpMA2 15nm | IC1:EG11 :TEY1 (45%: 45%:10%) 25nm | --- | ST2 45nm | LiQ 3nm |
| R12* | HATCN 5nm | SpMA1 70nm | SpMA2 15nm | L1:EG12:TEY1 (45%: 45%:10%) 25nm | --- | ST2 45nm | LiQ 3nm |
| R13* | SpA1 90nm | HATCN 5nm | SpMA1 130nm | EG13:TER3 (92%:8%) 40nm | --- | ST2:LiQ (50%: 50%) 40nm | --- |
| R14* | SpA1 70nm | HATCN 5nm | SpMA1 90nm | IC1:TEG1 (90%:10%) 30nm | IC1 10nm | EG14:LiQ (50%: 50%) 30nm | --- |
| R15* | SpA1 70nm | HATCN 5nm | SpMA1 90nm | EG15:TEG1 (90%:10%) 30nm | IC1 10nm | ST2:LiQ (50%: 50%) 30nm | --- |
| R16* | SpA1 70nm | HATCN 5nm | SpMA1 90nm | EG16:TEG1 (90%:10%) 30nm | IC1 10nm | ST2:LiQ (50%: 50%) 30nm | --- |

Tabelle 2: Daten der OLEDs

| Bsp. | U1000 (V) | SE1000 (cd/A) | LE1000 (lm/W) | EQE 1000 | CIE x/y bei 1000 cd/$m^2$ |
|---|---|---|---|---|---|
| R1 | 3.6 | 57 | 50 | 15.7% | 0.31/0.64 |
| R2 | 3.3 | 58 | 55 | 15.5% | 0.33/0.64 |
| R3 | 3.6 | 56 | 49 | 15.4% | 0.34/0.62 |
| R4 | 3.4 | 60 | 55 | 16.1% | 0.33/0.63 |
| R5 | 3.4 | 64 | 59 | 17.2% | 0.33/0.63 |
| R6 | 3.6 | 64 | 56 | 17.4% | 0.32/0.64 |
| R7 | 3.4 | 57 | 53 | 15.5% | 0.30/0.65 |
| R8 | 3.3 | 60 | 57 | 16.5% | 0.32/0.64 |
| R9 | 3.5 | 61 | 55 | 17.2% | 0.32/0.63 |
| R10 | 3.3 | 64 | 61 | 17.0% | 0.34/0.63 |
| R11 | 2.9 | 84 | 91 | 25.1% | 0.45/0.54 |
| R12 | 3.1 | 82 | 83 | 24.0% | 0.43/0.56 |
| R13 | 4.7 | 11 | 7 | 12.2% | 0.67/0.33 |
| R14 | 3.5 | 60 | 54 | 17.0% | 0.33/0.62 |
| R15 | 3.3 | 58 | 55 | 15.6% | 0.33/0.63 |
| R16 | 3.2 | 57 | 56 | 15.3% | 0.32/0.64 |

Tabelle 3: Strukturformeln der Materialien für die OLEDs

| | |
|---|---|
| 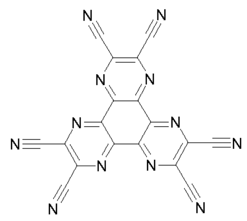 | 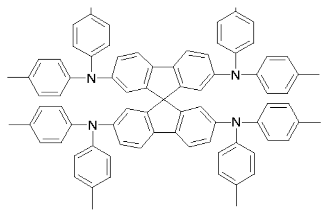 |
| HATCN | SpA1 |
| 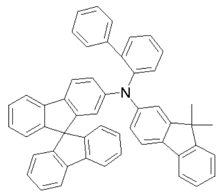 | 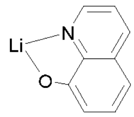 |
| SpMA1 | LiQ |
| 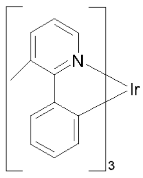 | 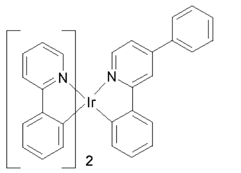 |
| TEG1 | TEY1 |
| 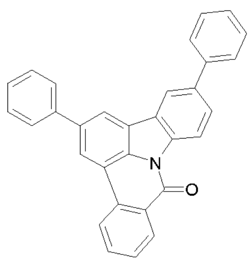 | 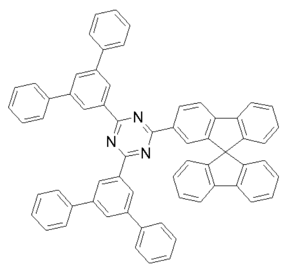 |
| L1 | ST2 |
| 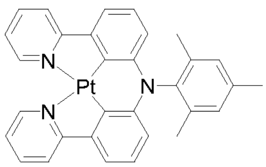 | 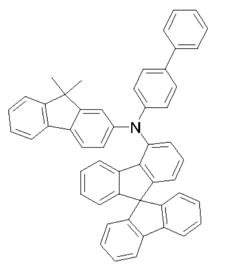 |
| TER3 | SpMA2 |
| 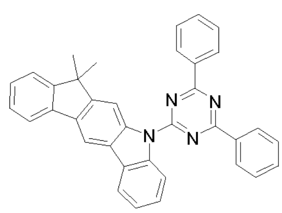 | 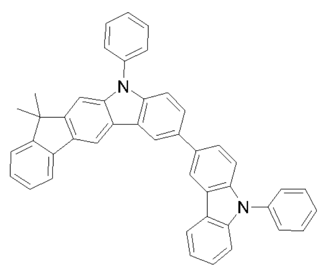 |

(fortgesetzt)

| IC1 | IC3 |
|---|---|
| 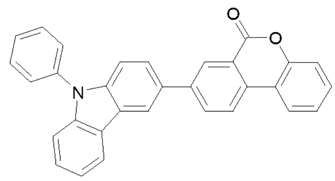 | 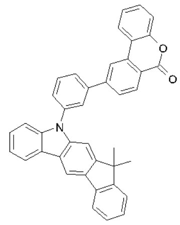 |
| EG1 | EG2 |
| 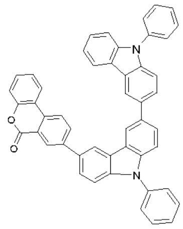 | 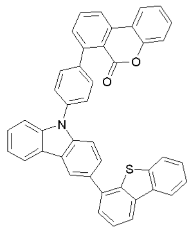 |
| EG3 | EG4 |
| 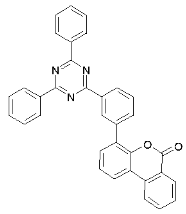 | 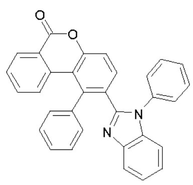 |
| EG5 | EG6 |
| 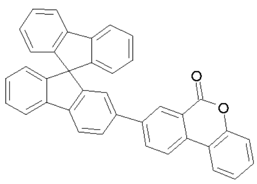 | 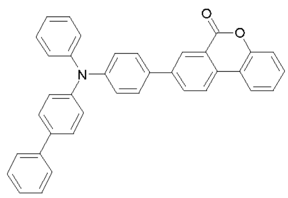 |
| EG7 | EG8 |
| 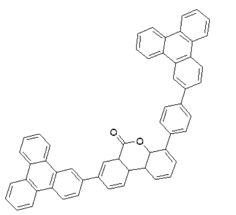 | 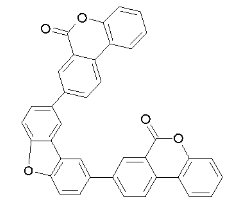 |
| EG9 | EG10 |
| 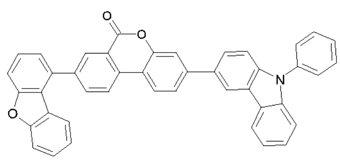 | 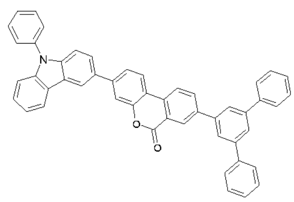 |
| EG11 | EG12 |

(fortgesetzt)

| | |
|---|---|
| 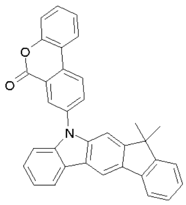 | 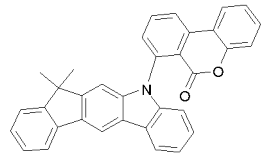 |
| EG13 | EG14 |
| 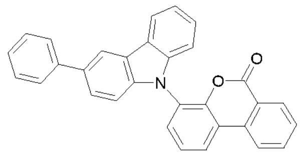 | 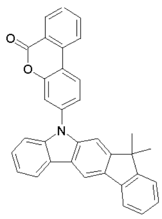 |
| EG15 | EG16 |

**Patentansprüche**

**1.** Elektronische Vorrichtung, enthaltend mindestens eine Verbindung, umfassend Strukturen der Formel (I), die sublimierbar ist und ein Molekulargewicht von höchstens 5000 g/mol hat,

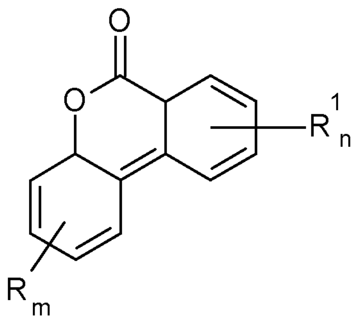

Formel (I),

wobei für die verwendeten Symbole gilt:
alle Gruppen R und/oder $R^1$ in Formel (I) stellen eine Gruppe L dar, ausgewählt aus den Formeln (L-7) bis (L-26),

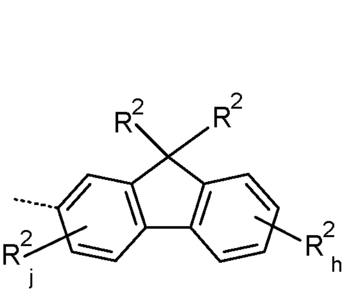

Formel (L-7)

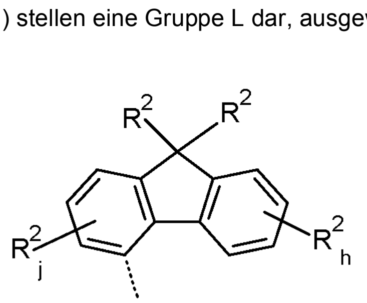

Formel (L-8)

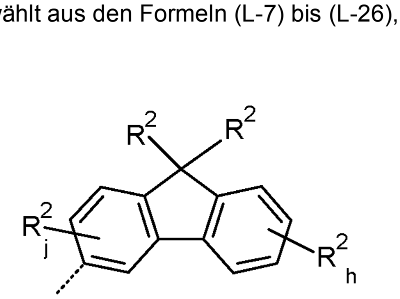

Formel (L-9)

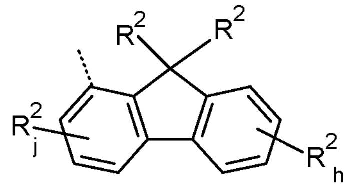
Formel (L-10)

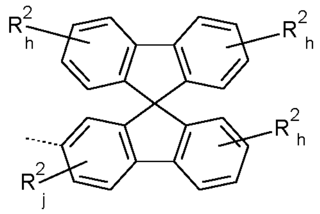
Formel (L-11)

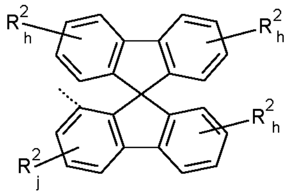
Formel (L-12)

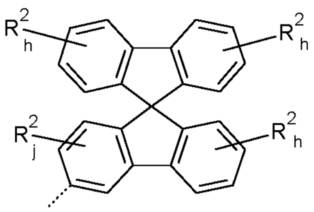
Formel (L-13)

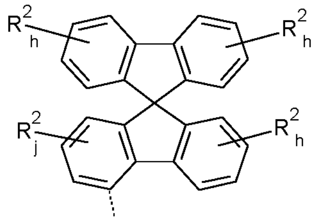
Formel (L-14)

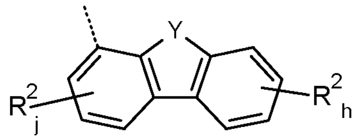
Formel (L-15)

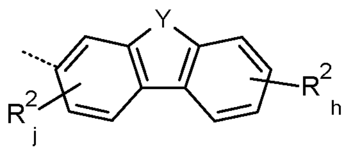
Formel (L-16)

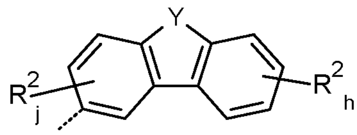
Formel (L-17)

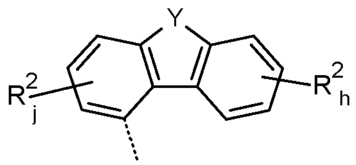
Formel (L-18)

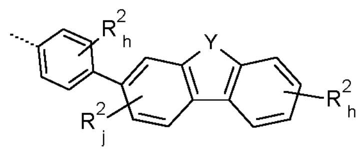
Formel (L-19)

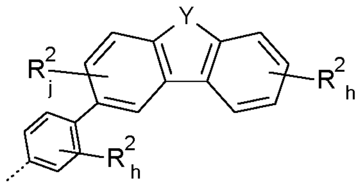
Formel (L-20)

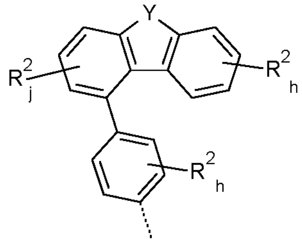
Formel (L-21)

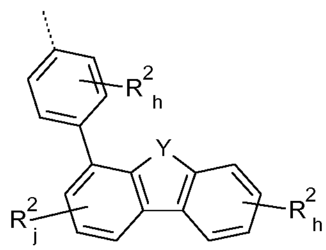
Formel (L-22)

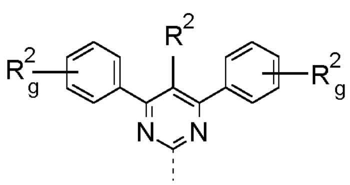
Formel (L-23)

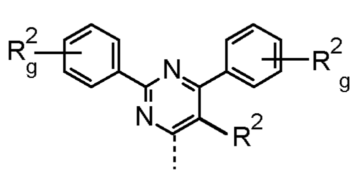
Formel (L-24)

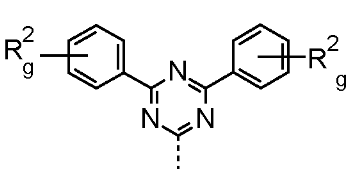
Formel (L-26),

wobei die gestrichelte Bindung die Anbindungsposition markiert,
g 0, 1, 2, 3, 4 oder 5 ist,
h 0, 1, 2, 3 oder 4 ist,
j 0, 1, 2 oder 3 ist,
Y für O oder S steht,
$R^2$ ist bei jedem Auftreten gleich oder verschieden H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^3)_3$, $B(OR^3)_2$, $OSO_2R^3$, eine geradkettige Alkyl-, Alkoxy- oder Thioalkoxygruppe mit 1 bis 10 C-Atomen oder eine verzweigte oder cyclische Alkyl-, Alkoxy- oder Thioalkoxygruppe mit 3 bis 10 C-Atomen, die jeweils mit einem oder mehreren Resten $R^3$ substituiert sein kann, wobei eine oder mehrere nicht benachbarte $CH_2$-Gruppen durch C≡C, $Si(R^3)_2$, $Ge(R^3)_2$, $Sn(R^3)_2$, C=O, C=S, C=Se, $P(=O)(R^3)$, SO, $SO_2$, O, S oder $CONR^3$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 24 aromatischen Ringatomen, das jeweils durch einen oder mehrere Reste $R^3$ substituiert sein kann, oder eine Aryloxy- oder Heteroaryloxygruppe mit 5 bis 24 aromatischen Ringatomen, die durch einen oder mehrere Reste $R^3$ substituiert sein kann; dabei können zwei oder mehrere benachbarte Substituenten $R^2$ auch miteinander ein mono- oder polycyclisches, aliphatisches oder aromatisches Ringsystem bilden;
$Ar^1$ ist bei jedem Auftreten gleich oder verschieden ein aromatisches oder heteroaromatisches Ringsystem mit 5 bis 30 aromatischen Ringatomen, das mit einem oder mehreren Resten $R^2$ substituiert sein kann; dabei können auch zwei Reste $Ar^1$, welche an dasselbe Phosphoratom binden, durch eine Einfachbindung oder eine Brücke, ausgewählt aus $B(R^3)$, $C(R^3)_2$, $Si(R^3)_2$, C=O, $C=NR^3$, $C=C(R^3)_2$, O, S, S=O, $SO_2$, $N(R^3)$, $P(R^3)$ und $P(=O)R^3$, miteinander verknüpft sein;
$R^3$ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten $R^3$ auch miteinander ein mono- oder polycyclisches, aliphatisches oder aromatisches Ringsystem bilden;
m, n ist jeweils unabhängig voneinander 0, 1 oder 2;
und
die Summe aus m und n ist 1 oder 2.

2. Vorrichtung gemäß Anspruch 1, **dadurch gekennzeichnet, dass** die Struktur gemäß Formel (I) zwei Gruppen L aufweist.

3. Vorrichtung gemäß Anspruch 1 oder 2, **dadurch gekennzeichnet, dass** die Gruppe L in Formel (I) mindestens eine Fluorenyl- und/oder Spirobifluorenyl-Gruppe umfasst.

4. Vorrichtung gemäß einem der Ansprüche 1 bis 3, **dadurch gekennzeichnet, dass** der Index m 1 oder 2 ist und der Rest R eine Gruppe L darstellt, wobei der Index n gleich 0 ist.

5. Vorrichtung gemäß einem der Ansprüche 1 bis 4, **dadurch gekennzeichnet, dass** der Index n 1 oder 2 ist und der Rest $R^1$ eine Gruppe L darstellt, wobei der Index m gleich 0 ist.

6. Vorrichtung gemäß einem der Ansprüche 1 bis 5, **dadurch gekennzeichnet, dass** die Gruppe L in Formel (I) mindestens eine Heteroarylgruppe mit einem Stickstoffatom umfasst.

7. Vorrichtung gemäß einem der Ansprüche 1 bis 6, **dadurch gekennzeichnet, dass** die Gruppe L in Formel (I) mindestens eine Diazin-, Triazin-, Benzothiophen- und/oder Benzofuran-Gruppe umfasst.

8. Vorrichtung gemäß Anspruch 1 enthaltend eine Verbindung gemäß den folgenden Formeln

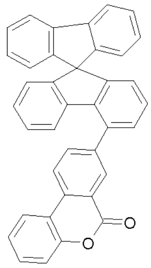

Formel 2

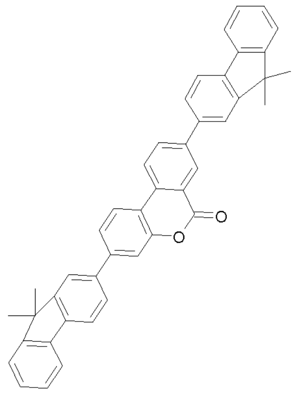

Formel 3

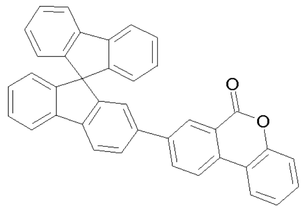

EG7

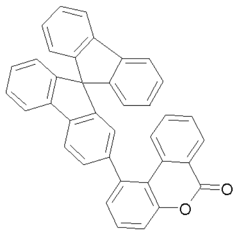

Formel 4

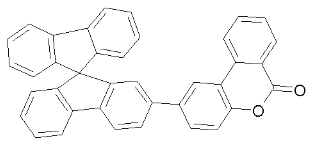

Formel 5

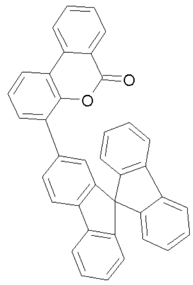

Formel 6

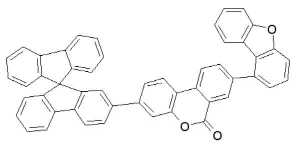

Formel 8

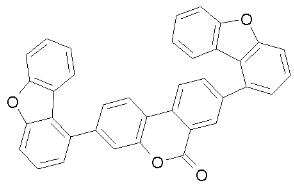

Formel 9

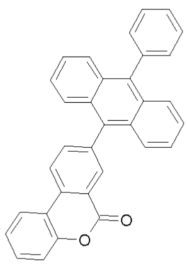

Formel 11

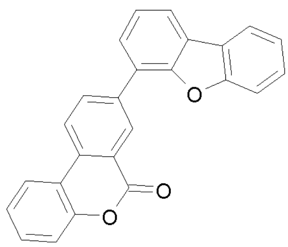

Formel 16

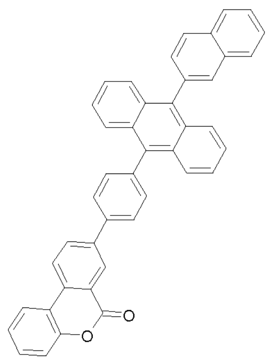

Formel 18

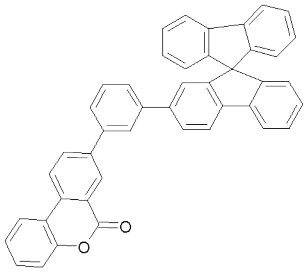

Formel 19

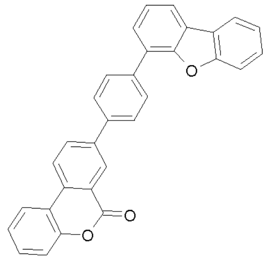

Formel 20

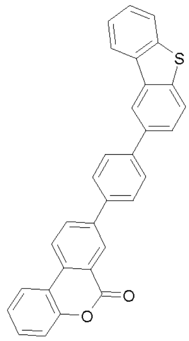

Formel 21

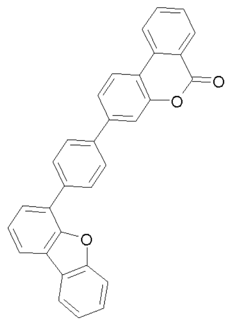

Formel 22

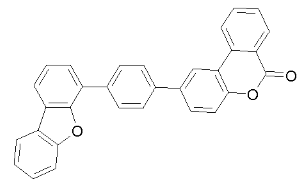

Formel 23

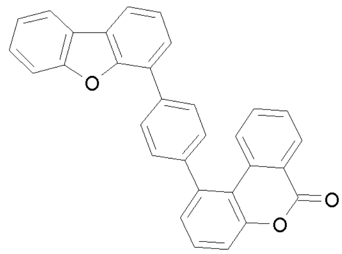

Formel 24

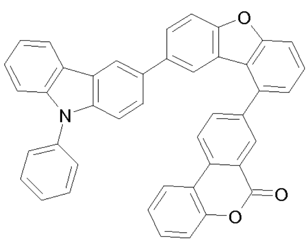

Formel 25

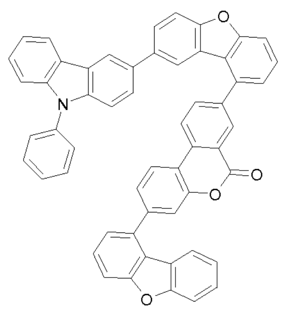

Formel 26

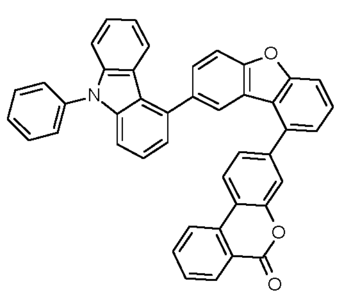

Formel 27

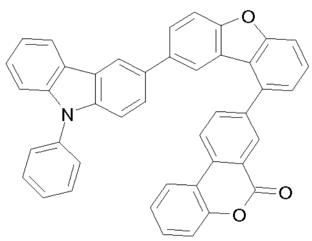

Formel 28

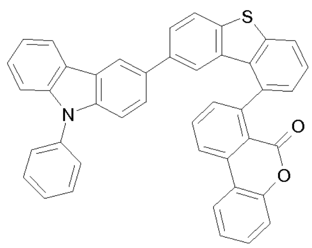

Formel 29

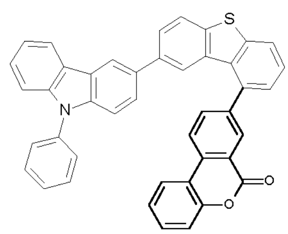

Formel 30

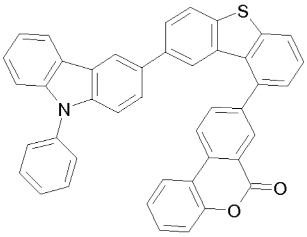

Formel 33

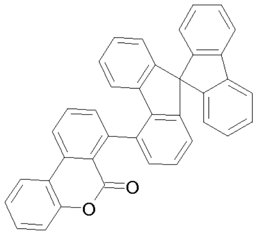

Formel 34

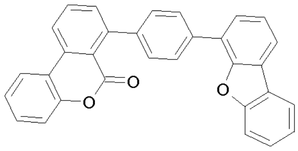

Formel 37

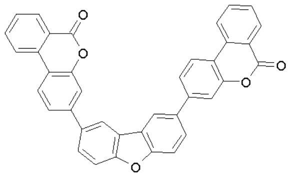

Formel 46

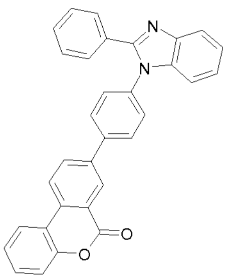

Formel 121

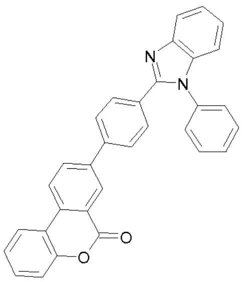

Formel 122

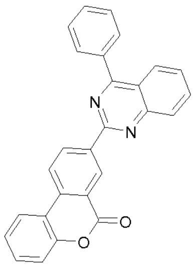

Formel 123

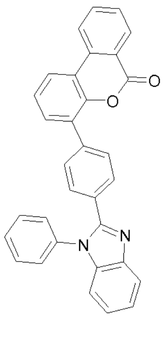

Formel 124

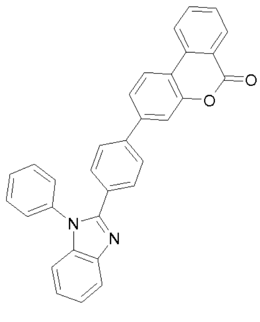

Formel 125

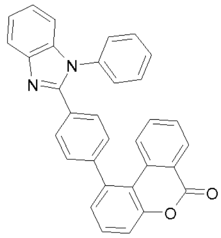

Formel 126

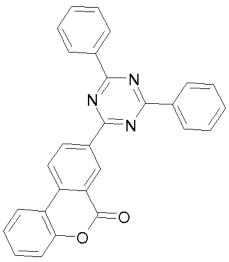

Formel 127

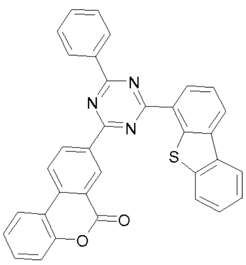

Formel 128

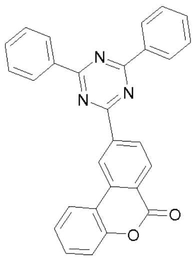

Formel 129

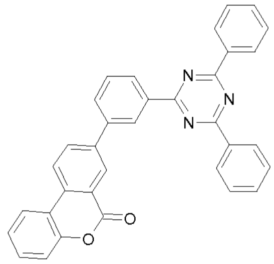

Formel 130

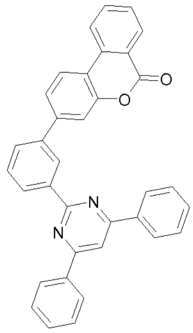

Formel 131

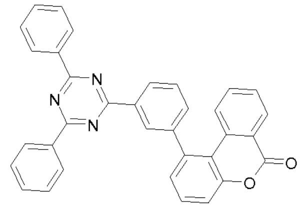

Formel 132

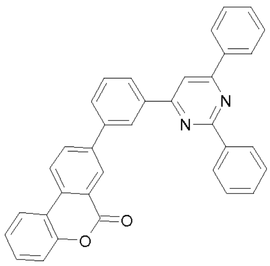

Formel 133

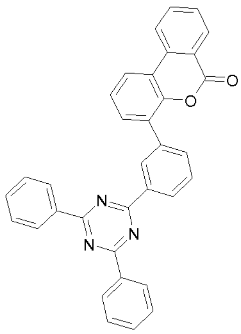

Formel 134

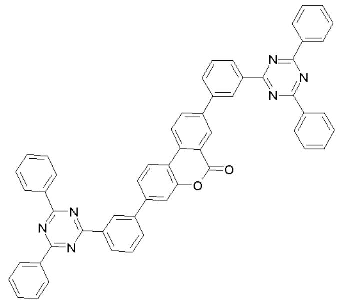

Formel 135

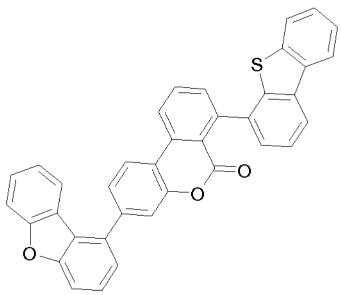

Formel 177

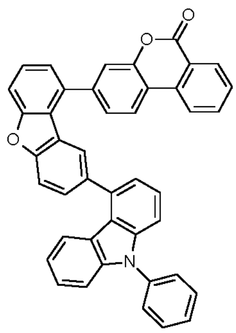

Formel 247.

9. Vorrichtung gemäß einem der Ansprüche 1 bis 7 enthaltend eine Verbindung nach einem oder mehreren der Ansprüche 1 bis 6 als Elektronentransportmaterial.

10. Vorrichtung gemäß einem der Ansprüche 1 bis 7 ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen oder organischen Laserdioden.

## Claims

1. Electronic device containing at least one compound comprising structures of the formula (I) which is sublimable and has a molecular weight of at most 5000 g/mol,

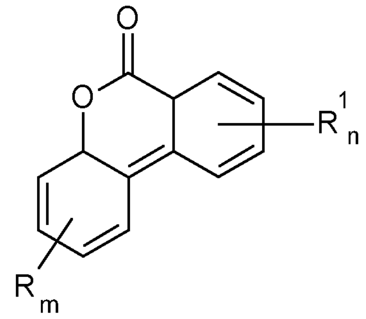

formula (I)

where the following applies to the symbols used:
all groups R and/or $R^1$ in formula (I) represent a group L selected from the formulae (L-7) to (L-26),

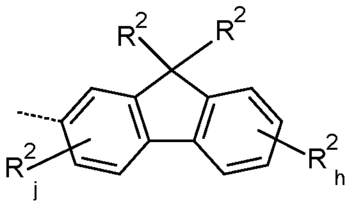

formula (L-7)

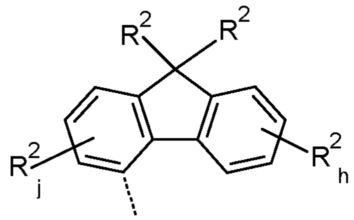

formula (L-8)

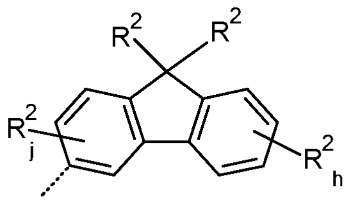

formula (L-9)

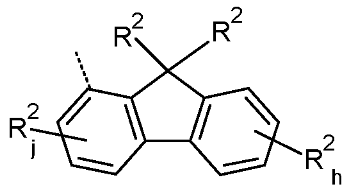

formula (L-10)

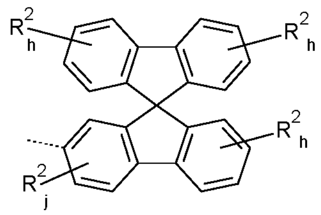

formula (L-11)

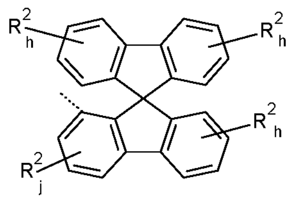

formula (L-12)

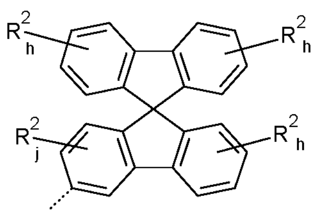

formula (L-13)

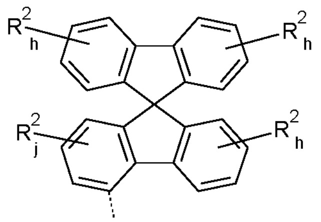

formula (L-14)

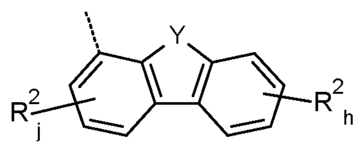

formula (L-15)

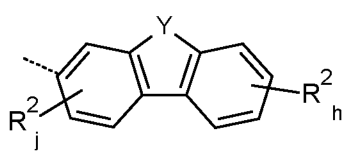

formula (L-16)

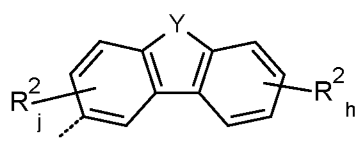

formula (L-17)

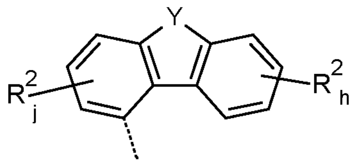

formula (L-18)

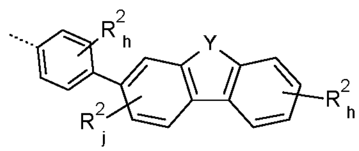

formula (L-19)

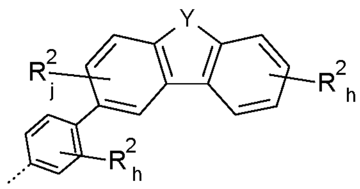

formula (L-20)

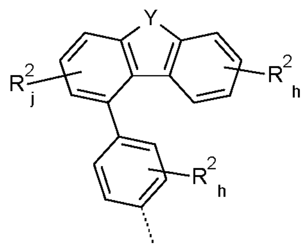

formula (L-21)

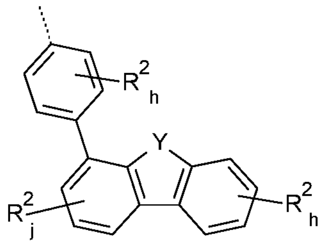

formula (L-22)

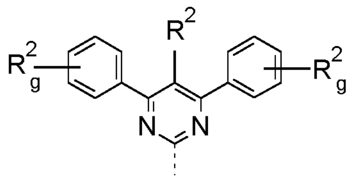

formula (L-23)

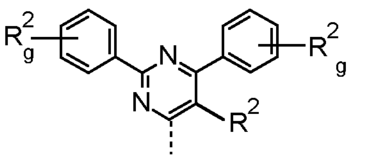

formula (L-24)

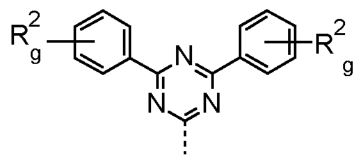

formula (L-26)

where the dashed bond marks the bonding position,
g is 0, 1, 2, 3, 4 or 5,
h is 0, 1, 2, 3 or 4,
j is 0, 1, 2 or 3,
Y stands for O or S,
$R^2$ is on each occurrence, identically or differently, H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^3)_3$, $B(OR^3)_2$, $OSO_2R^3$, a straight-chain alkyl, alkoxy orthioalkoxy group having 1 to 10 C atoms or a branched or cyclic alkyl, alkoxy or thioalkoxy group having 3 to 10 C atoms, which may in each case be substituted by one or more radicals $R^3$, where one or more non-adjacent $CH_2$ groups may be replaced by C≡C, $Si(R^3)_2$, $Ge(R^3)_2$, $Sn(R^3)_2$, C=O, C=S, C=Se, $P(=O)(R^3)$, SO, $SO_2$, O, S or $CONR^3$ and where one or more H atoms may be replaced by D, F, Cl, Br, I, CN or $NO_2$, or an aromatic or heteroaromatic ring system having 5 to 24 aromatic ring atoms, which may in each case be substituted by one or more radicals $R^3$, or an aryloxy or heteroaryloxy group having 5 to 24 aromatic ring atoms, which may be substituted by one or more radicals $R^3$; two or more adjacent substituents $R^2$ may also form a mono- or polycyclic, aliphatic or aromatic ring system with one another;
$Ar^1$ is on each occurrence, identically or differently, an aromatic or heteroaromatic ring system having 5 to 30 aromatic ring atoms, which may be substituted by one or more radicals $R^2$; two radicals $Ar^1$ which are bonded to the same phosphorus atom may also be linked to one another by a single bond or a bridge selected from $B(R^3)$, $C(R^3)_2$, $Si(R^3)_2$, C=O, $C=NR^3$, $C=C(R^3)_2$, O, S, S=O, $SO_2$, $N(R^3)$, $P(R^3)$ and $P(=O)R^3$;
$R^3$ is on each occurrence, identically or differently, H, D, F or an aliphatic, aromatic and/or heteroaromatic hydrocarbon radical having 1 to 20 C atoms, in which, in addition, H atoms may be replaced by F; two or more adjacent substituents $R^3$ may also form a mono- or polycyclic, aliphatic or aromatic ring system with one another;
m, n are in each case, independently of one another, 0, 1 or 2;
and
the sum of m and n is 1 or 2.

**2.** Device according to Claim 1, **characterised in that** the structure of the formula (I) has two groups L.

**3.** Device according to Claim 1 or 2, **characterised in that** the group L in formula (I) includes at least one fluorenyl

and/or spirobifluorenyl group.

4. Device according to one of Claims 1 to 3, **characterised in that** the index m is 1 or 2 and the radical R represents a group L, where the index n is equal to 0.

5. Device according to one of Claims 1 to 4, **characterised in that** the index n is 1 or 2 and the radical $R^1$ represents a group L, where the index m is equal to 0.

6. Device according to one of Claims 1 to 5, **characterised in that** the group L in formula (I) includes at least one heteroaryl group containing a nitrogen atom.

7. Device according to one of Claims 1 to 6, **characterised in that** the group L in formula (I) includes at least one diazine, triazine, benzothiophene and/or benzofuran group.

8. Device according to Claim 1 containing a compound of the following formulae

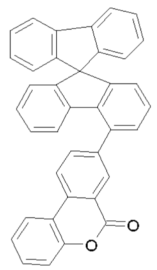

formula 2

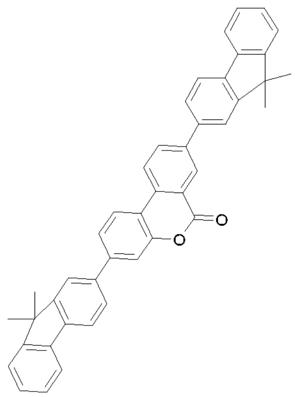

formula 3

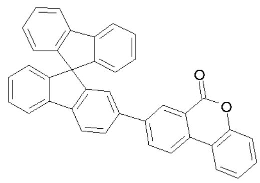

EG7

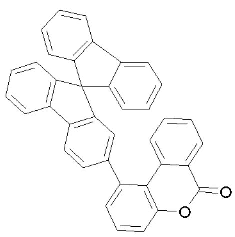

formula 4

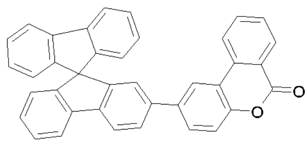

formula 5

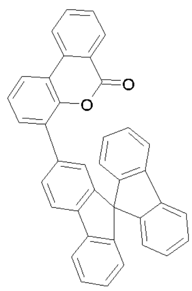

formula 6

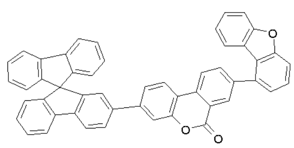

formula 8

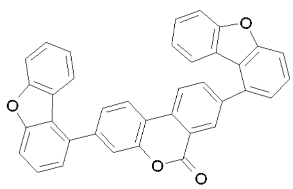

formula 9

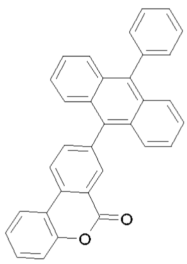

formula 11

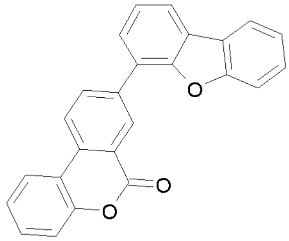

formula 16

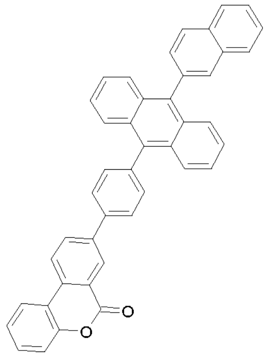

formula 18

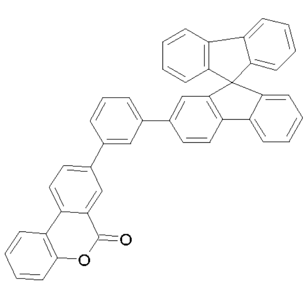

formula 19

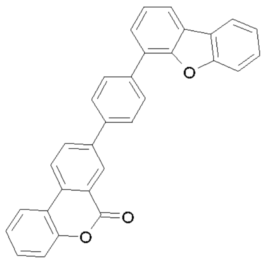

formula 20

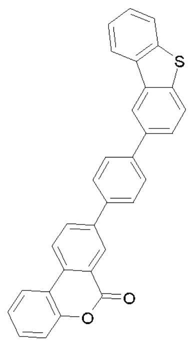

formula 21

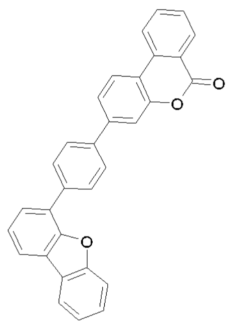

formula 22

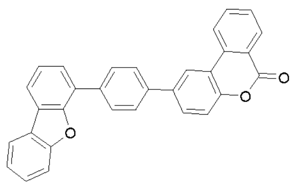

formula 23

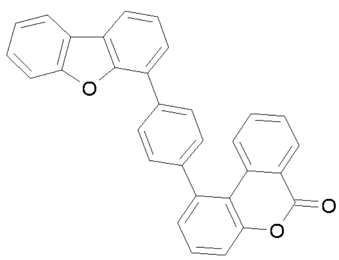

formula 24

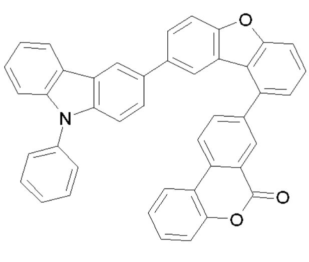

formula 25

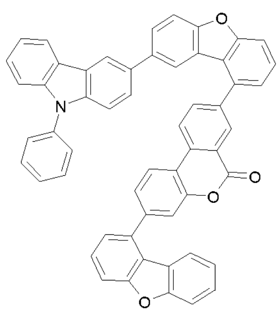

formula 26

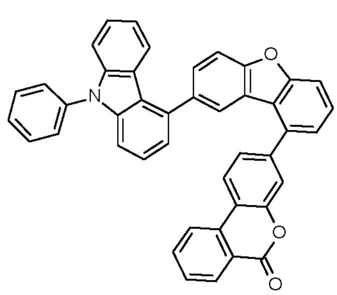

formula 27

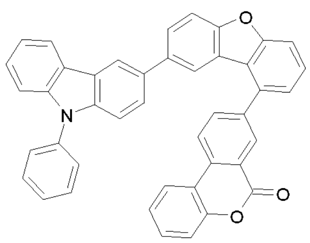
formula 28

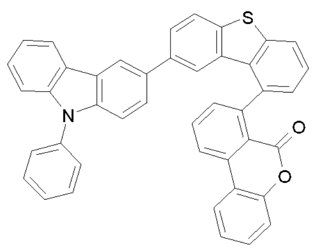
formula 29

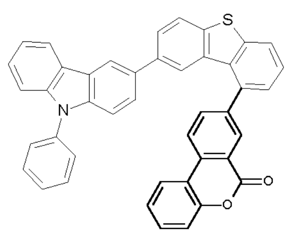
formula 30

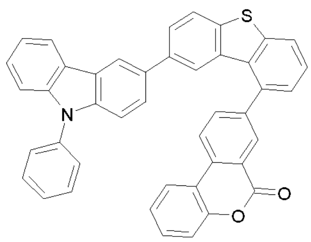
formula 33

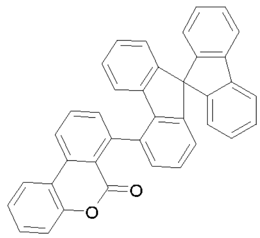
formula 34

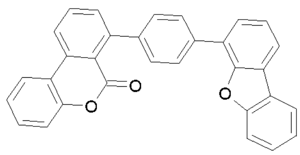
formula 37

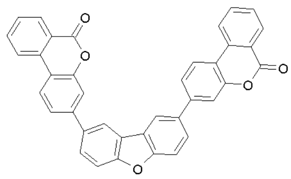
formula 46

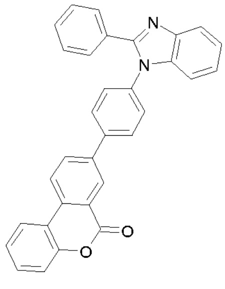
formula 121

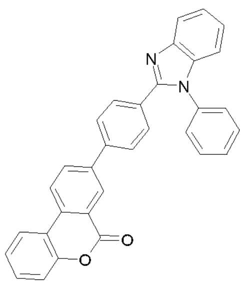
formula 122

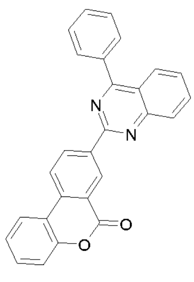
formula 123

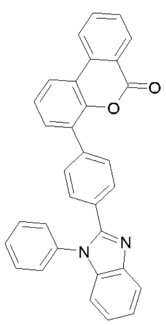
formula 124

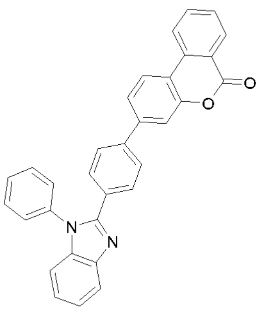
formula 125

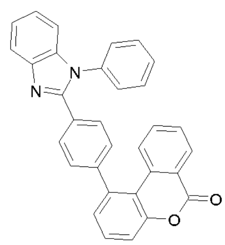
formula 126

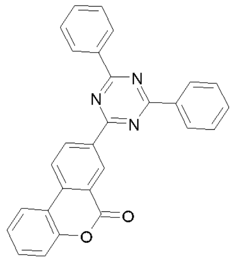

formula 127

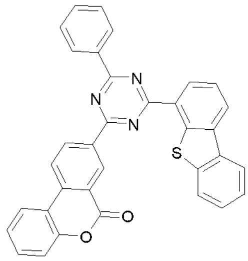

formula 128

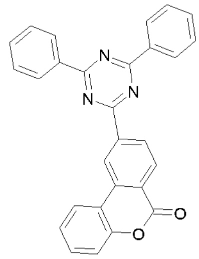

formula 129

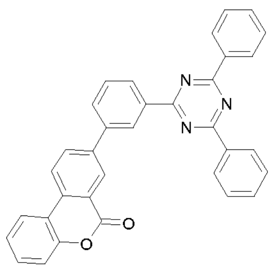

formula 130

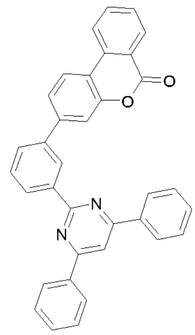

formula 131

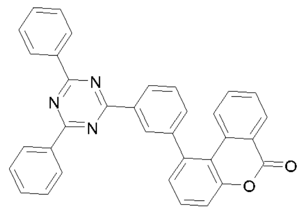

formula 132

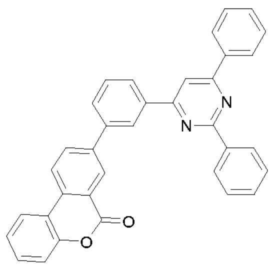

formula 133

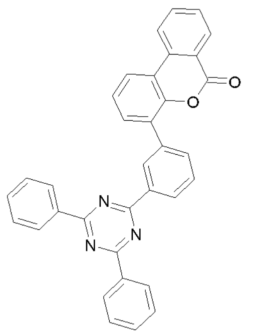

formula 134

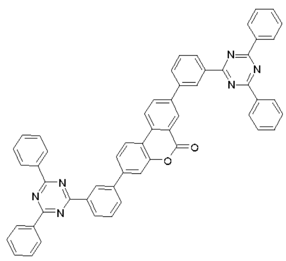

formula 135

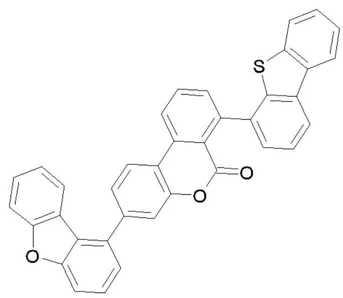

formula 177

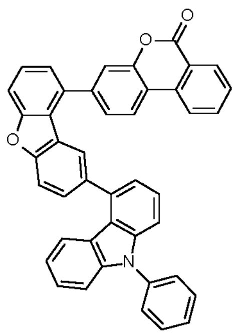

formula 247.

9. Device according to one of Claims 1 to 7 containing a compound according to one or more of Claims 1 to 6 as electron-transport material.

10. Device according to one of Claims 1 to 7 selected from the group consisting of organic electroluminescent devices, organic integrated circuits, organic field-effect transistors, organic thin-film transistors, organic light-emitting transistors, organic solar cells, organic optical detectors, organic photoreceptors, organic field-quench devices, light-emitting electrochemical cells or organic laser diodes.

## Revendications

**1.** Dispositif électronique contenant au moins un composé qui comprend des structures de la formule (I), lequel peut être sublimé et présente un poids moléculaire d'au plus 5000 g/mol,

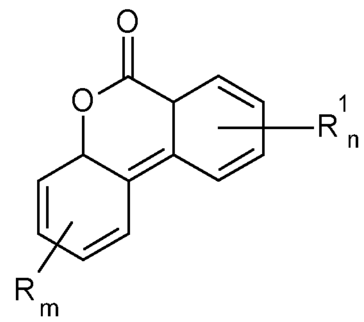

formule (I)

dans laquelle ce qui suit s'applique aux symboles qui sont utilisés : tous les groupes R et/ou $R^1$ dans la formule (I) représentent un groupe L qui est sélectionné parmi les formules (L-7) à (L-26),

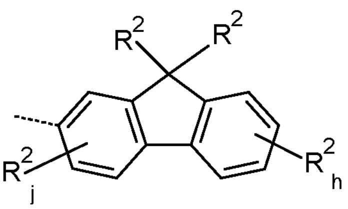

formule (L-7)

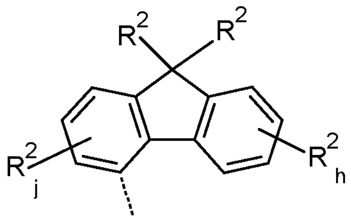

formule (L-8)

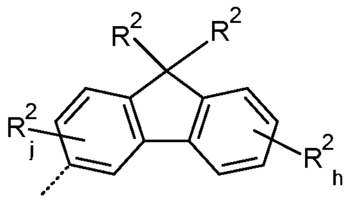

formule (L-9)

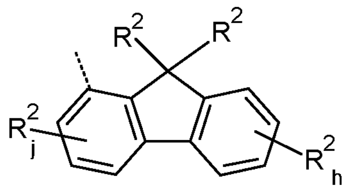

formule (L-10)

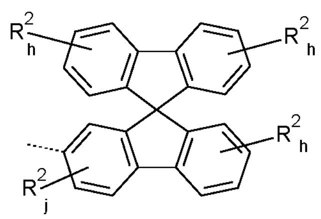

formule (L-11)

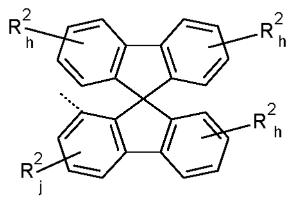

formule (L-12)

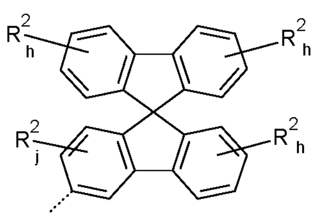

formule (L-13)

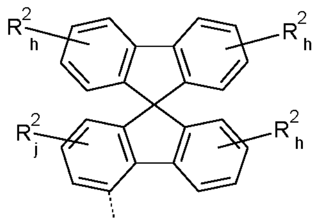

formule (L-14)

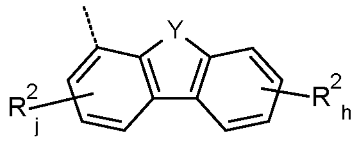

formule (L-15)

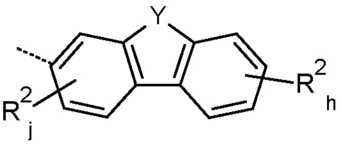

formule (L-16)

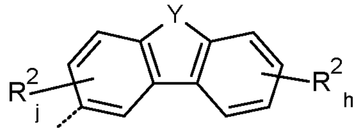

formule (L-17)

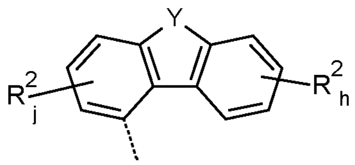

formule (L-18)

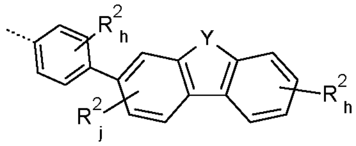

formule (L-19)

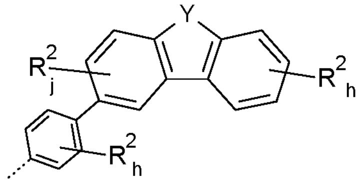

formule (L-20)

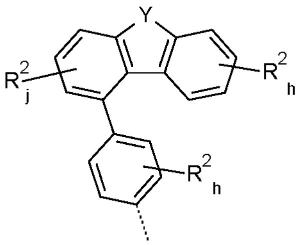

formule (L-21)

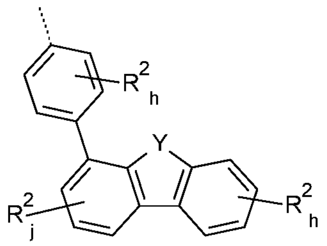

formule (L-22)

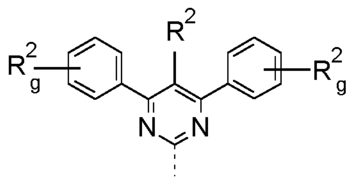

formule (L-23)

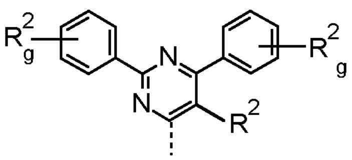

formule (L-24)

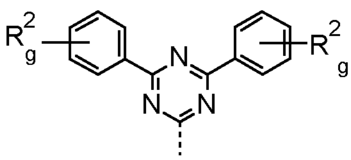

formule (L-26)

dans lesquelles la liaison en pointillés repère la position de liaison,

g est 0, 1, 2, 3, 4 ou 5,

h est 0, 1, 2, 3 ou 4,

j est 0, 1, 2 ou 3,

Y représente O ou S,

$R^2$ est pour chaque occurrence, de manière identique ou différente, H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^3)_3$, $B(OR^3)_2$, $OSO_2R^3$, un groupe alkyle, alcoxy ou thioalcoxy en chaîne droite qui comporte de 1 à 10 atome(s) de C ou un groupe alkyle, alcoxy ou thioalcoxy ramifié ou cyclique qui comporte de 3 à 10 atomes de C, lequel peut dans chaque cas être substitué par un radical ou par plusieurs radicaux $R^3$, où un ou plusieurs groupe(s) $CH_2$ non adjacents peut/peuvent être remplacé(s) par C=C, $Si(R^3)_2$, $Ge(R^3)_2$, $Sn(R^3)_2$, C=O, C=S, C=Se, $P(=O)(R^3)$, SO, $SO_2$, O, S ou $CONR^3$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 24 atomes de cycle aromatique, lequel peut dans chaque cas être substitué par un radical ou par plusieurs radicaux $R^3$, ou un groupe aryloxy ou hétéroaryloxy qui comporte de 5 à 24 atomes de cycle aromatique, lequel peut être substitué par un radical ou par plusieurs radicaux $R^3$; deux substituants $R^2$ adjacents ou plus peuvent également former un système de cycle aliphatique ou aromatique mono- ou polycyclique l'un avec l'autre ou les uns avec les autres ;

$Ar^1$ est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux $R^2$ ; deux radicaux $Ar^1$ qui sont liés au même atome de phosphore peuvent également être liés l'un à l'autre par une liaison simple ou un pont qui est sélectionné parmi $B(R^3)$, $C(R^3)_2$, $Si(R^3)_2$, C=O,

C=NR$^3$, C=C(R$^3$)$_2$, O, S, S=O, SO$_2$, N(R$^3$), P(R$^3$) et P(=O)R$^3$;
R$^3$ est pour chaque occurrence, de manière identique ou différente, H, D, F ou un radical hydrocarbone aliphatique, aromatique et/ou hétéroaromatique qui comporte de 1 à 20 atome(s) de C, où, en outre, des atomes de H peuvent être remplacés par F ; deux substituants R$^3$ adjacents ou plus peuvent également former un système de cycle aliphatique ou aromatique mono- ou polycyclique l'un avec l'autre ou les uns avec les autres ;
m, n sont dans chaque cas, de manière indépendante l'un de l'autre, 0, 1 ou 2 ;
et
la somme de m et de n est 1 ou 2.

**2.** Dispositif selon la revendication 1, **caractérisé en ce que** la structure de la formule (I) comporte deux groupes L.

**3.** Dispositif selon la revendication 1 ou 2, **caractérisé en ce que** le groupe L dans la formule (I) inclut au moins un groupe fluorényle et/ou spirobifluorényle.

**4.** Dispositif selon l'une des revendications 1 à 3, **caractérisé en ce que** l'indice m est 1 ou 2 et le radical R représente un groupe L, où l'indice n est égal à 0.

**5.** Dispositif selon l'une des revendications 1 à 4, **caractérisé en ce que** l'indice n est 1 ou 2 et le radical R$^1$ représente un groupe L, où l'indice m est égal à 0.

**6.** Dispositif selon l'une des revendications 1 à 5, **caractérisé en ce que** le groupe L dans la formule (I) inclut au moins un groupe hétéroaryle qui contient un atome d'azote.

**7.** Dispositif selon l'une des revendications 1 à 6, **caractérisé en ce que** le groupe L dans la formule (I) inclut au moins un groupe diazine, triazine, benzothiophène et/ou benzofurane.

**8.** Dispositif selon la revendication 1 contenant un composé des formules qui suivent

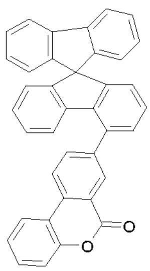
formule 2

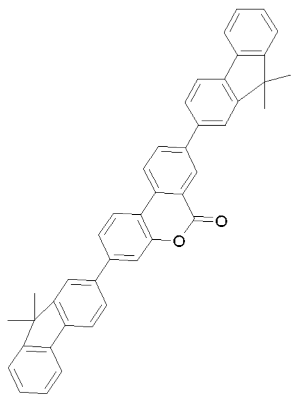
formule 3

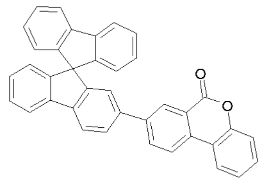
EG7

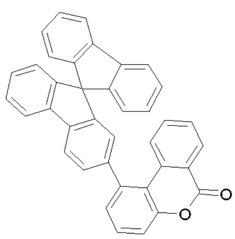
formule 4

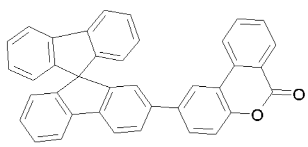
formule 5

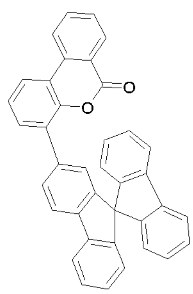
formule 6

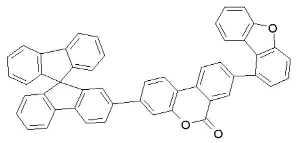

formule 8

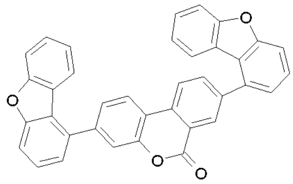

formule 9

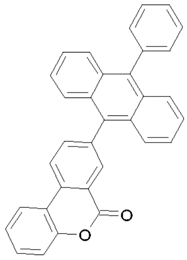

formule 11

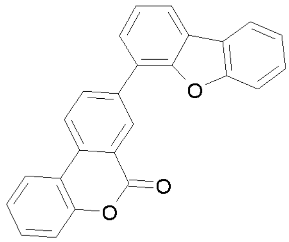

formule 16

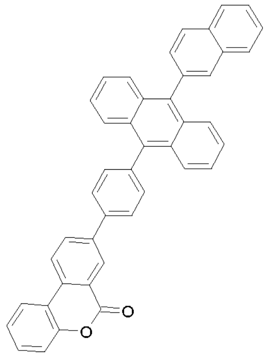

formule 18

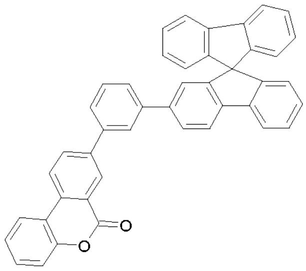

formule 19

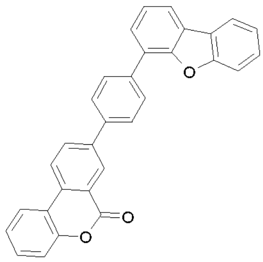

formule 20

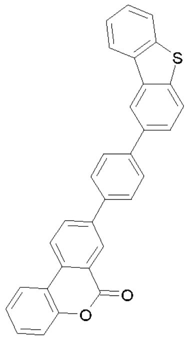

formule 21

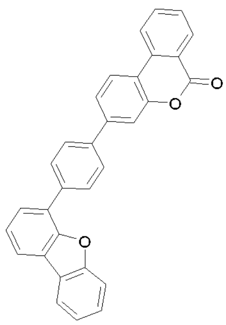

formule 22

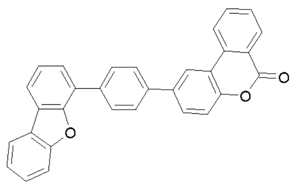

formule 23

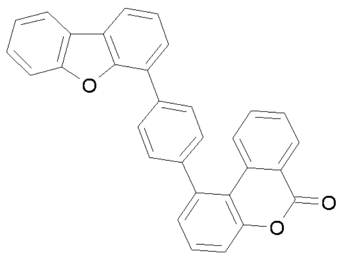

formule 24

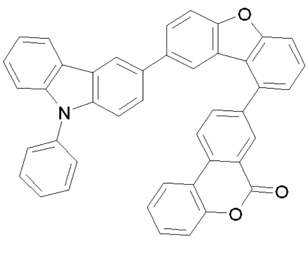
formule 25

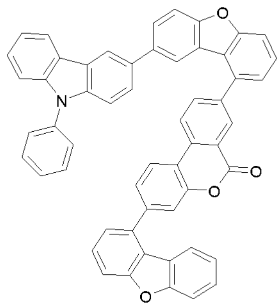
formule 26

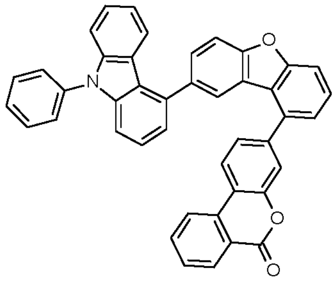
formule 27

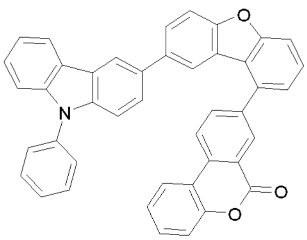
formule 28

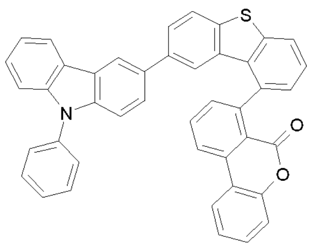
formule 29

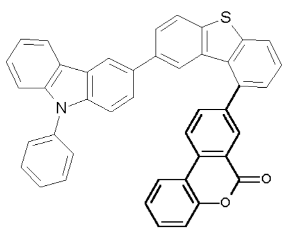
formule 30

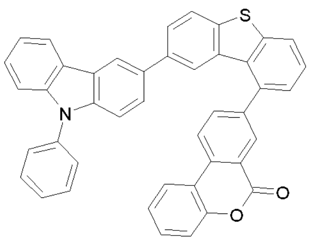
formule 33

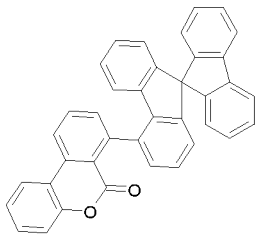
formule 34

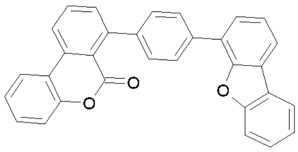
formule 37

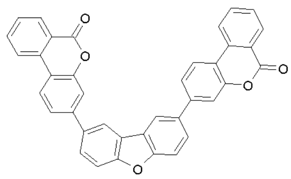
formule 46

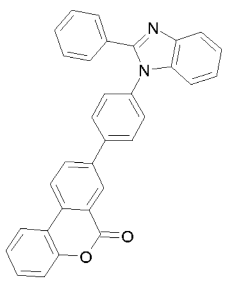
formule 121

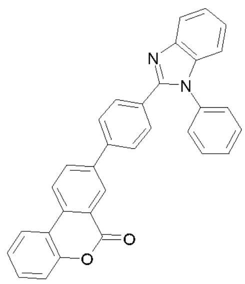
formule 122

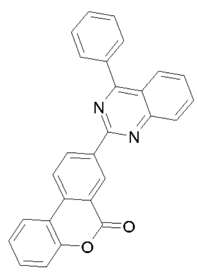
formule 123

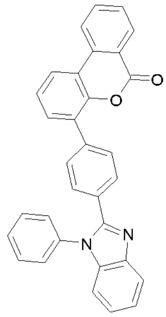

formule 124

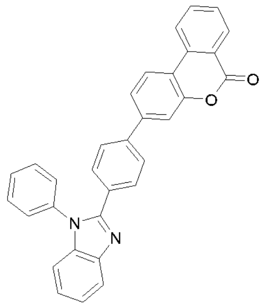

formule 125

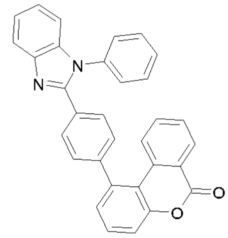

formule 126

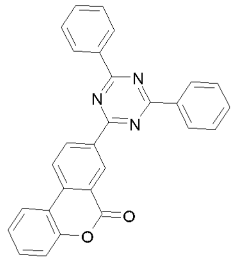

formule 127

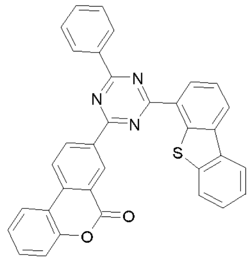

formule 128

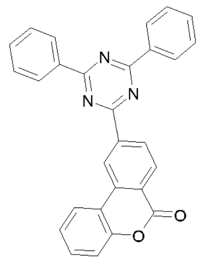

formule 129

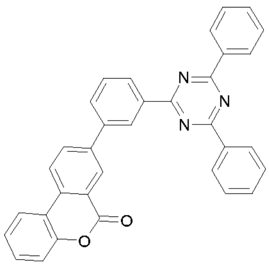

formule 130

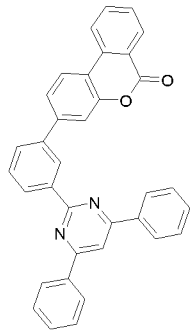

formule 131

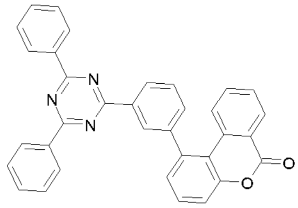

formule 132

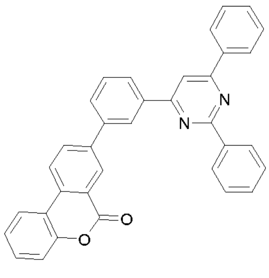

formule 133

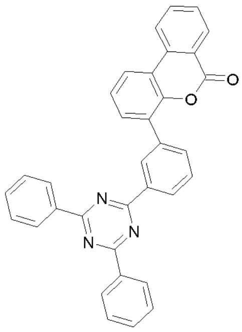

formule 134

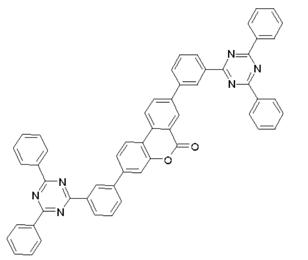

formule 135

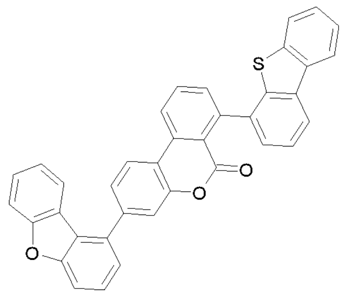

formule 177

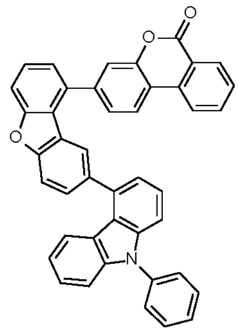

formule 247.

**9.** Dispositif selon l'une des revendications 1 à 7 contenant un composé selon une ou plusieurs des revendications 1 à 6 en tant que matériau de transport d'électrons.

**10.** Dispositif selon l'une des revendications 1 à 7 sélectionné parmi le groupe qui est constitué par les dispositifs électroluminescents organiques, les circuits intégrés organiques, les transistors à effet de champ organiques, les transistors à film mince organiques, les transistors à émission de lumière ou électroluminescents organiques, les cellules solaires organiques, les détecteurs optiques organiques, les photorécepteurs organiques, les dispositifs à extinction de champ organiques, les cellules électrochimiques à émission de lumière ou électroluminescentes ou les diodes laser organiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9013148 A1
- JP 2009073808 A
- WO 200533174 A
- WO 200439859 A
- WO 200399901 A
- WO 2014015935 A
- WO 2015082056 A
- US 2007051922 A
- EP 842208 A
- WO 2000022026 A
- EP 707020 A
- EP 894107 A
- WO 2006061181 A
- WO 9218552 A
- WO 2004070772 A
- WO 2004113468 A
- EP 1028136 A
- WO 2005014689 A
- WO 2004041901 A
- WO 2004113412 A
- WO 2005040302 A
- WO 2005104264 A
- WO 2007017066 A
- US 7294849 B
- WO 200070655 A
- WO 200141512 A
- WO 200202714 A
- WO 200215645 A
- EP 1191613 A
- EP 1191612 A
- EP 1191614 A
- WO 2005033244 A
- WO 2005019373 A
- US 20050258742 A
- WO 2005011013 A
- WO 2004013080 A
- WO 2004093207 A
- WO 2006005627 A
- WO 2010006680 A
- WO 2005039246 A
- US 20050069729 A
- JP 2004288381 A
- EP 1205527 A
- WO 2008086851 A
- US 20090134784 A
- WO 2007063754 A
- WO 2008056746 A
- WO 2010136109 A
- WO 2011000455 A
- EP 1617710 A
- EP 1617711 A
- EP 1731584 A
- JP 2005347160 A
- WO 2007137725 A
- WO 2005111172 A
- WO 2006117052 A
- WO 2010054729 A
- WO 2010054730 A
- WO 2010015306 A
- EP 652273 A
- WO 2009062578 A
- WO 2009148015 A
- US 20090136779 A
- WO 2010050778 A
- WO 2011042107 A
- WO 2011088877 A
- WO 2010108579 A


### In der Beschreibung aufgeführte Nicht-Patentliteratur


- **M. S. ARNOLD et al.** *Appl. Phys. Lett.,* 2008, vol. 92, 053301